# EUROPEAN PATENT SPECIFICATION

(11) **EP 1 133 564 B1**
(45) Date of publication and mention of the grant of the patent: **04.08.2004**
(21) Application number: 99965810.7
(22) Date of filing: 15.11.1999
(51) Int. Cl.: C12N 15/57, C12N 9/64, C12N 15/63, C07K 16/40, G01N 33/53, C07K 19/00, C07K 16/42

(54) **ZACE1: A HUMAN METALLOENZYME**
ZACE1: EIN MENSCHLICHES METALLOENZYM
ZACE1: METALLOENZYME HUMAINE

(30) Priority: 25.11.1998 US 199897
(43) Date of publication of application: 19.09.2001
(73) Proprietor: ZymoGenetics, Inc., Seattle, WA 98102 (US)
(72) Inventor: SHEPPARD, Paul, O., Redmond, WA 98053 (US)
(74) Representative: Schlich, George William
(86) International application number: PCT/US1999/027076
(87) International publication number: WO 2000/031278

(56) References cited:
- MICHAEL J. CORNELL ET AL.: "Cloning and expression of an evolutionary conserved single-domain angiotensin converting enzyme from Drosophila melanogaster" JOURNAL OF BIOLOGICAL CHEMISTRY, vol. 270, no. 23, 9 June 1995 (1995-06-09), pages 13613-13619, XP002135455 MD US
- EHLERS M R ET AL: "Molecular cloning of human testicular angiotensin - converting enzyme: the testis isozyme is identical to the C-terminal half of endothelial angiotensin - converting enzyme." PROCEEDINGS OF THE NATIONAL ACADEMY OF SCIENCES OF THE UNITED STATES OF AMERICA, (1989 OCT) 86 (20) 7741-5. , XP000113802

### SEQUENCE LISTING

<110> ZymoGenetics. Inc.
<120> Zace1: A Human Metalloenzyme
<130> 98-79PC
<160> 3
<170> FastSEQ for Windows Version 3.0
<210> 1
<211> 694
<212> PRT
<213> Homo sapiens
<400> 1
   Met Gly Ala Arg Trp Thr Cys Cys Pro Gly Pro Ser Leu Leu Val Leu
   1 5 10 15
   Leu Cys Tyr Gly Gln Leu Leu Pro Trp Leu Arg Ile Lys Gly Glu His
   20 25 30
   Ser Leu Gly Val Ala Gly Thr Pro Arg Ser Met Gly Pro Asp Lys Gly
   35 40 45
   Thr Gly Cys Asn Glu Thr Glu Thr Lys Ile Phe Leu Gln Phe Tyr Asp
   50 55 60
   Gln Thr Gly Glu Val Val Leu Asn Lys Phe Met Glu Ala Thr Trp Asn
   65 70 75 80
   Tyr Val Thr Asn Ile Thr Arg Lys Asn Gln Glu Glu Met Met Lys Asp
   85 90 95
   Met Glu Arg Ser Gln Phe Met Ile Tyr Phe Gly Thr Gln Ala His Leu
   100 105 110
   Phe Lys Val Thr Gln Phe Lys Asp Pro Asp Val Asn Gly Met Leu Ser
   115 120 125
   Lys Leu Gln Asn Ile Asp Lys Ala Ala Leu Ser Lys Asp Glu Leu Arg
   130 135 140
   Glu Tyr Asn Glu Leu Leu Ala Xaa Leu Glu Met Thr Tyr Ser Met Ala
   145 150 155 160
   Gln Val Cys Leu Asn Glu Gly Pro Cys Leu Ser Leu Glu Ser Glu Leu
   165 170 175
   Glu Val Met Ala Thr Ser Arg Asp Lys Glu Glu Leu Leu Trp Ala Trp
   180 185 190
   Gln Gly Trp Gln Asp Ala Val Gly Arg Gln Ile Cys Thr Thr Phe Glu
   195 200 205
   His Tyr Val Glu Leu Ser Asn Lys Ala Ala Gln Leu Asn Gly Val Xaa
   210 215 220
   Lys Asp Met Gly Ala Leu Trp His Ser Lys Tyr Glu Ser Asp Thr Leu
   225 230 235 240
   Glu Gln Asp Leu Glu Arg Leu Phe Gln Glu Leu Arg Pro Leu Tyr Leu
   245 250 255
   Asn Pro His Thr Tyr Val Arg Arg Ala Leu His Arg His Tyr Gly Pro
   260 265 270
   Glu Leu Ile Asp Leu Arg Gly Pro Ile Pro Ala His Leu Leu Gly Glu
   275 280 285
   Asn Thr Leu Ala Gln Ser Trp Val Asn Ile Leu Asp Pro Val Leu Pro
   290 295 300
   Phe Leu Lys Lys Ile Pro Glu Asp Val Thr Lys Ile Met Lys Val Gln
   305 310 315 320
   His Trp Lys Pro Glu Lys Leu Met Leu Glu Glu Ala Glu Thr Phe Phe
   325 330 335
   Thr Tyr Leu Gly Leu Ala Leu Pro Pro Ala Pro Pro Ser Phe Trp Lys
   340 345 350
   Lys Leu Met Leu Met Arg Pro Thr Asp Gly Arg Glu Val Glu Cys His
   355 360 365
   Ile Ser Ala Trp Asn Phe Tyr Gln Asp Asp Asp Phe Arg Ile Lys Lys
   370 375 380
   Cys Ala Glu Val Thr Thr Glu Asp Pro Leu Ser Ile Phe His Glu Met
   385 390 395 400
   Gly His Phe Gln Tyr Phe Leu Gln Tyr Lys Asn Leu Ser Ile Ile Phe
   405 410 415
   Arg Thr Gly Ala Asn Pro Ala Phe Glu Glu Ala Val Gly Ser Val Ile
   420 425 430
   Thr Leu Ser Ala Ser Ser His Lys His Leu Leu Asn Ile Gly Leu Leu
   435 440 445
   Ser Leu Leu Glu Asp Glu Val Asn Phe Leu Met His Ile Ala Leu Glu
   450 455 460
   Lys Ile Ala Phe Ile Pro Phe Gly Tyr Leu Met Asp Leu Phe Arg Trp
   465 470 475 480
   Lys Val Phe Asp Gly Thr Ile Trp Lys Asp Ile Tyr Asn Gln Glu Trp
   485 490 495
   Trp Asn Leu Arg Arg Leu Lys Tyr Gln Gly Leu Cys Pro Ala Ile Pro
   500 505 510
   His Ser Glu Glu Asp Phe Asp Pro Gly Ala Lys Phe His Phe Ser Ala
   515 520 525
   Gly Val Pro Tyr Ile Arg Arg Tyr Phe Leu Ser Leu Val Leu Gln Phe
   530 535 540
   Gln Phe His Glu Thr Leu Cys Lys Ala Ser Gly His Met Gly Pro Leu
   545                 550                 555                 560
   His Gln Cys Asp Ile Tyr Asn Ser Lys Ile Ala Gly Lys Leu Leu Ala
                   565                 570                 575
   Leu Lys Leu Gly Ser Ser Lys Pro Trp Pro Glu Val Leu Lys Met Leu
               580                 585                 590
   Thr Gly Glu Ser Glu Val Ser Thr Asn Val Phe Met Thr Tyr Phe Lys
           595                 600                 605
   Pro Leu Leu Thr Trp Leu Val Thr Glu His Ala Ala Arg Gly Glu Thr
       610                 615                 620
   Pro Gly Val Pro Leu Gln Phe Tyr Pro Pro Tyr Glu Thr Pro Met Ser
   625                 630                 635                 640
   Ser Thr Glu Lys Asp Thr Asp Lys Val Thr Phe Leu Ser Leu Lys Leu
                   645                 650                 655
   Asp Pro Asn Gln Ala Lys Phe Gly Tyr Trp Val Leu Leu Ala Leu Gly
               660                 665                 670
   Phe Val Met Ser Leu Val Val Leu Gly Leu Ala Cys Arg Leu Gln Ser
           675                 680                 685
   Leu Glu Lys Gln Ser Leu
       690
<210> 2
<211> 2082
<212> DNA
<213> Artificial Sequence
<220>
<223> This degenerate sequence encodes the amino acid
   sequence of SEQ ID NO:1.
<221> variation
<222> (1)...(2082)
<223> N is any nucleotide.
<400> 2
   atgggngcnm gntggacntg ytgyccnggn ccnwsnytny tngtnytnyt ntgytayggn    60
   carytnytnc cntggytnmg nathaarggn garcaywsny tnggngtngc nggnacnccn   120
   mgnwsnatgg gnccngayaa rggnacnggn tgyaaygara cngaracnaa rathttyytn   180
   carttytayg aycaracngg ngargtngtn ytnaayaart tyatggargc nacntggaay   240
   taygtnacna ayathacnmg naaraaycar gargaratga tgaargayat ggarmgnwsn   300
   carttyatga thtayttygg nacncargcn cayytnttya argtnacnca rttyaargay   360
   ccngaygtna ayggnatgyt nwsnaarytn caraayathg ayaargcngc nytnwsnaar   420
   gaygarytnm gngartayaa ygarytnytn gcnnnnytng aratgacnta ywsnatggcn   480
   cargtntgyy tnaaygargg nccntgyytn wsnytngarw sngarytnga rgtnatggcn   540
   acnwsnmgng ayaargarga rytnytntgg gcntggcarg gntggcarga ygcngtnggn   600
   mgncaratht gyacnacntt ygarcaytay gtngarytnw snaayaargc ngcncarytn   660
   aayggngtnn nnaargayat gggngcnytn tggcaywsna artaygarws ngayacnytn    720
   garcargayy tngarmgnyt nttycargar ytnmgnccny tntayytnaa yccncayacn    780
   taygtnmgnm gngcnytnca ymgncaytay ggnccngary tnathgayyt nmgnggnccn    840
   athccngcnc ayytnytngg ngaraayacn ytngcncarw sntgggtnaa yathytngay    900
   ccngtnytnc cnttyytnaa raarathccn gargaygtna cnaarathat gaargtncar    960
   caytggaarc cngaraaryt natgytngar gargcngara cnttyttyac ntayytnggn   1020
   ytngcnytnc cnccngcncc nccnwsntty tggaaraary tnatgytnat gmgnccnacn   1080
   gayggnmgng argtngartg ycayathwsn gcntggaayt tytaycarga ygaygaytty   1140
   mgnathaara artgygcnga rgtnacnacn gargayccny tnwsnathtt ycaygaratg   1200
   ggncayttyc artayttyyt ncartayaar aayytnwsna thathttymg nacnggngcn   1260
   aayccngcnt tygargargc ngtnggnwsn gtnathacny tnwsngcnws nwsncayaar   1320
   cayytnytna ayathggnyt nytnwsnytn ytngargayg argtnaaytt yytnatgcay   1380
   athgcnytng araarathgc nttyathccn ttyggntayy tnatggayyt nttymgntgg   1440
   aargtnttyg ayggnacnat htggaargay athtayaayc argartggtg gaayytnmgn   1500
   mgnytnaart aycarggnyt ntgyccngcn athccncayw sngargarga yttygayccn   1560
   ggngcnaart tycayttyws ngcnggngtn ccntayathm gnmgntaytt yytnwsnytn   1620
   gtnytncart tycarttyca ygaracnytn tgyaargcnw snggncayat gggnccnytn   1680
   caycartgyg ayathtayaa ywsnaarath gcnggnaary tnytngcnyt naarytnggn   1740
   wsnwsnaarc cntggccnga rgtnytnaar atgytnacng gngarwsnga rgtnwsnacn   1800
   aaygtnttya tgacntaytt yaarccnytn ytnacntggy tngtnacnga rcaygcngcn   1860
   mgnggngara cnccnggngt nccnytncar ttytayccnc cntaygarac nccnatgwsn   1920
   wsnacngara argayacnga yaargtnacn ttyytnwsny tnaarytnga yccnaaycar   1980
   gcnaarttyg gntaytgggt nytnytngcn ytnggnttyg tnatgwsnyt ngtngtnytn   2040
   ggnytngcnt gymgnytnca rwsnytngar aarcarwsny tn                      2082
<210> 3
<211> 16
<212> PRT
<213> Artificial, Sequence
<220>
<223> Peptide linker.
<400> 3
   Gly Gly Ser Gly Gly Ser Gly Gly Gly Gly Ser Gly Gly Gly Gly Ser
   1               5                   10                  15

## Description

### TECHNICAL FIELD

The present invention relates generally to a new protein expressed by human cells. In particular, the present invention relates to a novel gene that encodes a metalloenzyme, designated as "Zace1," and to nucleic acid molecules encoding Zace1 polypeptides.

### BACKGROUND OF THE INVENTION

Angiotensin-converting enzyme (ACE; peptidyl dipeptidase A; kininase II (EC 3.4.15.1)) is a zinc metallopeptidase that plays roles in blood pressure regulation and fertility. ACE is rather nonspecific and cleaves dipeptides from a broad range of substrates. In general, ACE cleaves a C-terminal dipeptide "A-B" from a polypeptide when A is not a proline residue, and B is neither an aspartate nor a glutamate residue. For example, ACE cleaves a single C-terminal dipeptide from angiotensin I to produce the potent vasopressor angiotensin II, and ACE cleaves the C-terminal dipeptide from [des-Asp']angiotensin I to produce angiotensin III. The enzyme also inactivates the vasodilatory peptide bradykinin by sequential removal of two C-terminal dipeptides. For a general review of angiotensin-converting enzyme, see Corvol *et al., Meth. Enzymol. 246*:283 (1995), Corvol *et al., J Hypertension 13(Suppl. 3)*:S3 (1995), Jackson and Garrison, "Renin and Angiotensin," in *Goodman and Gilman's The Pharmaceutical Basis of Therapeutics, 9*^{*th*} *Edition,* Molinoff and Ruddon (eds.), pages 733-758 (McGraw-Hill 1996), Matsusaka and Ichikawa, *Annu. Rev. Physiol. 59*:395 (1997), and Zimmerman and Dunham, *Annu. Rev. Pharmacol. Toxicol. 37*:53 (1997).

ACE is a cleavable ectoprotein anchored to the plasma membrane through a transmembrane domain. The majority of the membrane-bound form is extracellularly exposed, and this extracellular domain includes at least one active site. A soluble form of ACE circulates in plasma (see, for example, Hooper and Turner, *Biochem. Soc. Trans., 17*:660 (1989)).

Two ACE isoforms have been identified in mammalian tissues. The predominant form is referred to as "somatic" ACE, which has a molecular weight of about 150 kD to about 180 kD, and is predominantly found at the surface of vascular endothelial cells, epithelial cells, and neuroepithelial cells. The other isoform is referred to as "germinal" ACE or testis ACE (tACE), which has a molecular weight of about 90 kD to about 110 kD, and is expressed in post-meiotic cells and sperm. Human somatic ACE has two homologous domains, each comprising a catalytic site and a Zn²⁺-binding region, while human testis ACE contains one catalytic cite.

Cornell *et al, J. Biol. Chem, 270: 13613 (1995)* describe the identification of an apparent single domain (67 kDa) insect ACE in embryos of *Drosophila menalogaster,* and used the cDNA for human somatic ACE as a heterologous probe to clone a cDNA from a *Drosophila* cDNA library.

Hubert *et al., J. Biol. Chem. 266*:15377 (1991), describe the complete intron-exon structure of the human ACE gene. The human ACE gene contains 26 exons, wherein exon 1 to exon 26 is transcribed in somatic ACE mRNA, but exon 13 is removed by splicing; germinal ACE mRNA is transcribed from exon 13 to exon 26. Exons 4-11 and 17-24 encode the two homologous domains (N domain and C domain) within somatic ACE, and are very similar in size and structure. The intron sizes are not conserved. Since somatic ACE and tACE are transcribed from a single gene, alternate splicing or alternative start sites for transcription initiation may be involved. Two functional promoters reside within the gene, which would support initiation from distinct start sites under separate control. The tACE promoter is upstream of the 5' end of tACE mRNA, with a transcriptional initiation

Inhibitors of angiotensin-converting enzyme are used for the treatment of hypertension of various conditions, including left ventricular systolic dysfunction, progressive renal impairment, scleroderma renal crisis, congestive heart failure due to systolic dysfunction, and treatment of atherosclerosis (see, for example, Brown and Vaughan, *Circulation 97*:1411 (1998); Mancini, *Am. J. Med. 105*:40S (1998); Parmley, *Am. J. Med. 105*:27S (1998)). There are at least nine ACE inhibitors approved for use in the United States.

ACE inhibitors can be classified into at least three groups: (1) sulfhydryl-containing inhibitors structurally related to captopril (*e.g*., fentiapril, pivalopril, zofenopril, alacepril), (2) dicarboxyl-containing inhibitors structurally related to enalapril (e.g., lisinopril, benazepril, quinapril, moexipril, ramipril, spirapril, perindopril, indolapril, pentopril, indalapril, cilazapril), and (3) phosphorus-containing inhibitors structurally related to fosinopril. New classes of ACE inhibitors are sought that will inhibit ACE and other zinc metalloproteases. Moreover, new types of ACE inhibitors are also sought that will selectively inhibit ACE hydrolysis of *N*-acetyl-seryl-aspartyl-lysyl-prolyl (AcSDKP), a regulatory factor in hematopoiesis, without effect on angiotensin I or bradykinin metabolism.

Thus, a continuing need exists for the characterization of new forms of zinc metallopeptidases, and the use of the enzymes to identify therapeutically useful compounds.

### BRIEF SUMMARY OF THE INVENTION

The present invention provides a novel metallopeptidase, designated "Zace1." The present invention also provides Zace1 polypeptides and Zace1 fusion proteins, nucleic acid molecules encoding such polypeptides and proteins, and methods for using these nucleic acid molecules and amino acid sequences.

### DETAILED DESCRIPTION OF THE INVENTION

### 1. Overview

A Zace1 polypeptide has the following amino acid sequence:

Zace1 is a new zinc metalloprotease that is a paralog of testis ACE. Somatic ACE and testis ACE (also called germinal ACE) are ACE isoforms that exhibit similar enzymatic activities. Human endothelial somatic ACE has 1306 amino acid residues, including 14 cysteine residues and 17 potential N-linked glycosylation sites, but no Ser/Thr-rich region indicative of O-linked glycosylation sites. Additional features of ACE include a 29 amino acid residue hydrophobic signal peptide, and a 17 amino acid residue transmembrane domain. Somatic ACE also contains two homologous domains, designated as the "N domain" and the "C domain." The current belief is that these duplicative domains arose from duplication of an ancestral gene. The overall amino acid sequence similarity between the N domain and C domain of somatic ACE is about 60%, but is about 89% with respect to 40 amino acids in each domain that include the residues of each active site. The number and relative position of the Cys residues in the two domains is identical. There is little or no sequence similarity between the amino-terminal and carboxy-terminal portions of somatic ACE, and little or no sequence similarity between the stretch of residues that connects the N and C domains of somatic ACE. Each of the N and C domains contains a zinc-binding motif His-Glu-Xaa-Xaa-His (HEXXH) that is present in many zinc metalloproteases. The two His residues of this motif within somatic ACE provide two of the three zinc-coordinating ligands; the third zinc-coordinating ligand is a Glu residue downstream of the C domain's HEXXH C-terminal His.

Zinc is essential for the catalytic activity of ACE, and the zinc ion is predicted to function directly in the catalytic step of peptide hydrolysis by polarizing the zinc-bound water molecule, which then initiates nucleophilic attack on the substrate carbonyl scissile bond. Thus, ACE is a member of the thermolysin branch of the zinc metalloproteases. Monovalent anions enhance the enzymatic hydrolysis of some, but not all, ACE substrates. For some substrates, a concommitant increase in pH increases the amount of monovalent anion (such as chloride) stimulation.

Germinal ACE (testis ACE; tACE) has 732 amino acid residues, and corresponds to the C domain of somatic ACE. Thus, tACE has only one active site, only one HEXXH motif, and a third zinc-coordinating ligand that is a Glu residue 23 residues downstream of the C-terminal histidine residue of the HEXXH motif. The N-terminal 67 amino acid residues of tACE are specific to tACE, and contain a signal peptide that is distinct from that of somatic ACE, as well as a Ser/Thr-rich region for O-glycosylation. Somatic ACE and tACE mRNAs are transcribed from a single gene.

Zace1 contains 694 amino acid residues, whereas mammalian tACE contains 732 amino acid residues. When polypeptides Zace1 and tACE are aligned (with gap residues inserted in Zace1 and tACE to provide appropriate alignment), Zace1 exhibits 53% amino acid sequence identity to tACE. The seven, highly conserved Cys residues present in tACE and the C domain of somatic ACE are present in Zace1 (at residues 163, 169, 367, 385, 508, 551 and 563). The loops that would be formed by the predicted disulfide bonding pairs of Zace1 (residues 163 to 169, residues 367 to 385, and residues 551 to 563) correspond to the three loops of 5, 17 and 11 residues found in tACE. In addition, Zace1 has two additional Cys residues, at positions 51 and 204. The zinc-binding motif HEXXH is present at residues 398 to 402 of Zace1. An expanded zinc binding region signature of zinc metallopeptidases has the following sequence: [GSTALIVN]-x-x-H-E-[LIVMFYW]-{DEHRKP}-H-x-[LIVMFYWGSPQ], where "x" is any amino acid residue, acceptable amino acid residues are listed between square brackets, and unacceptable amino acid residues are listed between braces (PROSITE sequence No. PS00142 of Release 15.0; Bairoch *et* al., *Nucleic Acids Res. 25*:217 (1997)). This signature resides within the Zace1 polypeptide at amino acid residues 395 to 404 of SEQ ID NO: 1.

In Zace1, 23 residues separate the C-terminal His of the REXXH motif and the conserved EX(I/V)X(D/S) motif present at residues 426 to 430, where "(I/V)" indicates that either I or V may be present and "(D/S)" indicates that either D or S may be present. The Glu domain at position 426 is predicted to be the third zinc-binding (or zinc-coordinating) ligand within Zace1. At position 430, Zace1 has a serine residue substituted for an aspartic acid residue, which occurs at a corresponding position in ACE. The transmembrane domain of Zace1 includes amino acid residues 663 to 684 of SEQ ID NO:1

As described below, the present invention provides isolated polypeptides having an amino acid sequence that is at least 70%, at least 80%, or at least 90% identical to a reference amino acid sequence selected from the group consisting of: (a) amino acid residues 367 to 430 of SEQ ID NO:1, (b) amino acid residues 163 to 563 of SEQ ID NO:1. (c) amino acid residues 52 to 563 of SEQ ID NO:1, (d) amino acid residues 52 to 644 of SEQ ID NO:1, (e) amino acid residues 52 to 648 of SEQ ID NO:1, (f) amino acid residues 52 to 655 of SEQ ID NO:1, (g) amino acid residues 52 to 662 of SEQ ID NO:1, (h) amino acid residues 52 to 682 of SEQ ID NO:1, (i) amino acid residues 52 to 694 of SEQ ID NO:1, and (j) amino acid residues 1 to 694 of SEQ ID NO:1, wherein the isolated polypeptide either (i) specifically binds with an antibody that specifically binds with a polypeptide consisting of the amino acid sequence of SEQ ID NO:1, or (ii) exhibits dipeptidyl carboxypeptidase activity.

Illustrative polypeptides include polypeptides comprising an amino acid sequence selected from the group consisting of: (a) amino acid residues 367 to 430 of SEQ ID NO:1, (b) amino acid residues 163 to 563 of SEQ ID NO:1, (c) amino acid residues 52 to 563 of SEQ ID NO: 1. (d) amino acid residues 52 to 644 of SEQ ID NO:1, (e) amino acid residues 52 to 648 of SEQ ID NO:1, (f) amino acid residues 52 to 655 of SEQ ID NO:1, (g) amino acid residues 52 to 662 of SEQ ID NO:1, (h) amino acid residues 52 to 682 of SEQ ID NO: 1, (i) amino acid residues 52 to 694 of SEQ ID NO:1, and (j) amino acid residues 1 to 694 of SEQ ID NO:1. Such a polypeptide can be a metallopeptidase.

Additional exemplary polypeptides include polypeptides that comprise an amino acid sequence comprising the motif "[GSTALIVN]-x-x-H-E-[LIVMFYW]-{DEHRKP}-H-x-[LIVMFYWGSPQ)]," where "x" is any amino acid residue, acceptable amino acid residues are listed between square brackets, and unacceptable amino acid residues are listed between braces. For example, an illustrative polypeptide comprises amino acid residues 395 to 404 of SEQ ID NO:1.

Accordingly, the present invention provides an isolated polypeptide, as described above, wherein the polypeptide comprises an amino acid sequence comprising the motif "X₁-X₂ X₃-H-E-X₄-X₅-H-X₆-X₇" wherein:
X₁ is selected from G, S, T, A , L,I , V and N,
X₂, X₃ and X₆ are any amino acid residue,
X₄ is selected from L, I, V, M, F, Y and W,
X₅ is any amino acid residue except D, E, H, R, K or P, and
X₇ is selected from L, I, V, M, F, Y, W, G, S, P and Q.

The present invention also provides isolated polypeptides comprising an extracellular domain, wherein the extracellular domain comprises amino acid residues 52 to 662 of the amino acid sequence of SEQ ID NO:1. Such polypeptides can further comprise a transmembrane domain that resides in a carboxyl-terminal position relative to the extracellular domain, wherein the transmembrane domain comprises amino acid residues 663 to- 684 of SEQ ID NO:1. These polypeptides can also comprise an intracellular domain that resides in a carboxyl-terminal position relative to the transmembrane domain, wherein the intracellular domain comprises amino acid residues 685 to 694 of SEQ ID NO:1. Such polypeptides can also include a signal secretory sequence that resides in an amino-terminal position relative to the extracellular domain. A signal secretory sequence is provided by amino acid residues 1 to 51 of the amino acid sequence of SEQ ID NO:1.

The present invention also includes variant Zace1 polypeptides, wherein the amino acid sequence of the variant polypeptide shares an identity with the amino acid sequence of SEQ ID NO:1 selected from the group consisting of at least 70% identity, at least 80% identity, at least 90% identity, at least 95% identity, or greater than 95% identity, and wherein any difference between the amino acid sequence of the variant polypeptide and the amino acid sequence of SEQ ID NO: 1 is due to one or more conservative amino acid substitutions. In addition, the present invention contemplates isolated polypeptides, consisting of an amino acid sequence selected from the group consisting of: (a) amino acid residues 367 to 430 of SEQ ID NO:1, (b) amino acid residues 163 to 563 of SEQ ID NO:1, (c) amino acid residues 52 to 563 of SEQ ID NO:1, (d) amino acid residues 52 to 644 of SEQ ID NO:1, (e) amino acid residues 52 to 648 of SEQ ID NO:1, (f) amino acid residues 52 to 655 of SEQ ID NO:1, (g) amino acid residues 52 to 662 of SEQ ID NO: 1, (h) amino acid residues 52 to 682 of SEQ ID NO:1, (i) amino acid residues 52 to 694 of SEQ ID NO:1, and (j) amino acid residues 1 to 694 of SEQ ID NO:1.

The present invention also contemplate allelic variants and orthologs of the Zace1 polypeptides described herein.

The present invention further provides antibodies and antibody fragments that specifically bind with such polypeptides. Exemplary antibodies include polyclonal antibodies, murine monoclonal antibodies, humanized antibodies derived from murine monoclonal antibodies, and human monoclonal antibodies. Illustrative antibody fragments include F(ab')₂, F(ab)₂, Fab', Fab, Fv, scFv, and minimal recognition units.

The present invention also provides isolated nucleic acid molecules that encode a polypeptide comprising an amino acid sequence selected from the group consisting of: (a) amino acid residues 367 to 430 of SEQ ID NO:1, (b) amino acid residues 163 to 563 of SEQ ID NO:1, (c) amino acid residues 52 to 563 of SEQ ID NO: 1, (d) amino acid residues 52 to 644 of SEQ ID NO:1, (e) amino acid residues 52 to 648 of SEQ ID NO:1, (f) amino acid residues 52 to 655 of SEQ ID NO:1, (g) amino acid residues 52 to 662 of SEQ ID NO:1, (h) amino acid residues 52 to 682 of SEQ ID NO:1, (i) amino acid residues 52 to 694 of SEQ ID NO:1, and (j) amino acid residues 1 to 694 of SEQ ID NO:1. An illustrative nucleic acid molecule encodes amino acid residues 1 to 694 of SEQ ID NO:1.

The present invention also includes vectors and expression vectors comprising such nucleic acid molecules. Such expression vectors can comprise a transcription promoter, and a transcription terminator, wherein the promoter is operably linked with the nucleic acid molecule, and wherein the nucleic acid molecule is operably linked with the transcription terminator. The present invention further includes recombinant host cells and recombinant viruses comprising these vectors and expression vectors. Illustrative host cells include bacterial, yeast, fungal, insect, mammalian, and plant cells. Recombinant host cells comprising such expression vectors can be used to produce Zace1 polypeptides by culturing such recombinant host cells that comprise the expression vector and that produce the Zace1 protein, and, optionally, isolating the Zace 1 protein from the cultured recombinant host cells.

The present invention further includes fusion proteins comprising a Zace1 polypeptide or peptide, and nucleic acid molecules that encode such fusion proteins.

In addition, the present invention provides pharmaceutical compositions comprising a pharmaceutically acceptable carrier and at least one of such an expression vector or recombinant virus comprising such expression vectors. The present invention further includes pharmaceutical compositions, comprising a pharmaceutically acceptable carrier and a Zace1 polypeptide or Zace1 antibody described herein.

These and other aspects of the invention will become evident upon reference to the following detailed description.

### 2. Definitions

In the description that follows, a number of terms are used extensively. The following definitions are provided to facilitate understanding of the invention.

As used herein, "nucleic acid" or "nucleic acid molecule" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (*e.g.*, α-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded.

The term "complement of a nucleic acid molecule" refers to a nucleic acid molecule having a complementary nucleotide sequence and reverse orientation as compared to a reference nucleotide sequence. For example, the sequence 5' ATGCACGGG 3' is complementary to 5' CCCGTGCAT 3'.

The term "contig" denotes a nucleic acid molecule that has a contiguous stretch of identical or complementary sequence to another nucleic acid molecule. Contiguous sequences are said to "overlap" a given stretch of a nucleic acid molecule either in their entirety or along a partial stretch of the nucleic acid molecule. For example, representative contigs to the polynucleotide sequence 5' ATGGAGCTT 3' are 5' AGCTTgagt 3' and 3' tcgacTACC 5'.

The term "degenerate nucleotide sequence" denotes a sequence of nucleotides that includes one or more degenerate codons as compared to a reference nucleic acid molecule that encodes a polypeptide. Degenerate codons contain different triplets of nucleotides, but encode the same amino acid residue (*i.e.*, GAU and GAC triplets each encode Asp).

The term "structural gene" refers to a nucleic acid molecule that is transcribed into messenger RNA (mRNA), which is then translated into a sequence of amino acids characteristic of a specific polypeptide.

The term "isolated genomic DNA" denotes DNA obtained from the genome of a cell that contains exons, introns and nontranscribed DNA.

An "isolated nucleic acid molecule" is a nucleic acid molecule that is not integrated in the genomic DNA of an organism. For example, a DNA molecule that encodes a growth factor that has been separated from the genomic DNA of a cell is an isolated DNA molecule. Another example of an isolated nucleic acid molecule is a chemically-synthesized nucleic acid molecule that is not integrated in the genome of an organism. A nucleic acid molecule that has been isolated from a particular species is smaller than the complete DNA molecule of a chromosome from that species.

A "nucleic acid molecule construct" is a nucleic acid molecule, either single- or double-stranded, that has been modified through human intervention to contain segments of nucleic acid combined and juxtaposed in an arrangement not existing in nature.

"Linear DNA" denotes non-circular DNA molecules having free 5' and 3' ends. Linear DNA can be prepared from closed circular DNA molecules, such as plasmids, by enzymatic digestion or physical disruption.

"Complementary DNA (cDNA)" is a single-stranded DNA molecule that is formed from an mRNA template by the enzyme reverse transcriptase. Typically, a primer complementary to portions of mRNA is employed for the initiation of reverse transcription. Those skilled in the art also use the term "cDNA" to refer to a double-stranded DNA molecule consisting of such a single-stranded DNA molecule and its complementary DNA strand. The term "cDNA" also refers to a clone of a cDNA molecule synthesized from an RNA template.

A "promoter" is a nucleotide sequence that directs the transcription of a structural gene. Typically, a promoter is located in the 5' non-coding region of a gene, proximal to the transcriptional start site of a structural gene. Sequence elements within promoters that function in the initiation of transcription are often characterized by consensus nucleotide sequences. These promoter elements include RNA polymerase binding sites, TATA sequences, CAAT sequences, differentiation-specific elements (DSEs; McGehee *et al., Mol. Endocrinol. 7*:551 (1993)), cyclic AMP response elements (CREs), serum response elements (SREs; Treisman, *Seminars in Cancer Biol. 1*:47 (1990)), glucocorticoid response elements (GREs), and binding sites for other transcription factors, such as CRE/ATF (O'Reilly *et al., J Biol. Chem. 267*:19938 (1992)), AP2 (Ye *et al., J Biol. Chem. 269*:25728 (1994)), SP1, cAMP response element binding protein (CREB; Loeken, *Gene Expr. 3*:253 (1993)) and octamer factors (see, in general, Watson *et al.,* eds., *Molecular Biology of the Gene,* 4th ed. (The Benjamin/Cummings Publishing Company, Inc. 1987), and Lemaigre and Rousseau, *Biochem. J. 303*:1 (1994)). If a promoter is an inducible promoter, then the rate of transcription increases in response to an inducing agent. In contrast, the rate of transcription is not regulated by an inducing agent if the promoter is a constitutive promoter. Repressible promoters are also known.

A "core promoter" contains essential nucleotide sequences for promoter function, including the TATA box and start of transcription. By this definition, a core promoter may or may not have detectable activity in the absence of specific sequences that may enhance the activity or confer tissue specific activity.

A "regulatory element" is a nucleotide sequence that modulates the activity of a core promoter. For example, a regulatory element may contain a nucleotide sequence that binds with cellular factors enabling transcription exclusively or preferentially in particular cells, tissues, or organelles. These types of regulatory elements are normally associated with genes that are expressed in a "cell-specific," "tissue-specific," or "organelle-specific" manner.

An "enhancer" is a type of regulatory element that can increase the efficiency of transcription, regardless of the distance or orientation of the enhancer relative to the start site of transcription.

"Heterologous DNA" refers to a DNA molecule, or a population of DNA molecules, that does not exist naturally within a given host cell. DNA molecules heterologous to a particular host cell may contain DNA derived from the host cell species (*i.e.*, endogenous DNA) so long as that host DNA is combined with non-host DNA (*i.e.*, exogenous DNA). For example, a DNA molecule containing a non-host DNA segment encoding a polypeptide operably linked to a host DNA segment comprising a transcription promoter is considered to be a heterologous DNA molecule. Conversely, a heterologous DNA molecule can comprise an endogenous gene operably linked with an exogenous promoter. As another illustration, a DNA molecule comprising a gene derived from a wild-type cell is considered to be heterologous DNA if that DNA molecule is introduced into a mutant cell that lacks the wild-type gene.

A "polypeptide" is a polymer of amino acid residues joined by peptide bonds, whether produced naturally or synthetically. Polypeptides of less than about 10 amino acid residues are commonly referred to as "peptides."

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

A peptide or polypeptide encoded by a non-host DNA molecule is a "heterologous" peptide or polypeptide.

An "integrated genetic element" is a segment of DNA that has been incorporated into a chromosome of a host cell after that element is introduced into the cell through human manipulation. Within the present invention, integrated genetic elements are most commonly derived from linearized plasmids that are introduced into the cells by electroporation or other techniques. Integrated genetic elements are passed from the original host cell to its progeny.

A "cloning vector" is a nucleic acid molecule, such as a plasmid, cosmid, or bacteriophage, that has the capability of replicating autonomously in a host cell. Cloning vectors typically contain one or a small number of restriction endonuclease recognition sites that allow insertion of a nucleic acid molecule in a determinable fashion without loss of an essential biological function of the vector, as well as nucleotide sequences encoding a marker gene that is suitable for use in the identification and selection of cells transformed with the cloning vector. Marker genes typically include genes that provide tetracycline resistance or ampicillin resistance.

An "expression vector" is a nucleic acid molecule encoding a gene that is expressed in a host cell. Typically, an expression vector comprises a transcription promoter, a gene, and a transcription terminator. Gene expression is usually placed under the control of a promoter, and such a gene is said to be "operably linked to" the promoter. Similarly, a regulatory element and a core promoter are operably linked if the regulatory element modulates the activity of the core promoter.

A "recombinant host" is a cell that contains a heterologous nucleic acid molecule, such as a cloning vector or expression vector. In the present context, an example of a recombinant host is a cell that produces Zace1 from an expression vector. In contrast, Zace 1 can be produced by a cell that is a "natural source" of Zace1, and that lacks an expression vector.

"Integrative transformants" are recombinant host cells, in which heterologous DNA has become integrated into the genomic DNA of the cells.

A "fusion protein" is a hybrid protein expressed by a nucleic acid molecule comprising nucleotide sequences of at least two genes. For example, a fusion protein can comprise at least part of a Zace1 polypeptide fused with a polypeptide that binds an affinity matrix. Such a fusion protein provides a means to isolate large quantities of Zace1 using affinity chromatography.

The term "receptor" denotes a cell-associated protein that binds to a bioactive molecule termed a "ligand." This interaction mediates the effect of the ligand on the cell. Receptors can be membrane bound, cytosolic or nuclear; monomeric (*e.g.*, thyroid stimulating hormone receptor, beta-adrenergic receptor) or multimeric (*e.g.*, PDGF receptor, growth hormone receptor, IL-3 receptor, GM-CSF receptor, G-CSF receptor, erythropoietin receptor and IL-6 receptor). Membrane-bound receptors are characterized by a multi-domain structure comprising an extracellular ligand-binding domain and an intracellular effector domain that is typically involved in signal transduction. In certain membrane-bound receptors, the extracellular ligand-binding domain and the intracellular effector domain are located in separate polypeptides that comprise the complete functional receptor.

In general, the binding of ligand to receptor results in a conformational change in the receptor that causes an interaction between the effector domain and other molecule(s) in the cell, which in turn leads to an alteration in the metabolism of the cell. Metabolic events that are often linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids.

The term "secretory signal sequence" denotes a DNA sequence that encodes a peptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

An "isolated polypeptide" is a polypeptide that is essentially free from contaminating cellular components, such as carbohydrate, lipid, or other proteinaceous impurities associated with the polypeptide in nature. Typically, a preparation of isolated polypeptide contains the polypeptide in a highly purified form, *i.e.*, at least about 80% pure, at least about 90% pure, at least about 95% pure, greater than 95% pure, or greater than 99% pure. One way to show that a particular protein preparation contains an isolated polypeptide is by the appearance of a single band following sodium dodecyl sulfate (SDS)-polyacrylamide gel electrophoresis of the protein preparation and Coomassie Brilliant Blue staining of the gel. However, the term "isolated" does not exclude the presence of the same polypeptide in alternative physical forms, such as dimers or alternatively glycosylated or derivatized forms.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "expression" refers to the biosynthesis of a gene product. For example, in the case of a structural gene, expression involves transcription of the structural gene into mRNA and the translation of mRNA into one or more polypeptides.

The term "splice variant" is used herein to denote alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a polypeptide encoded by a splice variant of an mRNA transcribed from a gene.

As used herein, the term "immunomodulator" includes cytokines, stem cell growth factors, lymphotoxins, co-stimulatory molecules, hematopoietic factors, and synthetic analogs of these molecules.

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of less than 10⁹ M⁻¹.

An "anti-idiotype antibody" is an antibody that binds with the variable region domain of an immunoglobulin. In the present context, an anti-idiotype antibody binds with the variable region of an anti-Zace1 antibody, and thus, an anti-idiotype antibody mimics an epitope of Zace1.

An "antibody fragment" is a portion of an antibody such as F(ab')₂, F(ab)₂, Fab', Fab, and the like. Regardless of structure, an antibody fragment binds with the same antigen that is recognized by the intact antibody. For example, an anti-Zace1 monoclonal antibody fragment binds with an epitope of Zace1.

The term "antibody fragment" also includes a synthetic or a genetically engineered polypeptide that binds to a specific antigen, such as polypeptides consisting of the light chain variable region, "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

A "chimeric antibody" is a recombinant protein that contains the variable domains and complementary determining regions derived from a rodent antibody, while the remainder of the antibody molecule is derived from a human antibody.

"Humanized antibodies" are recombinant proteins in which murine complementarity determining regions of a monoclonal antibody have been transferred from heavy and light variable chains of the murine immunoglobulin into a human variable domain.

As used herein, a "therapeutic agent" is a molecule or atom which is conjugated to an antibody moiety to produce a conjugate which is useful for therapy. Examples of therapeutic agents include drugs, toxins, immunomodulators, chelators, boron compounds, photoactive agents or dyes, and radioisotopes.

A "detectable label" is a molecule or atom which can be conjugated to an antibody moiety to produce a molecule useful for diagnosis. Examples of detectable labels include chelators, photoactive agents, radioisotopes, fluorescent agents, paramagnetic ions, or other marker moieties.

The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a polyhistidine tract, protein A (Nilsson *et al., EMBO J. 4*:1075 (1985); Nilsson *et al., Methods Enzymol. 198*:3 (1991)), glutathione S transferase (Smith and Johnson, *Gene 67*:31 (1988)), Glu-Glu affinity tag (Grussenmeyer *et al., Proc. Natl. Acad. Sci. USA 82*:7952 (1985)), substance P, FLAG peptide (Hopp *et al., Biotechnology 6*:1204 (1988)), streptavidin binding peptide, or other antigenic epitope or binding domain. See, in general, *Ford et al., Protein Expression and Purification 2*:95 (1991). DNA molecules encoding affinity tags are available from commercial suppliers (*e.g.*, Pharmacia Biotech, Piscataway, NJ).

A "naked antibody" is an entire antibody, as opposed to an antibody fragment, which is not conjugated with a therapeutic agent. Naked antibodies include both polyclonal and monoclonal antibodies, as well as certain recombinant antibodies, such as chimeric and humanized antibodies.

As used herein, the term "antibody component" includes both an entire antibody and an antibody fragment.

An "immunoconjugate" is a conjugate of an antibody component with a therapeutic agent or a detectable label.

As used herein, the term "antibody fusion protein" refers to a recombinant molecule that comprises an antibody component and a Zace1 polypeptide component. Examples of an antibody fusion protein is a protein that comprises a Zace1 catalytic domain and either an Fc domain or an antigen-biding region.

A "target polypeptide" or a "target peptide" is an amino acid sequence that comprises at least one epitope, and that is expressed on a target cell, such as a tumor cell, or a cell that carries an infectious agent antigen. T cells recognize peptide epitopes presented by a major histocompatibility complex molecule to a target polypeptide or target peptide and typically lyse the target cell or recruit other immune cells to the site of the target cell, thereby killing the target cell.

An "antigenic peptide" is a peptide which will bind a major histocompatibility complex molecule to form an MHC-peptide complex which is recognized by a T cell, thereby inducing a cytotoxic lymphocyte response upon presentation to the T cell. Thus, antigenic peptides are capable of binding to an appropriate major histocompatibility complex molecule and inducing a cytotoxic T cells response, such as cell lysis or specific cytokine release against the target cell which binds or expresses the antigen. The antigenic peptide can be bound in the context of a class I or class II major histocompatibility complex molecule, on an antigen presenting cell or on a target cell.

In eukaryotes, RNA polymerase II catalyzes the transcription of a structural gene to produce mRNA. A nucleic acid molecule can be designed to contain an RNA polymerase II template in which the RNA transcript has a sequence that is complementary to that of a specific mRNA. The RNA transcript is termed an "anti-sense RNA" and a nucleic acid molecule that encodes the anti-sense RNA is termed an "anti-sense gene." Anti-sense RNA molecules are capable of binding to mRNA molecules, resulting in an inhibition of mRNA translation.

An "anti-sense oligonucleotide specific for Zace1" or a "Zace1 anti-sense oligonucleotide" is an oligonucleotide having a sequence (a) capable of forming a stable triplex with a portion of the *Zace1* gene, or (b) capable of forming a stable duplex with a portion of an mRNA transcript of the *Zace1* gene.

A "ribozyme" is a nucleic acid molecule that contains a catalytic center. The term includes RNA enzymes, self-splicing RNAs, self-cleaving RNAs, and nucleic acid molecules that perform these catalytic functions. A nucleic acid molecule that encodes a ribozyme is termed a "ribozyme gene."

An "external guide sequence" is a nucleic acid molecule that directs the endogenous ribozyme, RNase P, to a particular species of intracellular mRNA, resulting in the cleavage of the mRNA by RNase P. A nucleic acid molecule that encodes an external guide sequence is termed an "external guide sequence gene."

The term "variant *Zace1* gene" refers to nucleic acid molecules that encode a polypeptide having an amino acid sequence that is a modification of SEQ ID NO:1. Such variants include naturally-occurring polymorphisms of *Zace1* genes, as well as synthetic genes that contain conservative amino acid substitutions of the amino acid sequence of SEQ ID NO:1.

Alternatively, variant *Zace1* genes can be identified by sequence comparison. Two amino acid sequences have "100% amino acid sequence identity" if the amino acid residues of the two amino acid sequences are the same when aligned for maximal correspondence. Similarly, two nucleotide sequences have "100% nucleotide sequence identity" if the nucleotide residues of the two nucleotide sequences are the same when aligned for maximal correspondence. Sequence comparisons can be performed using standard software programs such as those included in the LASERGENE bioinformatics computing suite, which is produced by DNASTAR (Madison, Wisconsin). Other methods for comparing two nucleotide or amino acid sequences by determining optimal alignment are well-known to those of skill in the art (see, for example, Peruski and Peruski, *The Internet and the New Biology: Tools for Genomic and Molecular Research* (ASM Press, Inc. 1997), *Wu et al.* (eds.), "Information Superhighway and Computer Databases of Nucleic Acids and Proteins," in *Methods in Gene Biotechnology,* pages 123-151 (CRC Press, Inc. 1997), and Bishop (ed.), *Guide to Human Genome Computing,* 2nd Edition (Academic Press, Inc. 1998)). Particular methods for determining sequence identity are described below.

Regardless of the particular method used to identify a variant *Zace1* gene or variant Zace1 polypeptide, a variant gene or polypeptide encoded by a variant gene may be functionally characterized the ability to bind specifically to an anti-Zace1 antibody or by the dipeptidase (e.g., dipeptidyl carboxypeptidase) activity of the variant Zace 1 polypeptide.

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The term "ortholog" denotes a polypeptide or protein obtained from one species that is the functional counterpart of a polypeptide or protein from a different species. Sequence differences among orthologs are the result of speciation.

"Paralogs" are distinct but structurally related proteins made by an organism. Paralogs are believed to arise through gene duplication. For example, α-globin, β-globin, and myoglobin are paralogs of each other.

The present invention includes functional fragments of *Zace1* genes. Within the context of this invention, a "functional fragment" of a *Zace1* gene refers to a nucleic acid molecule that encodes a portion of a Zace1 polypeptide which either has peptidyl dipeptidase activity or specifically binds with an anti-Zace1 antibody.

The term "dipeptidyl peptidase" refers to an enzyme that cleaves dipeptides from the amino terminus of a polypeptide, whereas the term "dipeptidyl carboxypeptidase" refers to an enzyme that cleaves dipeptides from the carboxyl terminus of a polypeptide.

A "metallopeptidase" is a peptide hydrolase which uses a metal in the catalytic mechanism. Typically, metallopeptidases contain a tightly bound transition metal, such as zinc or iron. Angiotensin-converting enzyme (ACE) is an example of a zinc metallopeptidase. The enzymatic activities of ACE include cleavage of the carboxyl-terminal dipeptide from angiotensin I to produce angiotensin II, removal of two carboxyl-terminal dipeptides from bradykinin, hydrolysis of N-acetyl-Ser-Gly-Lys-Pro at the Gly-Lys bond, cleavage of a carboxyl-terminal tripeptide amide from substance P, and luteinizing hormone releasing hormone, and an amino-terminal tripeptide from luteinizing hormone releasing hormone. Several examples of artificial ACE substrate are described herein.

Due to the imprecision of standard analytical methods, molecular weights and lengths of polymers are understood to be approximate values. When such a value is expressed as "about" X or "approximately" X, the stated value of X will be understood to be accurate to ±10%.

### 3. Production of Zace1 Polynucleotides

Nucleic acid molecules encoding a human *Zace1* gene can be obtained by screening a human cDNA or genomic library using polynucleotide probes based upon the amino acid sequence of SEQ ID NO: 1. These techniques are standard and well-established.

As an illustration, a nucleic acid molecule that encodes a human *Zace1* gene can be isolated from a cDNA library. In this case, the first step would be to prepare the cDNA library by isolating RNA from a tissue, using methods well-known to those of skill in the art. In general, RNA isolation techniques must provide a method for breaking cells, a means of inhibiting RNase-directed degradation of RNA, and a method of separating RNA from DNA, protein, and polysaccharide contaminants. For example, total RNA can be isolated by freezing tissue in liquid nitrogen, grinding the frozen tissue with a mortar and pestle to lyse the cells, extracting the ground tissue with a solution of phenol/chloroform to remove proteins, and separating RNA from the remaining impurities by selective precipitation with lithium chloride (see, for example, Ausubel *et al.* (eds.), *Short Protocols in Molecular Biology, 3*^{*rd*} *Edition,* pages 4-1 to 4-6 (John Wiley & Sons 1995) ["Ausubel (1995)"]; Wu *et al., Methods in Gene Biotechnology,* pages 33-41 (CRC Press, Inc. 1997) ["Wu (1997)"]). Alternatively, total RNA can be isolated by extracting ground tissue with guanidinium isothiocyanate, extracting with organic solvents, and separating RNA from contaminants using differential centrifugation (see, for example, Chirgwin *et al., Biochemistry 18*:52 (1979); Ausubel (1995) at pages 4-1 to 4-6; Wu (1997) at pages 33-41).

In order to construct a cDNA library, poly(A)⁺ RNA must be isolated from a total RNA preparation. Poly(A)⁺ RNA can be isolated from total RNA using the standard technique of oligo(dT)-cellulose chromatography (see, for example, Aviv and Leder, *Proc. Nat'l Acad. Sci. USA 69*:1408 (1972); Ausubel (1995) at pages 4-11 to 4-12).

Double-stranded cDNA molecules are synthesized from poly(A)⁺ RNA using techniques well-known to those in the art. (see, for example, Wu (1997) at pages 41-46). Moreover, commercially available kits can be used to synthesize double-stranded cDNA molecules. For example, such kits are available from Life Technologies, Inc. (Gaithersburg, MD), CLONTECH Laboratories, Inc. (Palo Alto, CA), Promega Corporation (Madison, WI) and STRATAGENE (La Jolla, CA).

Various cloning vectors are appropriate for the construction of a cDNA library. For example, a cDNA library can be prepared in a vector derived from bacteriophage, such as a λgt10 vector. See, for example, Huynh *et al.*, "Constructing and Screening cDNA Libraries in λgt10 and λgt11," in *DNA Cloning: A Practical Approach Vol. I,* Glover (ed.), page 49 (IRL Press, 1985); Wu (1997) at pages 47-52.

Alternatively, double-stranded cDNA molecules can be inserted into a plasmid vector, such as a PBLUESCRIPT vector (STRATAGENE; La Jolla, CA), a LAMDAGEM-4 (Promega Corp.) or other commercially available vectors. Suitable cloning vectors also can be obtained from the American Type Culture Collection (Manassas, VA).

To amplify the cloned cDNA molecules, the cDNA library is inserted into a prokaryotic host, using standard techniques. For example, a cDNA library can be introduced into competent *E. coli* DH5 cells, which can be obtained, for example, from Life Technologies, Inc. (Gaithersburg, MD).

A human genomic library can be prepared by means well-known in the art (see, for example, Ausubel (1995) at pages 5-1 to 5-6; Wu (1997) at pages 307-327). Genomic DNA can be isolated by lysing tissue with the detergent Sarkosyl, digesting the lysate with proteinase K, clearing insoluble debris from the lysate by centrifugation, precipitating nucleic acid from the lysate using isopropanol, and purifying resuspended DNA on a cesium chloride density gradient.

DNA fragments that are suitable for the production of a genomic library can be obtained by the random shearing of genomic DNA or by the partial digestion of genomic DNA with restriction endonucleases. Genomic DNA fragments can be inserted into a vector, such as a bacteriophage or cosmid vector, in accordance with conventional techniques, such as the use of restriction enzyme digestion to provide appropriate termini, the use of alkaline phosphatase treatment to avoid undesirable joining of DNA molecules, and ligation with appropriate ligases. Techniques for such manipulation are well-known in the art (see, for example, Ausubel (1995) at pages 5-1 to 5-6; Wu (1997) at pages 307-327).

Alternatively, human genomic libraries can be obtained from commercial sources such as Research Genetics (Huntsville, AL) and the American Type Culture Collection (Manassas, VA).

A library containing cDNA or genomic clones can be screened with one or more polynucleotide probes, which have a nucleotide sequence based upon the amino acid sequence of SEQ ID NO:1, using standard methods (see, for example, Ausubel (1995) at pages 6-1 to 6-11).

Anti-Zace1 antibodies, produced as described below, can also be used to isolate DNA sequences that encode human *Zace1* genes from cDNA libraries. For example, the antibodies can be used to screen λgt11 expression libraries, or the antibodies can be used for immunoscreening following hybrid selection and translation (see, for example, Ausubel (1995) at pages 6-12 to 6-16; Margolis *et al.*, "Screening λ expression libraries with antibody and protein probes," in *DNA Cloning 2*: *Expression Systems, 2nd Edition,* Glover *et al.* (eds.), pages 1-14 (Oxford University Press 1995)).

The sequence of a *Zace1* cDNA or *Zace1* genomic fragment can be determined using standard methods. Promoter elements from a *Zace1* gene can be used to direct the expression of heterologous genes in transgenic animals or patients treated with gene therapy. The identification of genomic fragments containing a *Zace1* promoter or regulatory element can be achieved using well-established techniques, such as deletion analysis (see, generally, Ausubel (1995)).

Cloning of 5' flanking sequences also facilitates production of Zace1 proteins by "gene activation," as disclosed in U.S. Patent No. 5,641,670. Briefly, expression of an endogenous *Zace1* gene in a cell is altered by introducing into the *Zace1* locus a DNA construct comprising at least a targeting sequence, a regulatory sequence, an exon, and an unpaired splice donor site. The targeting sequence is a *Zace1* 5' non-coding sequence that permits homologous recombination of the construct with the endogenous *Zace1* locus, whereby the sequences within the construct become operably linked with the endogenous *Zace1* coding sequence. In this way, an endogenous *Zace1* promoter can be replaced or supplemented with other regulatory sequences to provide enhanced, tissue-specific, or otherwise regulated expression.

### 4. Production of Zace 1 Gene Variants

The present invention provides a variety of nucleic acid molecules, including DNA and RNA molecules, that encode the Zace1 polypeptides disclosed herein. Those skilled in the art will readily recognize that, in view of the degeneracy of the genetic code, considerable sequence variation is possible among these polynucleotide molecules. SEQ ID NO:2 is a degenerate nucleotide sequence that encompasses all nucleic acid molecules that encode the Zace1 polypeptide of SEQ ID NO:1. Those skilled in the art will recognize that the degenerate sequence of SEQ ID NO:2 also provides all RNA sequences encoding SEQ ID NO: 1, by substituting U for T.

Table 1 sets forth the one-letter codes used within SEQ ID NO:2 to denote degenerate nucleotide positions. "Resolutions" are the nucleotides denoted by a code letter. "Complement" indicates the code for the complementary nucleotide(s). For example, the code Y denotes either C or T, and its complement R denotes A or G, A being complementary to T, and G being complementary to C.

**Table 1**

| **Nucleotide** | **Resolution** | **Complement** | **Resolution** |
|---|---|---|---|
| A | A | T | T |
| C | C | G | G |
| G | G | C | C |
| T | T | A | A |
| R | A\|G | Y | C\|T |
| Y | C\|T | R | A\|G |
| M | A\|C | K | G\|T |
| K | G\|T | M | A\|C |
| S | C\|G | S | C\|G |
| W | A\|T | W | A\|T |
| H | A\|C\|T | D | A\|G\|T |
| B | C\|G\|T | V | A\|C\|G |
| V | A\|C\|G | B | C\|G\|T |
| D | A\|G\|T | H | A\|C\|T |
| N | A\|C\|G\|T | N | A\|C\|G\|T |

The degenerate codons used in SEQ ID NO:2, encompassing all possible codons for a given amino acid, are set forth in Table 2.

**Table 2**

| **Amino Acid** | **One Letter Code** | **Codons** | **Degenerate Codon** |
|---|---|---|---|
| Cys | C | TGC TGT | TGY |
| Ser | S | AGC AGT TCA TCC TCG TCT | WSN |
| Thr | T | ACA ACC ACG ACT | CAN |
| Pro | P | CCA CCC CCG CCT | CCN |
| Ala | A | GCA GCC GCG GCT | GCN |
| Gly | G | GGA GGC GGG GGT | GGN |
| Asn | N | AAC AAT | AAY |
| Asp | D | GAC GAT | GAY |
| Glu | E | GAA GAG | GAR |
| GIn | Q | CAA CAG | CAR |
| His | H | CAC CAT | CAY |
| Arg | R | AGA AGG CGA CGC CGG CGT | MGN |
| Lys | K | AAA AAG | AAR |
| Met | M | ATG | ATG |
| Ile | I | ATA ATC ATT | ATH |
| Leu | L | CTA CTC CTG CTT TTA TTG | YTN |
| Val | V | GTA GTC GTG GTT | GTN |
| Phe | F | TTC TTT | TTY |
| Tyr | Y | TAC TAT | TAY |
| Trp | W | TGG | TGG |
| Ter | . | TAA TAG TGA | TRR |
| Asn\|Asp | B | | RAY |
| Glu\|Gln | Z | | SAR |
| Any | X | | NNN |

One of ordinary skill in the art will appreciate that some ambiguity is introduced in determining a degenerate codon, representative of all possible codons encoding an amino acid. For example, the degenerate codon for serine (WSN) can, in some circumstances, encode arginine (AGR), and the degenerate codon for arginine (MGN) can, in some circumstances, encode serine (AGY). A similar relationship exists between codons encoding phenylalanine and leucine. Thus, some polynucleotides encompassed by the degenerate sequence may encode variant amino acid sequences, but one of ordinary skill in the art can easily identify such variant sequences by reference to the amino acid sequence of SEQ ID NO: 1. Variant sequences can be readily tested for functionality as described herein.

Different species can exhibit "preferential codon usage." In general, see, Grantham *et al., Nuc. Acids Res. 8*:1893 (1980), Haas *et al. Curr. Biol. 6*:315 (1996), Wain-Hobson *et al., Gene 13*:355 (1981), Grosjean and Fiers, *Gene 18*:199 (1982), Holm, *Nuc. Acids Res. 14*:3075 (1986), Ikemura, *J. Mol. Biol. 158*:573 (1982), Sharp and Matassi, *Curr. Opin. Genet. Dev. 4*:851 (1994), Kane, *Curr. Opin. Biotechnol. 6*:494 (1995), and Makrides, *Microbiol. Rev. 60*:512 (1996). As used herein, the term "preferential codon usage" or "preferential codons" is a term of art referring to protein translation codons that are most frequently used in cells of a certain species, thus favoring one or a few representatives of the possible codons encoding each amino acid (See Table 2). For example, the amino acid Threonine (Thr) may be encoded by ACA, ACC, ACG, or ACT, but in mammalian cells ACC is the most commonly used codon; in other species, for example, insect cells, yeast, viruses or bacteria, different Thr codons may be preferential. Preferential codons for a particular species can be introduced into the polynucleotides of the present invention by a variety of methods known in the art. Introduction of preferential codon sequences into recombinant DNA can, for example, enhance production of the protein by making protein translation more efficient within a particular cell type or species. Therefore, the degenerate codon sequence disclosed in SEQ ID NO:2 serves as a template for optimizing expression of polynucleotides in various cell types and species commonly used in the art and disclosed herein. Sequences containing preferential codons can be tested and optimized for expression in various species, and tested for functionality as disclosed herein.

The present invention further provides variant polypeptides and nucleic acid molecules that represent counterparts from other species (orthologs). These species include, but are not limited to mammalian, avian, amphibian, reptile, fish, insect and other vertebrate and invertebrate species. Of particular interest are Zace1 polypeptides from other mammalian species, including mouse, porcine, ovine, bovine, canine, feline, equine, and other primate polypeptides. Orthologs of human Zace1 can be cloned using information and compositions provided by the present invention in combination with conventional cloning techniques. For example, a *Zace1* cDNA can be cloned using mRNA obtained from a tissue or cell type that expresses the Zace1 polypeptide disclosed herein. A library is then prepared from mRNA of a positive tissue or cell line.

Those skilled in the art will recognize that the sequence disclosed in SEQ ID NO:1 represents a single allele of human *Zace1,* and that allelic variation and alternative splicing are expected to occur. Allelic variants of this sequence can be cloned by probing cDNA or genomic libraries from different individuals according to standard procedures. Allelic variants of the nucleotide sequence shown in SEQ ID NO: 1, including those containing silent mutations and those in which mutations result in amino acid sequence changes, are within the scope of the present invention, as are proteins which are allelic variants of SEQ ID NO:1. cDNA molecules generated from alternatively spliced mRNAs, which retain the properties of the Zace1 polypeptide are included within the scope of the present invention, as are polypeptides encoded by such cDNAs and mRNAs. Allelic variants and splice variants of these sequences can be cloned by probing cDNA or genomic libraries from different individuals or tissues according to standard procedures known in the art.

Isolated polynucleotides that encode the Zace1 polypeptide of SEQ ID NO:1 will hybridize to similar sized regions of a related (homologous) polynucleotide, or a sequence complementary thereto, under stringent conditions. In general, stringent conditions are selected to be about 5°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Typical stringent conditions are those in which the salt concentration is up to about 0.03 M at pH 7 and the temperature is at least about 60°C.

A pair of nucleic acid molecules, such as DNA-DNA, RNA-RNA and DNA-RNA, can hybridize if the nucleotide sequences have some degree of complementarity. Hybrids can tolerate mismatched base pairs in the double helix, but the stability of the hybrid is influenced by the degree of mismatch. The Tₘ of the mismatched hybrid decreases by 1°C for every 1-1.5% base pair mismatch. Varying the stringency of the hybridization conditions allows control over the degree of mismatch that will be present in the hybrid. The degree of stringency increases as the hybridization temperature increases and the ionic strength of the hybridization buffer decreases. Stringent hybridization conditions encompass temperatures of about 5-25°C below the Tₘ of the hybrid and a hybridization buffer having up to 1 M Na⁺. Higher degrees of stringency at lower temperatures can be achieved with the addition of formamide which reduces the Tₘ of the hybrid about 1°C for each 1% formamide in the buffer solution. Generally, such stringent conditions include temperatures of 20-70°C and a hybridization buffer containing up to 6x SSC and 0-50% formamide. A higher degree of stringency can be achieved at temperatures of from 40-70°C with a hybridization buffer having up to 4x SSC and from 0-50% formamide. Highly stringent conditions typically encompass temperatures of 42-70°C with a hybridization buffer having up to 1x SSC and 0-50% formamide. Different degrees of stringency can be used during hybridization and washing to achieve maximum specific binding to the target sequence. Typically, the washes following hybridization are performed at increasing degrees of stringency to remove non-hybridized polynucleotide probes from hybridized complexes.

The above conditions are meant to serve as a guide and it is well within the abilities of one skilled in the art to adapt these conditions for use with a particular polypeptide hybrid. The Tₘ for a specific target sequence is the temperature (under defined conditions) at which 50% of the target sequence will hybridize to a perfectly matched probe sequence. Those conditions which influence the Tₘ include, the size and base pair content of the polynucleotide probe, the ionic strength of the hybridization solution, and the presence of destabilizing agents in the hybridization solution. Numerous equations for calculating Tₘ are known in the art, and are specific for DNA, RNA and DNA-RNA hybrids and polynucleotide probe sequences of varying length (see, for example, Sambrook *et al., Molecular Cloning: A Laboratory Manual,* Second Edition (Cold Spring Harbor Press 1989); Ausubel *et al.*, (eds.), *Current Protocols in Molecular Biology (*John Wiley and Sons, Inc. 1987); Berger and Kimmel (eds.), *Guide to Molecular Cloning Techniques,* (Academic Press, Inc. 1987); and Wetmur, *Crit. Rev. Biochem. Mol. Biol. 26*:227 (1990)). Sequence analysis software such as OLIGO 6.0 (LSR; Long Lake, MN) and *Primer Premier 4.0* (Premier Biosoft International; Palo Alto, CA), as well as sites on the Internet, are available tools for analyzing a given sequence and calculating Tₘ based on user defined criteria. Such programs can also analyze a given sequence under defined conditions and identify suitable probe sequences. Typically, hybridization of longer polynucleotide sequences, >50 base pairs, is performed at temperatures of about 20-25°C below the calculated Tₘ. For smaller probes, <50 base pairs, hybridization is typically carried out at the Tₘ or 5-10°C below. This allows for the maximum rate of hybridization for DNA-DNA and DNA-RNA hybrids.

The length of the polynucleotide sequence influences the rate and stability of hybrid formation. Smaller probe sequences, <50 base pairs, reach equilibrium with complementary sequences rapidly, but may form less stable hybrids. Incubation times of anywhere from minutes to hours can be used to achieve hybrid formation. Longer probe sequences come to equilibrium more slowly, but form more stable complexes even at lower temperatures. Incubations are allowed to proceed overnight or longer. Generally, incubations are carried out for a period equal to three times the calculated Cot time. Cot time, the time it takes for the polynucleotide sequences to reassociate, can be calculated for a particular sequence by methods known in the art.

The base pair composition of polynucleotide sequence will effect the thermal stability of the hybrid complex, thereby influencing the choice of hybridization temperature and the ionic strength of the hybridization buffer. A-T pairs are less stable than G-C pairs in aqueous solutions containing sodium chloride. Therefore, the higher the G-C content, the more stable the hybrid. Even distribution of G and C residues within the sequence also contribute positively to hybrid stability. In addition, the base pair composition can be manipulated to alter the Tₘ of a given sequence. For example, 5-methyldeoxycytidine can be substituted for deoxycytidine and 5-bromodeoxuridine can be substituted for thymidine to increase the Tₘ, whereas 7-deazz-2'-deoxyguanosine can be substituted for guanosine to reduce dependence on Tₘ .

The ionic concentration of the hybridization buffer also affects the stability of the hybrid. Hybridization buffers generally contain blocking agents such as Denhardt's solution (Sigma Chemical Co., St. Louis, Mo.), denatured salmon sperm DNA, tRNA, milk powders (BLOTTO), heparin or SDS, and a Na⁺ source, such as SSC (1x SSC: 0.15 M sodium chloride, 15 mM sodium citrate) or SSPE (1x SSPE: 1.8 M NaCl, 10 mM NaH₂PO₄, 1 mM EDTA, pH 7.7). Typically, hybridization buffers contain from between 10 mM - 1 M Na⁺. The addition of destabilizing or denaturing agents such as formamide, tetralkylammonium salts, guanidinium cations or thiocyanate cations to the hybridization solution will alter the Tₘ of a hybrid. Typically, formamide is used at a concentration of up to 50% to allow incubations to be carried out at more convenient and lower temperatures. Formamide also acts to reduce non-specific background when using RNA probes.

As an illustration, a nucleic acid molecule encoding a variant Zace1 polypeptide can be hybridized with a nucleic acid molecule comprising a nucleotide sequence that encodes the amino acid sequence of SEQ ID NO: at 42°C overnight in a solution comprising 50% formamide, 5x SSC, 50 mM sodium phosphate (pH 7.6), 5x Denhardt's solution (100x Denhardt's solution: 2% (w/v) Ficoll 400, 2% (w/v) polyvinylpyrrolidone, and 2% (w/v) bovine serum albumin), 10% dextran sulfate, and 20 µg/ml denatured, sheared salmon sperm DNA. One of skill in the art can devise variations of these hybridization conditions. For example, the hybridization mixture can be incubated at a higher temperature, such as about 65°C, in a solution that does not contain formamide. Moreover, premixed hybridization solutions are available (*e.g.*, EXPRESSHYB Hybridization Solution from CLONTECH Laboratories, Inc.), and hybridization can be performed according to the manufacturer's instructions.

Following hybridization, the nucleic acid molecules can be washed to remove non-hybridized nucleic acid molecules under stringent conditions, or under highly stringent conditions. Typical stringent washing conditions include washing in a solution of 0.5x - 2x SSC with 0.1% sodium dodecyl sulfate (SDS) at 55 - 65°C. That is, nucleic acid molecules encoding a variant Zace1 polypeptide hybridize with a nucleic acid molecule comprising a nucleotide sequence that encodes the amino acid sequence of SEQ ID NO:1 under stringent washing conditions, in which the wash stringency is equivalent to 0.5x - 2x SSC with 0.1% SDS at 55 - 65°C, including 0.5x SSC with 0.1% SDS at 55°C, or 2xSSC with 0.1% SDS at 65°C. One of skill in the art can readily devise equivalent conditions, for example, by substituting SSPE for SSC in the wash solution.

Typical highly stringent washing conditions include washing in a solution of 0.1 x - 0.2x SSC with 0.1 % sodium dodecyl sulfate (SDS) at 50 - 65°C. In other words, nucleic acid molecules encoding a variant Zace1 polypeptide hybridize with a nucleic acid molecule comprising a nucleotide sequence that encodes the amino acid sequence of SEQ ID NO:1 under highly stringent washing conditions, in which the wash stringency is equivalent to 0.1x - 0.2x SSC with 0.1% SDS at 50 - 65°C, including 0.1x SSC with 0.1% SDS at 50°C, or 0.2xSSC with 0.1% SDS at 65°C.

The present invention also provides isolated Zace1 polypeptides that have a substantially similar sequence identity to the polypeptides of SEQ ID NO:1, or their orthologs. The term "substantially similar sequence identity" is used herein to denote polypeptides having at least 70%, at least 80%, at least 90%, at least 95% or greater than 95% sequence identity to the sequences shown in SEQ ID NO:1, or their orthologs.

Percent sequence identity is determined by conventional methods. See, for example, Altschul *et al., Bull. Math. Bio. 48*:603 (1986), and Henikoff and Henikoff, *Proc. Natl. Acad. Sci. USA 89*:10915 (1992). Briefly, two amino acid sequences are aligned to optimize the alignment scores using a gap opening penalty of 10, a gap extension penalty of 1, and the "BLOSUM62" scoring matrix of Henikoff and Henikoff (*ibid.*) as shown in Table 3 (amino acids are indicated by the standard one-letter codes). The percent identity is then calculated as: ([Total number of identical matches]/ [length of the longer sequence plus the number of gaps introduced into the longer sequence in order to align the two sequences])(100).

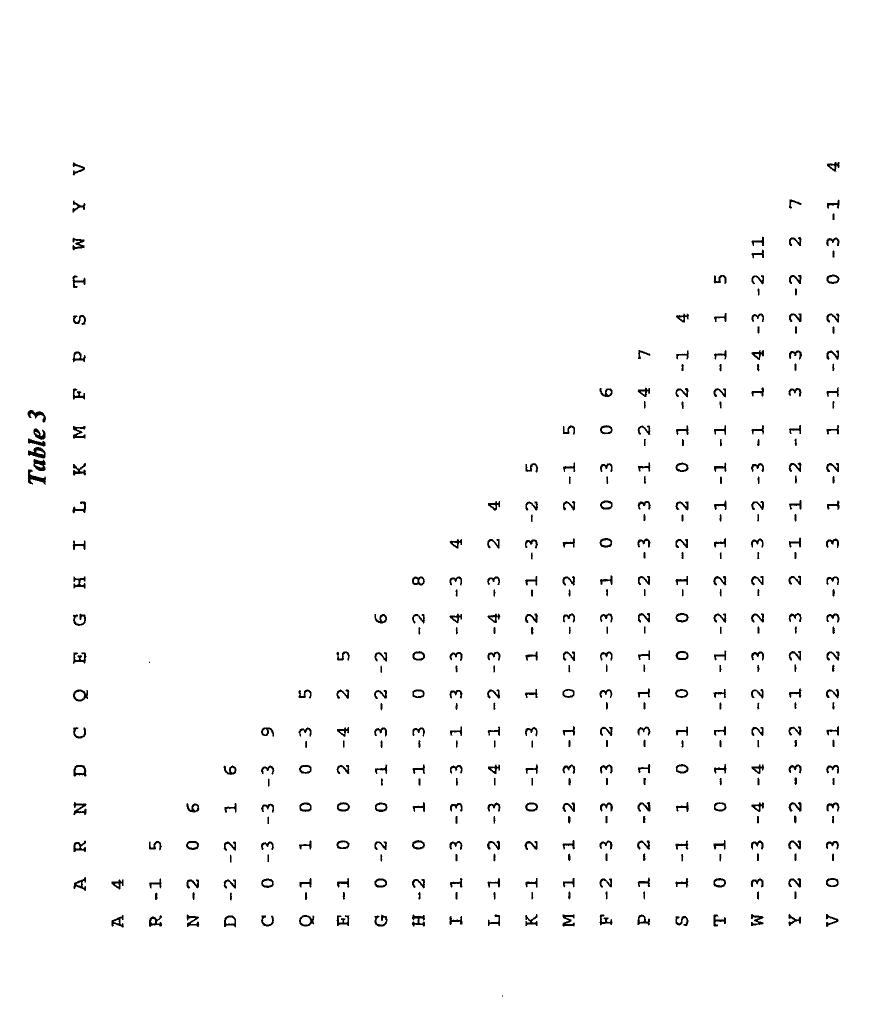

Those skilled in the art appreciate that there are many established algorithms available to align two amino acid sequences. The "FASTA" similarity search algorithm of Pearson and Lipman is a suitable protein alignment method for examining the level of identity shared by an amino acid sequence disclosed herein and the amino acid sequence of a putative Zace1 variant. The FASTA algorithm is described by Pearson and Lipman, *Proc. Nat'l Acad. Sci. USA 85*:2444 (1988), and by Pearson, *Meth. Enzymol. 183*:63 (1990). Briefly, FASTA first characterizes sequence similarity by identifying regions shared by the query sequence (e.g., SEQ ID NO: 1) and a test sequence that have either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff' value (calculated by a predetermined formula based upon the length of the sequence and the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm (Needleman and Wunsch, *J. Mol. Biol. 48*:444 (1970); Sellers, *SIAM J. Appl. Math. 26*:787 (1974)), which allows for amino acid insertions and deletions. Illustrative parameters for FASTA analysis are: ktup=1, gap opening penalty=10, gap extension penalty=1 and substitution matrix=BLOSUM62. These parameters can be introduced into a FASTA program by modifying the scoring matrix file ("SMATRIX"), as explained in Appendix 2 of Pearson, *Meth. Enzymol. 183*:63 (1990).

FASTA can also be used to determine the sequence identity of nucleic acid molecules using a ratio as disclosed above. For nucleotide sequence comparisons, the ktup value can range between one to six, preferably from four to six.

The present invention includes polypeptides having one or more conservative amino acid changes, compared with the amino acid sequence of SEQ ID NO:1. That is, variants can be obtained that contain one or more amino acid substitutions of SEQ ID NO:1, in which an alkyl amino acid is substituted for an alkyl amino acid in a Zace1 amino acid sequence, an aromatic amino acid is substituted for an aromatic amino acid in a Zace 1 amino acid sequence, a sulfur-containing amino acid is substituted for a sulfur-containing amino acid in a Zace1 amino acid sequence, a hydroxy-containing amino acid is substituted for a hydroxy-containing amino acid in a Zace1 amino acid sequence, an acidic amino acid is substituted for an acidic amino acid in a Zace1 amino acid sequence, a basic amino acid is substituted for a basic amino acid in a Zace1 amino acid sequence, or a dibasic monocarboxylic amino acid is substituted for a dibasic monocarboxylic amino acid in a Zace1 amino acid sequence. Among the common amino acids, for example, a "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine.

The BLOSUM62 table is an amino acid substitution matrix derived from about 2,000 local multiple alignments of protein sequence segments, representing highly conserved regions of more than 500 groups of related proteins (Henikoff and Henikoff, *Proc. Nat'l Acad. Sci. USA 89*:10915 (1992)). Accordingly, the BLOSUM62 substitution frequencies can be used to define conservative amino acid substitutions that may be introduced into the amino acid sequences of the present invention. Although it is possible to design amino acid substitutions based solely upon chemical properties (as discussed above), the language "conservative amino acid substitution" preferably refers to a substitution represented by a BLOSUM62 value of greater than -1. For example, an amino acid substitution is conservative if the substitution is characterized by a BLOSUM62 value of 0, 1, 2, or 3. According to this system, preferred conservative amino acid substitutions are characterized by a BLOSUM62 value of at least 1 (*e.g.*, 1, 2 or 3), while more preferred conservative amino acid substitutions are characterized by a BLOSUM62 value of at least 2 (*e.g.*, 2 or 3).

Particular variants of Zace1 are characterized by having at least 70%, at least 80%, at least 90%, at least 95% or greater than 95% sequence identity to the corresponding amino acid sequence (*i.e.*, SEQ ID NO:1), wherein the variation in amino acid sequence is due to one or more conservative amino acid substitutions.

Conservative amino acid changes in a *Zace1* gene can be introduced, for example, by nucleotide substitution into a nucleotide sequence that encodes the amino acid sequence SEQ ID NO:1. Such "conservative amino acid" variants can be obtained, for example, by oligonucleotide-directed mutagenesis, linker-scanning mutagenesis, mutagenesis using the polymerase chain reaction, and the like (see Ausubel (1995) at pages 8-10 to 8-22; and McPherson (ed.), *Directed Mutagenesis: A Practical Approach* (IRL Press 1991)). A variant Zace1 polypeptide can be identified by the ability to specifically bind anti-Zace1 antibodies.

The proteins of the present invention can also comprise non-naturally occurring amino acid residues. Non-naturally occurring amino acids include, without limitation, *trans*-3-methylproline, 2,4-methanoproline, *cis*-4-hydroxyproline, *trans-4-* hydroxyproline, N-methylglycine, allo-threonine, methylthreonine, hydroxyethylcysteine, hydroxyethylhomocysteine, nitroglutamine, homoglutamine, pipecolic acid, thiazolidine carboxylic acid, dehydroproline, 3- and 4-methylproline, 3,3-dimethylproline, tert-leucine, norvaline, 2-azaphenylalanine, 3-azaphenylalanine, 4-azaphenylalanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is typically carried out in a cell-free system comprising an *E. coli* S30 extract and commercially available enzymes and other reagents. Proteins are purified by chromatography. See, for example, *Robertson et al., J. Am. Chem. Soc. 113*:2722 (1991), Ellman *et al., Methods Enzymol. 202*:301 (1991), Chung *et al., Science 259*:806 (1993), and Chung *et al., Proc. Nat'l Acad Sci. USA 90*:10145 (1993).

In a second method, translation is carried out in *Xenopus* oocytes by microinjection of mutated mRNA and chemically aminoacylated suppressor tRNAs (Turcatti *et al., J. Biol. Chem. 271*:19991 (1996)). Within a third method, *E. coli* cells are cultured in the absence of a natural amino acid that is to be replaced (*e.g.*, phenylalanine) and in the presence of the desired non-naturally occurring amino acid(s) (*e.g.*, 2-azaphenylalanine, 3-azaphenylalanine, 4-azaphenylalanine, or 4-fluorophenylalanine). The non-naturally occurring amino acid is incorporated into the protein in place of its natural counterpart. See, Koide *et al., Biochem. 33*:7470 (1994). Naturally occurring amino acid residues can be converted to non-naturally occurring species by *in vitro* chemical modification. Chemical modification can be combined with site-directed mutagenesis to further expand the range of substitutions (Wynn and Richards, *Protein Sci. 2*:395 (1993)).

A limited number of non-conservative amino acids, amino acids that are not encoded by the genetic code, non-naturally occurring amino acids, and unnatural amino acids may be substituted for Zace1 amino acid residues.

Essential amino acids in the polypeptides of the present invention can be identified according to procedures known in the art, such as site-directed mutagenesis or alanine-scanning mutagenesis (Cunningham and Wells, *Science 244*:1081 (1989), Bass *et al., Proc. Nat'l Acad. Sci. USA 88*:4498 (1991), Coombs and Corey, "Site-Directed Mutagenesis and Protein Engineering," in *Proteins: Analysis and Design,* Angeletti (ed.), pages 259-311 (Academic Press, Inc. 1998)). In the latter technique, single alanine mutations are introduced at every residue in the molecule, and the resultant mutant molecules are tested for biological activity to identify amino acid residues that are critical to the activity of the molecule. See also, *Hilton et al., J. Biol. Chem. 271*:4699 (1996).

As discussed above, amino acid sequence analysis indicates that the following amino acids play a role in Zace1 enzymatic activity: His³⁹⁸, His⁴⁰², and Glu⁴²⁶. Although sequence analysis can be used to further define the Zace1 active site, domains that play a role in Zace1 activity can also be determined by physical analysis of structure, as determined by such techniques as nuclear magnetic resonance, crystallography, electron diffraction or photoaffinity labeling, in conjunction with mutation of putative contact site amino acids. See, for example, de Vos *et al., Science 255*:306 (1992), Smith *et al., J. Mol. Biol. 224*:899 (1992), and Wlodaver *et al., FEBS Lett. 309*:59 (1992).

Multiple amino acid substitutions can be made and tested using known methods of mutagenesis and screening, such as those disclosed by Reidhaar-Olson and Sauer (*Science 241*:53 (1988)) or Bowie and Sauer (*Proc. Nat'l Acad. Sci. USA 86*:2152 (1989)). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (*e.g.*, Lowman *et al., Biochem. 30*:10832 (1991), Ladner *et al.,* U.S. Patent No. 5,223,409, Huse, international publication No. WO 92/06204, and region-directed mutagenesis (Derbyshire *et al., Gene 46*:145 (1986), and Ner *et al., DNA 7*:127, (1988)).

Variants of the disclosed Zace1 nucleotide and polypeptide sequences can also be generated through DNA shuffling as disclosed by Stemmer, *Nature 370*:389 (1994), Stemmer, *Proc. Nat'l Acad. Sci. USA 91*:10747 (1994), and international publication No. WO 97/20078. Briefly, variant DNA molecules are generated by *in vitro* homologous recombination by random fragmentation of a parent DNA followed by reassembly using PCR, resulting in randomly introduced point mutations. This technique can be modified by using a family of parent DNA molecules, such as allelic variants or DNA molecules from different species, to introduce additional variability into the process. Selection or screening for the desired activity, followed by additional iterations of mutagenesis and assay provides for rapid "evolution" of sequences by selecting for desirable mutations while simultaneously selecting against detrimental changes.

Mutagenesis methods as disclosed herein can be combined with high-throughput, automated screening methods to detect activity of cloned, mutagenized polypeptides in host cells. Mutagenized DNA molecules that encode biologically active polypeptides, or polypeptides that bind with anti-Zace1 antibodies, can be recovered from the host cells and rapidly sequenced using modem equipment. These methods allow the rapid determination of the importance of individual amino acid residues in a polypeptide of interest, and can be applied to polypeptides of unknown structure.

The present invention also includes "functional fragments" of Zace1 polypeptides and nucleic acid molecules encoding such functional fragments. Functional analysis of the amino acid sequence described herein can be performed using standard techniques. For example, studies on the truncation at either or both termini of interferons have been summarized by Horisberger and Di Marco, *Pharmac. Ther. 66*:507 (1995). Moreover, standard techniques for functional analysis of proteins are described by, for example, Treuter *et al., Molec. Gen. Genet. 240*:113 (1993), Content *et al.*, "Expression and preliminary deletion analysis of the 42 kDa 2-5A synthetase induced by human interferon," in *Biological Interferon Systems, Proceedings of ISIR-TNO Meeting on Interferon Systems,* Cantell (ed.), pages 65-72 (Nijhoff 1987), Herschman, "The EGF Receptor," in *Control of Animal Cell Proliferation, Vol. 1,* Boynton *et al.,* (eds.) pages 169-199 (Academic Press 1985), Coumailleau *et al., J. Biol. Chem. 270*:29270 (1995); Fukunaga *et al., J. Biol. Chem. 270*:25291 (1995); Yamaguchi *et al., Biochem. Pharmacol. 50*:1295 (1995), and Meisel *et al., Plant Molec. Biol. 30*:1 (1996).

Illustrative functional fragments include polypeptides, comprising amino acid residues 367 to 430 of SEQ ID NO:1, amino acid residues 163 to 563 of SEQ ID NO:1, amino acid residues 52 to 563 of SEQ ID NO:1, amino acid residues 52 to 644 of SEQ ID NO:1, amino acid residues 52 to 648 of SEQ ID NO: 1, amino acid residues 52 to 655 of SEQ ID NO:1, amino acid residues 52 to 662 of SEQ ID NO:1, amino acid residues 52 to 682 of SEQ ID NO: 1, or amino acid residues 52 to 694 of SEQ ID NO:1, and the like. Particular functional fragments of a Zace1 polypeptide are soluble forms of Zace1 that lack a transmembrane domain. Illustrative Zace1 soluble forms include polypeptides consisting of amino acid residues 1 to 662 of SEQ ID NO:1, amino acid residues 52 to 662 of SEQ ID NO:1, and the like.

The present invention also contemplates functional fragments of a *Zace1* gene that have amino acid changes, compared with the amino acid sequence of SEQ ID NO:1. A variant *Zace1* gene can be identified on the basis of structure by determining the level of identity with the amino acid sequence of SEQ ID NO:1, as discussed above.

The present invention also provides polypeptide fragments or peptides comprising an epitope-bearing portion of a Zace1 polypeptide described herein. Such fragments or peptides may comprise an "immunogenic epitope," which is a part of a protein that elicits an antibody response when the entire protein is used as an immunogen. Immunogenic epitope-bearing peptides can be identified using standard methods (see, for example, Geysen *et al., Proc. Nat'l Acad. Sci. USA 81*:3998 (1983)).

In contrast, polypeptide fragments or peptides may comprise an "antigenic epitope," which is a region of a protein molecule to which an antibody can specifically bind. Certain epitopes consist of a linear or contiguous stretch of amino acids, and the antigenicity of such an epitope is not disrupted by denaturing agents. It is known in the art that relatively short synthetic peptides that can mimic epitopes of a protein can be used to stimulate the production of antibodies against the protein (see, for example, Sutcliffe *et al., Science 219*:660 (1983)). Accordingly, antigenic epitope-bearing peptides and polypeptides of the present invention are useful to raise antibodies that bind with the polypeptides described herein.

Antigenic epitope-bearing peptides and polypeptides preferably contain at least four to ten amino acids, at least ten to fifteen amino acids, or about 15 to about 30 amino acids of SEQ ID NO:1. Such epitope-bearing peptides and polypeptides can be produced by fragmenting a Zace1 polypeptide, or by chemical peptide synthesis, as described herein. Moreover, epitopes can be selected by phage display of random peptide libraries (see, for example, Lane and Stephen, *Curr. Opin. Immunol. 5*:268 (1993), and Cortese *et al., Curr. Opin. Biotechnol. 7*:616 (1996)). Standard methods for identifying epitopes and producing antibodies from small peptides that comprise an epitope are described, for example, by Mole, "Epitope Mapping," in *Methods in Molecular Biology, Vol. 10,* Manson (ed.), pages 105-116 (The Humana Press, Inc. 1992), Price, "Production and Characterization of Synthetic Peptide-Derived Antibodies," in *Monoclonal Antibodies: Production, Engineering, and Clinical Application,* Ritter and Ladyman (eds.), pages 60-84 (Cambridge University Press 1995), and Coligan *et al.* (eds.), *Current Protocols in Immunology,* pages 9.3.1 - 9.3.5 and pages 9.4.1 - 9.4.11 (John Wiley & Sons 1997).

For any Zace1 polypeptide, including variants and fusion proteins, one of ordinary skill in the art can readily generate a fully degenerate polynucleotide sequence encoding that variant using the information set forth in Tables 1 and 2 above. Moreover, those of skill in the art can use standard software to devise Zace1 variants based upon the nucleotide and amino acid sequences described herein. Accordingly, the present invention includes a computer-readable medium encoded with a data structure that provides at least one of the following sequences: SEQ ID NO: 1, and SEQ ID NO:2. Suitable forms of computer-readable media include magnetic media and optically-readable media. Examples of magnetic media include a hard or fixed drive, a random access memory (RAM) chip, a floppy disk, digital linear tape (DLT), a disk cache, and a ZIP disk. Optically readable media are exemplified by compact discs (*e.g.,* CD-read only memory (ROM), CD-rewritable (RW), and CD-recordable), and digital versatile/video discs (DVD) (*e.g*., DVD-ROM. DVD-RAM, and DVD+RW).

### 5. Production of Zace1 Polypeptides

The polypeptides of the present invention, including full-length polypeptides, functional fragments, and fusion proteins, can be produced in recombinant host cells following conventional techniques. To express a *Zace1* gene, a nucleic acid molecule encoding the polypeptide must be operably linked to regulatory sequences that control transcriptional expression in an expression vector and then, introduced into a host cell. In addition to transcriptional regulatory sequences, such as promoters and enhancers, expression vectors can include translational regulatory sequences and a marker gene which is suitable for selection of cells that carry the expression vector.

Expression vectors that are suitable for production of a foreign protein in eukaryotic cells typically contain (1) prokaryotic DNA elements coding for a bacterial replication origin and an antibiotic resistance marker to provide for the growth and selection of the expression vector in a bacterial host; (2) eukaryotic DNA elements that control initiation of transcription, such as a promoter; and (3) DNA elements that control the processing of transcripts, such as a transcription termination/polyadenylation sequence. As discussed above, expression vectors can also include nucleotide sequences encoding a secretory sequence that directs the heterologous polypeptide into the secretory pathway of a host cell. For example, a *Zace1* expression vector may comprise a *Zace1* gene and a secretory sequence derived from any secreted gene.

Zace1 proteins of the present invention may be expressed in mammalian cells. Examples of suitable mammalian host cells include African green monkey kidney cells (Vero; ATCC CRL 1587), human embryonic kidney cells (293-HEK; ATCC CRL 1573), baby hamster kidney cells (BHK-21, BHK-570; ATCC CRL 8544, ATCC CRL 10314), canine kidney cells (MDCK; ATCC CCL 34), Chinese hamster ovary cells (CHO-K1; ATCC CCL61; CHO DG44 (*Chasin et al., Som. Cell. Molec. Genet. 12*:555, 1986)), rat pituitary cells (GH1; ATCC CCL82), HeLa S3 cells (ATCC CCL2.2), rat hepatoma cells (H-4-II-E; ATCC CRL 1548) SV40-transformed monkey kidney cells (COS-1; ATCC CRL 1650) and murine embryonic cells (NIH-3T3; ATCC CRL 1658).

For a mammalian host, the transcriptional and translational regulatory signals may be derived from viral sources, such as adenovirus, bovine papilloma virus, simian virus, or the like, in which the regulatory signals are associated with a particular gene which has a high level of expression. Suitable transcriptional and translational regulatory sequences also can be obtained from mammalian genes, such as actin, collagen, myosin, and metallothionein genes.

Transcriptional regulatory sequences include a promoter region sufficient to direct the initiation of RNA synthesis. Suitable eukaryotic promoters include the promoter of the mouse *metallothionein I* gene (Hamer *et al., J. Molec. Appl. Genet. 1*:273 (1982)), the *TK* promoter of *Herpes* virus (McKnight, *Cell 31*:355 (1982)), the *SV40* early promoter (Benoist *et al., Nature 290*:304 (1981)), the *Rous* sarcoma virus promoter (Gorman *et al., Proc. Nat'l Acad. Sci. USA 79*:6777 (1982)), the cytomegalovirus promoter (Foecking *et al., Gene 45*:101 (1980)), and the mouse mammary tumor virus promoter (see, generally, Etcheverry, "Expression of Engineered Proteins in Mammalian Cell Culture," in *Protein Engineering: Principles and Practice,* Cleland *et al.* (eds.), pages 163-181 (John Wiley & Sons, Inc. 1996)).

Alternatively, a prokaryotic promoter, such as the bacteriophage T3 RNA polymerase promoter, can be used to control *Zace1* gene expression in mammalian cells if the prokaryotic promoter is regulated by a eukaryotic promoter (Zhou *et al., Mol. Cell. Biol. 10*:4529 (1990), and Kaufman *et al., Nucl. Acids Res. 19*:4485 (1991)).

An expression vector can be introduced into host cells using a variety of standard techniques including calcium phosphate transfection, liposome-mediated transfection, microprojectile-mediated delivery, electroporation, and the like. Preferably, the transfected cells are selected and propagated to provide recombinant host cells that comprise the expression vector stably integrated in the host cell genome. Techniques for introducing vectors into eukaryotic cells and techniques for selecting such stable transformants using a dominant selectable marker are described, for example, by Ausubel (1995) and by Murray (ed.), *Gene Transfer and Expression Protocols* (Humana Press 1991).

For example, one suitable selectable marker is a gene that provides resistance to the antibiotic neomycin. In this case, selection is carried out in the presence of a neomycin-type drug, such as G-418 or the like. Selection systems can also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. A preferred amplifiable selectable marker is dihydrofolate reductase, which confers resistance to methotrexate. Other drug resistance genes (*e.g.*, hygromycin resistance, multi-drug resistance, puromycin acetyltransferase) can also be used. Alternatively, markers that introduce an altered phenotype, such as green fluorescent protein, or cell surface proteins such as CD4, CD8, Class I MHC, placental alkaline phosphatase may be used to sort transfected cells from untransfected cells by such means as FACS sorting or magnetic bead separation technology.

Zace1 polypeptides can also be produced by cultured mammalian cells using a viral delivery system. Exemplary viruses for this purpose include adenovirus, herpesvirus, vaccinia virus and adeno-associated virus (AAV). Adenovirus, a double-stranded DNA virus, is currently the best studied gene transfer vector for delivery of heterologous nucleic acid (for a review, see Becker *et al., Meth. Cell Biol. 43*:161 (1994), and Douglas and Curiel, *Science & Medicine 4*:44 (1997)). Advantages of the adenovirus system include the accommodation of relatively large DNA inserts, the ability to grow to high-titer, the ability to infect a broad range of mammalian cell types, and flexibility that allows use with a large number of available vectors containing different promoters.

By deleting portions of the adenovirus genome, larger inserts (up to 7 kb) of heterologous DNA can be accommodated. These inserts can be incorporated into the viral DNA by direct ligation or by homologous recombination with a co-transfected plasmid. An option is to delete the essential *E1* gene from the viral vector, which results in the inability to replicate unless the *E1* gene is provided by the host cell. Adenovirus vector-infected human 293 cells (ATCC Nos. CRL-1573, 45504, 45505), for example, can be grown as adherent cells or in suspension culture at relatively high cell density to produce significant amounts of protein (see Garnier *et al., Cytotechnol. 15*:145 (1994)).

Zace1 can also be expressed in other higher eukaryotic cells, such as avian, fungal, insect, yeast, or plant cells. The baculovirus system provides an efficient means to introduce cloned *Zace1* genes into insect cells. Suitable expression vectors are based upon the *Autographa californica* multiple nuclear polyhedrosis virus (AcMNPV), and contain well-known promoters such as *Drosophila heat shock protein* (*hsp*) 70 promoter, *Autographa californica nuclear polyhedrosis virus immediate-early* gene promoter (*ie-1*) and the *delayed early 39K* promoter, baculovirus *p10* promoter, and the *Drosophila metallothionein* promoter. A second method of making recombinant baculovirus utilizes a transposon-based system described by Luckow (Luckow, *et al., J. Virol. 67*:4566 (1993)). This system, which utilizes transfer vectors, is sold in the BAC-to-BAC kit (Life Technologies, Rockville, MD). This system utilizes a transfer vector, PFASTBAC (Life Technologies) containing a Tn7 transposon to move the DNA encoding the Zace1 polypeptide into a baculovirus genome maintained in *E. coli* as a large plasmid called a "bacmid." See, Hill-Perkins and Possee, *J. Gen. Virol. 71*:971 (1990), Bonning, *et al., J. Gen. Virol. 75*:1551 (1994), and Chazenbalk, and Rapoport, *J. Biol. Chem. 270*:1543 (1995). In addition, transfer vectors can include an in-frame fusion with DNA encoding an epitope tag at the C- or N-terminus of the expressed Zace1 polypeptide, for example, a Glu-Glu epitope tag (Grussenmeyer *et al., Proc. Nat'l Acad. Sci. 82*:7952 (1985)). Using a technique known in the art, a transfer vector containing a *Zace1* gene is transformed into *E. coli,* and screened for bacmids which contain an interrupted *lacZ* gene indicative of recombinant baculovirus. The bacmid DNA containing the recombinant baculovirus genome is then isolated using common techniques.

The illustrative PFASTBAC vector can be modified to a considerable degree. For example, the polyhedrin promoter can be removed and substituted with the baculovirus basic protein promoter (also known as P*cor*, p6.9 or MP promoter) which is expressed earlier in the baculovirus infection, and has been shown to be advantageous for expressing secreted proteins (see, for example, Hill-Perkins and Possee, *J. Gen. Virol. 71*:971 (1990), Bonning, *et al., J. Gen. Virol. 75*:1551 (1994), and Chazenbalk and Rapoport, *J. Biol. Chem. 270*:1543 (1995). In such transfer vector constructs, a short or long version of the basic protein promoter can be used. Moreover, transfer vectors can be constructed which replace the native Zace1 secretory signal sequences with secretory signal sequences derived from insect proteins. For example, a secretory signal sequence from Ecdysteroid Glucosyltransferase (EGT), honey bee Melittin (Invitrogen Corporation; Carlsbad, CA), or baculovirus gp67 (PharMingen: San Diego, CA) can be used in constructs to replace the native Zace1 secretory signal sequence.

The recombinant virus or bacmid is used to transfect host cells. Suitable insect host cells include cell lines derived from IPLB-*Sf*-21, a *Spodoptera frugiperda* pupal ovarian cell line, such as Sƒ9 (ATCC CRL 1711), Sƒ21AE, and S*ƒ*21 (Invitrogen Corporation; San Diego, CA), as well as *Drosophila* Schneider-2 cells, and the HIGH FIVEO cell line (Invitrogen) derived from *Trichoplusia ni* (U.S. Patent No. 5,300,435). Commercially available serum-free media can be used to grow and to maintain the cells. Suitable media are Sf900 II™ (Life Technologies) or ESF 921™ (Expression Systems) for the Sf9 cells; and Ex-cellO405™ (JRH Biosciences, Lenexa, KS) or Express FiveO™ (Life Technologies) for the *T. ni* cells. When recombinant virus is used, the cells are typically grown up from an inoculation density of approximately 2-5 x 10⁵ cells to a density of 1-2 x 10⁶ cells at which time a recombinant viral stock is added at a multiplicity of infection (MOI) of 0.1 to 10, more typically near 3.

Established techniques for producing recombinant proteins in baculovirus systems are provided by Bailey *et al* , "Manipulation of Baculovirus Vectors," in *Methods in Molecular Biology. Volume 7*: *Gene Transfer and Expression Protocols,* Murray (ed.), pages 147-168 (The Humana Press, Inc. 1991), by Patel *et al.,* "The baculovirus expression system," in *DNA Cloning 2*: *Expression Systems, 2nd Edition,* Glover *et al.* (eds.), pages 205-244 (Oxford University Press 1995), by Ausubel (1995) at pages 16-37 to 16-57, by Richardson (ed.), *Baculovirus Expression Protocols* (The Humana Press, Inc. 1995), and by Lucknow, "Insect Cell Expression Technology," in *Protein Engineering: Principles and Practice,* Cleland *et al.* (eds.), pages 183-218 (John Wiley & Sons, Inc. 1996).

Fungal cells, including yeast cells, can also be used to express the genes described herein. Yeast species of particular interest in this regard include *Saccharomyces cerevisiae, Pichia pastoris,* and *Pichia methanolica.* Suitable promoters for expression in yeast include promoters from *GAL1* (galactose), *PGK* (phosphoglycerate kinase), *ADH* (alcohol dehydrogenase), *AOX1* (alcohol oxidase), HIS4 (histidinol dehydrogenase), and the like. Many yeast cloning vectors have been designed and are readily available. These vectors include YIp-based vectors, such as YIp5, YRp vectors, such as YRp17, YEp vectors such as YEp13 and YCp vectors, such as YCp19. Methods for transforming *S. cerevisiae* cells with exogenous DNA and producing recombinant polypeptides therefrom are disclosed by, for example, Kawasaki, U.S. Patent No. 4,599,311, Kawasaki *et al.*, U.S. Patent No. 4,931,373, Brake, U.S. Patent No. 4,870,008, Welch *et al.,* U.S. Patent No. 5,037,743, and Murray *et al.,* U.S. Patent No. 4,845,075. Transformed cells are selected by phenotype determined by the selectable marker, commonly drug resistance or the ability to grow in the absence of a particular nutrient (e.g., leucine). A preferred vector system for use in *Saccharomyces cerevisiae* is the *POTI* vector system disclosed by Kawasaki *et al.* (U.S. Patent No. 4,931,373), which allows transformed cells to be selected by growth in glucose-containing media. Additional suitable promoters and terminators for use in yeast include those from glycolytic enzyme genes (see, e.g., Kawasaki, U.S. Patent No. 4,599,311, Kingsman *et al*., U.S. Patent No. 4,615,974, and Bitter, U.S. Patent No. 4,977,092) and alcohol dehydrogenase genes. See also U.S. Patents Nos. 4,990,446, 5,063,154, 5,139,936, and 4,661,454.

Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii* and *Candida maltosa* are known in the art. See, for example, Gleeson *et al., J. Gen. Microbiol. 132*:3459 (1986), and Cregg, U.S. Patent No. 4,882,279. *Aspergillus* cells may be utilized according to the methods of McKnight *et al.*, U.S. Patent No. 4,935,349. Methods for transforming *Acremonium chrysogenum* are disclosed by Sumino *et al.,* U.S. Patent No. 5,162,228. Methods for transforming *Neurospora* are disclosed by Lambowitz, U.S. Patent No. 4,486,533.

For example, the use of *Pichia methanolica* as host for the production of recombinant proteins is disclosed by Raymond, U.S. Patent No. 5,716,808, Raymond, U.S. Patent No. 5,736,383, Raymond *et al., Yeast 14*:11-23 (1998), and in international publication Nos. WO 97/17450, WO 97/17451, WO 98/02536, and WO 98/02565. DNA molecules for use in transforming *P. methanolica* will commonly be prepared as double-stranded, circular plasmids, which are preferably linearized prior to transformation. For polypeptide production in *P. methanolica,* it is preferred that the promoter and terminator in the plasmid be that of a *P. methanolica* gene, such as a *P. methanolica* alcohol utilization gene (*AUG1* or *AUG2*). Other useful promoters include those of the dihydroxyacetone synthase (DHAS), formate dehydrogenase (FMD), and catalase (CAT) genes. To facilitate integration of the DNA into the host chromosome, it is preferred to have the entire expression segment of the plasmid flanked at both ends by host DNA sequences. A preferred selectable marker for use in *Pichia methanolica* is a *P. methanolica ADE2* gene, which encodes phosphoribosyl-5-aminoimidazole carboxylase (AIRC; EC 4.1.1.21), and which allows *ade2* host cells to grow in the absence of adenine. For large-scale, industrial processes where it is desirable to minimize the use of methanol, it is preferred to use host cells in which both methanol utilization genes (*AUG1* and *AUG2*) are deleted. For production of secreted proteins, host cells deficient in vacuolar protease genes (*PEP4* and *PRB1*) are preferred. Electroporation is used to facilitate the introduction of a plasmid containing DNA encoding a polypeptide of interest into *P. methanolica* cells. *P. methanolica* cells can be transformed by electroporation using an exponentially decaying, pulsed electric field having a field strength of from 2.5 to 4.5 kV/cm, preferably about 3.75 kV/cm, and a time constant (t) of from 1 to 40 milliseconds, most preferably about 20 milliseconds.

Expression vectors can also be introduced into plant protoplasts, intact plant tissues, or isolated plant cells. Methods for introducing expression vectors into plant tissue include the direct infection or co-cultivation of plant tissue with *Agrobacterium tumefaciens,* microprojectile-mediated delivery, DNA injection, electroporation, and the like. See, for example, Horsch *et al., Science 227*:1229 (1985), Klein *et al., Biotechnology 10*:268 (1992), and Miki *et al.,* "Procedures for Introducing Foreign DNA into Plants," in *Methods in Plant Molecular Biology and Biotechnology,* Glick *et al.* (eds.), pages 67-88 (CRC Press, 1993).

Alternatively, *Zace1* genes can be expressed in prokaryotic host cells. Suitable promoters that can be used to express Zace1 polypeptides in a prokaryotic host are well-known to those of skill in the art and include promoters capable of recognizing the T4, T3, Sp6 and T7 polymerases, the P_{R} and P_{L} promoters of bacteriophage lambda, the *trp, recA,* heat shock, *lac UV5, tac, lpp-lacSpr, phoA,* and *lacZ* promoters of *E. coli,* promoters of *B. subtilis,* the promoters of the bacteriophages of *Bacillus, Streptomyces* promoters, the *int* promoter of bacteriophage lambda, the *bla* promoter of pBR322, and the CAT promoter of the chloramphenicol acetyl transferase gene. Prokaryotic promoters have been reviewed by Glick, *J. Ind. Microbiol. 1*:277 (1987), Watson *et al., Molecular Biology of the Gene, 4th Ed.* (Benjamin Cummins 1987), and by Ausubel *et al.* (1995).

Preferred prokaryotic hosts include *E. coli* and *Bacillus subtilus.* Suitable strains of *E. coli* include BL21(DE3), BL21(DE3)pLysS, BL21(DE3)pLysE, DH1, DH4I, DH5, DH5I, DHSIF', DH5IMCR, DH10B, DH10B/p3, DH11S, C600, HB101, JM101, JM105, JM109, JM110, K38, RR1, Y1088, Y1089, CSH18, ER1451, and ER1647 (see, for example, Brown (ed.), *Molecular Biology Labfax* (Academic Press 1991)). Suitable strains of *Bacillus subtilus* include BR151, YB886, MI119, MI120, and B170 (see, for example, Hardy, "Bacillus Cloning Methods," in *DNA Cloning: A Practical Approach,* Glover (ed.) (IRL Press 1985)).

When expressing a Zace1 polypeptide in bacteria such as *E. coli,* the polypeptide may be retained in the cytoplasm, typically as insoluble granules, or may be directed to the periplasmic space by a bacterial secretion sequence. In the former case, the cells are lysed, and the granules are recovered and denatured using, for example, guanidine isothiocyanate or urea. The denatured polypeptide can then be refolded and dimerized by diluting the denaturant, such as by dialysis against a solution of urea and a combination of reduced and oxidized glutathione, followed by dialysis against a buffered saline solution. In the latter case, the polypeptide can be recovered from the periplasmic space in a soluble and functional form by disrupting the cells (by, for example, sonication or osmotic shock) to release the contents of the periplasmic space and recovering the protein, thereby obviating the need for denaturation and refolding.

Methods for expressing proteins in prokaryotic hosts are well-known to those of skill in the art (see, for example, Williams *et al*., "Expression of foreign proteins in *E. coli* using plasmid vectors and purification of specific polyclonal antibodies," in *DNA Cloning 2*: *Expression Systems, 2nd Edition,* Glover *et al.* (eds.), page 15 (Oxford University Press 1995), Ward *et al*., "Genetic Manipulation and Expression of Antibodies," in *Monoclonal Antibodies: Principles and Applications,* page 137 (Wiley-Liss, Inc. 1995), and Georgiou, "Expression of Proteins in Bacteria," in *Protein Engineering: Principles and Practice,* Cleland *et al.* (eds.), page 101 (John Wiley & Sons, Inc. 1996)).

Standard methods for introducing expression vectors into bacterial, yeast, insect, and plant cells are provided, for example, by Ausubel (1995).

General methods for expressing and recovering foreign protein produced by a mammalian cell system are provided by, for example, Etcheverry, "Expression of Engineered Proteins in Mammalian Cell Culture," in *Protein Engineering: Principles and Practice,* Cleland *et al.* (eds.), pages 163 (Wiley-Liss, Inc. 1996). Standard techniques for recovering protein produced by a bacterial system is provided by, for example, Grisshammer *et al.,* "Purification of over-produced proteins from *E. coli* cells," in *DNA Cloning 2*: *Expression Systems, 2nd Edition,* Glover *et al.* (eds.), pages 59-92 (Oxford University Press 1995). Established methods for isolating recombinant proteins from a baculovirus system are described by Richardson (ed.), *Baculovirus Expression Protocols* (The Humana Press, Inc. 1995).

As an alternative, polypeptides of the present invention can be synthesized by exclusive solid phase synthesis, partial solid phase methods, fragment condensation or classical solution synthesis. These synthesis methods are well-known to those of skill in the art (see, for example, Merrifield, *J. Am. Chem. Soc. 85*:2149 (1963), Stewart *et al.*, "Solid Phase Peptide Synthesis" (2nd Edition), (Pierce Chemical Co. 1984), Bayer and Rapp, *Chem. Pept. Prot. 3*:3 (1986), Atherton *et al., Solid Phase Peptide Synthesis: A Practical Approach* (IRL Press 1989), Fields and Colowick, "Solid-Phase Peptide Synthesis," *Methods in Enzymology Volume 289* (Academic Press 1997), and Lloyd-Williams *et al., Chemical Approaches to the Synthesis of Peptides and Proteins* (CRC Press, Inc. 1997)). Variations in total chemical synthesis strategies, such as "native chemical ligation" and "expressed protein ligation" are also standard (see, for example, Dawson *et al., Science 266*:776 (1994), Hackeng *et al., Proc. Nat'l Acad. Sci. USA 94*:7845 (1997), Dawson, *Methods Enzymol. 287*: 34 (1997), Muir *et al, Proc. Nat'l Acad. Sci. USA 95*:6705 (1998), and Severinov and Muir, *J. Biol. Chem. 273*:16205 (1998)).

Peptides and polypeptides of the present invention comprise at least six, preferably at least nine, and more preferably at least 15 contiguous amino acid residues of SEQ ID NO: 1. Within certain embodiments of the invention, the polypeptides comprise 20, 30, 40, 50, 100, or more contiguous residues of SEQ ID NO:1. Nucleic acid molecules encoding such peptides and polypeptides are useful as polymerase chain reaction primers and probes.

The present invention contemplates compositions comprising a peptide or polypeptide described herein. Such compositions can further comprise a carrier. The carrier can be a conventional organic or inorganic carrier. Examples of carriers include water, buffer solution, alcohol, propylene glycol, macrogol, sesame oil, corn oil, and the like.

### 6. Production of Zace1 Fusion Proteins and Conjugates

Fusion proteins of Zace1 can be used to express *Zace1* in a recombinant host, and to isolate the produced Zace1. As described below, particular Zace1 fusion proteins also have uses in diagnosis and therapy.

One type of fusion protein comprises a peptide that guides a Zace1 polypeptide from a recombinant host cell. To direct a Zace1 polypeptide into the secretory pathway of a eukaryotic host cell, a secretory signal sequence (also known as a signal peptide, a leader sequence, prepro sequence or pre sequence) is provided in the *Zace1* expression vector. While the secretory signal sequence may be derived from Zace1, a suitable signal sequence may also be derived from another secreted protein or synthesized *de novo.* The secretory signal sequence is operably linked to a *Zace1*-encoding sequence such that the two sequences are joined in the correct reading frame and positioned to direct the newly synthesized polypeptide into the secretory pathway of the host cell. Secretory signal sequences are commonly positioned 5' to the nucleotide sequence encoding the polypeptide of interest, although certain secretory signal sequences may be positioned elsewhere in the nucleotide sequence of interest (see, *e.g.*, Welch *et al*., U.S. Patent No. 5,037,743; Holland *et al.,* U.S. Patent No. 5,143,830).

Although the secretory signal sequence of Zace1 or another protein produced by mammalian cells (e.g., tissue-type plasminogen activator signal sequence, as described, for example, in U.S. Patent No. 5,641,655) is useful for expression of Zace1 in recombinant mammalian hosts, a yeast signal sequence is preferred for expression in yeast cells. Examples of suitable yeast signal sequences are those derived from yeast mating phermone α-factor (encoded by the *MFα1* gene), invertase (encoded by the *SUC2* gene), or acid phosphatase (encoded by the *PHO5* gene). See, for example, Romanos *et al.*, "Expression of Cloned Genes in Yeast," in *DNA Cloning 2*: *A Practical Approach,* 2^{nd} Edition, Glover and Hames (eds.), pages 123-167 (Oxford University Press 1995).

In bacterial cells, it is often desirable to express a heterologous protein as a fusion protein to decrease toxicity, increase stability, and to enhance recovery of the expressed protein. For example, *Zace1* can be expressed as a fusion protein comprising a glutathione S-transferase polypeptide. Glutathione S-transferease fusion proteins are typically soluble, and easily purifiable from *E. coli* lysates on immobilized glutathione columns. In similar approaches, a Zace1 fusion protein comprising a maltose binding protein polypeptide can be isolated with an amylose resin column, while a fusion protein comprising the C-terminal end of a truncated Protein A gene can be purified using IgG-Sepharose. Established techniques for expressing a heterologous polypeptide as a fusion protein in a bacterial cell are described, for example, by Williams *et al*., "Expression of Foreign Proteins in *E. coli* Using Plasmid Vectors and Purification of Specific Polyclonal Antibodies," in *DNA Cloning 2*: *A Practical Approach,* 2^{nd} Edition, Glover and Hames (Eds.), pages 15-58 (Oxford University Press 1995). In addition, commercially available expression systems are available. For example, the PINPOINT Xa protein purification system (Promega Corporation; Madison, WI) provides a method for isolating a fusion protein comprising a polypeptide that becomes biotinylated during expression with a resin that comprises avidin.

Peptide tags that are useful for isolating heterologous polypeptides expressed by either prokaryotic or eukaryotic cells include polyHistidine tags (which have an affinity for nickel-chelating resin), *c-myc* tags, calmodulin binding protein (isolated with calmodulin affinity chromatography), substance P, the RYIRS tag (which binds with anti-RYIRS antibodies), the Glu-Glu tag, and the FLAG tag (which binds with anti-FLAG antibodies). See, for example, Luo *et al., Arch. Biochem. Biophys. 329*:215 (1996), Morganti *el al.. Biotechnol. Appl. Biochem. 23*:67 (1996), and Zheng *et al., Gene 186*:55 (1997). Nucleic acid molecules encoding such peptide tags are available, for example, from Sigma-Aldrich Corporation (St. Louis, MO).

Another form of fusion protein comprises a Zace1 polypeptide and an immunoglobulin heavy chain constant region, typically an F_{c} fragment, which contains two or three constant region domains and a hinge region but lacks the variable region. As an illustration, Chang *et al.,* U.S. Patent No. 5,723,125, describe a fusion protein comprising a human interferon and a human immunoglobulin Fc fragment. The C-terminal of the interferon is linked to the N-terminal of the Fc fragment by a peptide linker moiety. An example of a peptide linker is a peptide comprising primarily a T cell inert sequence, which is immunologically inert. An exemplary peptide linker has the amino acid sequence: GGSGG SGGGG SGGGG S (SEQ ID NO:3). In this fusion protein, a preferred Fc moiety is a human γ4 chain, which is stable in solution and has little or no complement activating activity. Accordingly, the present invention contemplates a Zace1 fusion protein that comprises a Zace1 moiety and a human Fc fragment, wherein the C-terminus of the Zace1 moiety is attached to the N-terminus of the Fc fragment via a peptide linker, such as a peptide consisting of the amino acid sequence of SEQ ID NO:3. The Zace1 moiety can be a Zace1 molecule or a fragment thereof. For example, a fusion protein can comprise a fragment of Zace1 that contains the catalytic domain (*e.g.*, a soluble Zace1 fragment) and an Fc fragment (*e.g.*, a human Fc fragment).

In another variation, a Zace1 fusion protein comprises an IgG sequence, a Zace1 moiety covalently joined to the aminoterminal end of the IgG sequence, and a signal peptide that is covalently joined to the aminoterminal of the Zace1 moiety, wherein the IgG sequence consists of the following elements in the following order: a hinge region, a CH₂ domain, and a CH₃ domain. Accordingly, the IgG sequence lacks a CH₁ domain. The Zace1 moiety displays a Zace1 activity, as described herein, such as the ability to react with a substrate. This general approach to producing fusion proteins that comprise both antibody and nonantibody portions has been described by LaRochelle *et al.*, EP 742830 (WO 95/21258).

Fusion proteins comprising a Zace1 moiety and an Fc moiety can be used, for example, as an *in vitro* assay tool. For example, the presence of an Zace1 substrate in a biological sample can be detected using a Zace1-immunoglobulin fusion protein, in which the Zace1 moiety is used to bind the substrate, and a macromolecule, such as Protein A or anti-Fc antibody, is used to bind the fusion protein to a solid support. Such systems can also be used to identify Zace 1 substrates and inhibitors.

Other examples of antibody fusion proteins include polypeptides that comprise an antigen-binding domain and a Zace1 fragment that contains a Zace1 catalytic domain. Such molecules can be used to target particular tissues for the benefit of Zace1 enzymatic activity.

The present invention further provides a variety of other polypeptide fusions. For example, a Zace 1 polypeptide (corresponding to the C domain of somatic ACE) can be prepared as a fusion to an N domain of somatic ACE. The native Zace 1 signal sequence may also be recombinantly exchanged with the signal sequence of somatic ACE or tACE. Likewise, the transmembrane domain of Zace1 can be recombinantly exchanged with that of somatic ACE or tACE. The catalytic domain of Zace1 can also be recombinantly exchanged for the corresponding region of somatic ACE, tACE, thermolysin or another zinc metalloprotease. Accordingly, part or all of a domain(s) conferring a biological function can be swapped between Zace1 of the present invention with the functionally equivalent domain(s) from another family member, such as tACE or somatic ACE. For example, the region from His³⁹⁸ to Ser⁴³⁰ of Zace1 can be recombinantly exchanged for the corresponding region of somatic ACE, tACE, thermolysin, or other zinc metalloprotease.

Polypeptide fusions can be expressed in recombinant host cells to produce a variety of Zace1 fusion analogs. A Zace1 polypeptide can be fused to two or more moieties or domains, such as an affinity tag for purification and a targeting domain. Polypeptide fusions can also comprise one or more cleavage sites, particularly between domains. See, for example, *Tuan et al., Connective Tissue Research 34*:1 (1996).

Fusion proteins can be prepared by methods known to those skilled in the art by preparing each component of the fusion protein and chemically conjugating them. Alternatively, a polynucleotide encoding both components of the fusion protein in the proper reading frame can be generated using known techniques and expressed by the methods described herein. For example, part or all of a domain(s) conferring a biological function can be swapped between Zace1 of the present invention with the functionally equivalent domain(s) from another family member, such as tACE or somatic ACE. Such domains include, but are not limited to, the secretory signal sequence, conserved motifs (such as the HEXXH and EX(I/V)X(D/S) domains), and the transmembrane or intracellular domain. Such fusion proteins would be expected to have a biological functional profile that is the same or similar to polypeptides of the present invention or other known zinc metalloprotease family proteins, depending on the fusion constructed. General methods for enzymatic and chemical cleavage of fusion proteins are described, for example, by Ausubel (1995) at pages 16-19 to 16-25.

The present invention also contemplates chemically modified Zace1 compositions, in which a Zace1 polypeptide is linked with a polymer. Examples of suitable Zace1 polypeptides include soluble polypeptides that lack a functional transmembrane domain. Typically, the polymer is water soluble so that the Zace1 conjugate does not precipitate in an aqueous environment, such as a physiological environment. An example of a suitable polymer is one that has been modified to have a single reactive group, such as an active ester for acylation, or an aldehyde for alkylation, In this way, the degree of polymerization can be controlled. An example of a reactive aldehyde is polyethylene glycol propionaldehyde, or mono-(C1-C10) alkoxy, or aryloxy derivatives thereof (see, for example, Harris, *et al.*, U.S. Patent No. 5,252,714). The polymer may be branched or unbranched. Moreover, a mixture of polymers can be used to produce Zace1 conjugates.

Zace1 conjugates used for therapy should preferably comprise pharmaceutically acceptable water-soluble polymer moieties. Suitable water-soluble polymers include polyethylene glycol (PEG), monomethoxy-PEG, mono-(C1-C10)alkoxy-PEG, aryloxy-PEG, poly-(N-vinyl pyrrolidone)PEG, tresyl monomethoxy PEG, PEG propionaldehyde, bis-succinimidyl carbonate PEG, propylene glycol homopolymers, a polypropylene oxide/ethylene oxide co-polymer, polyoxyethylated polyols (*e.g*., glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. A Zace1 conjugate can also comprise a mixture of such water-soluble polymers.

One example of a Zace1 conjugate comprises a Zace1 moiety and a polyalkyl oxide moiety attached to the *N*-terminus of the Zace1 moiety. PEG is one suitable polyalkyl oxide. As an illustration, Zace1 can be modified with PEG, a process known as "PEGylation." PEGylation of Zace1 can be carried out by any of the PEGylation reactions known in the art (see, for example, EP 0 154 316, Delgado *et al., Critical Reviews in Therapeutic Drug Carrier Systems 9*:249 (1992), Duncan and Spreafico, *Clin. Pharmacokinet. 27*:290 (1994), and Francis *et al., Int J Hematol 68*:1 (1998)). For example, PEGylation can be performed by an acylation reaction or by an alkylation reaction with a reactive polyethylene glycol molecule. In an alternative approach, Zace1 conjugates are formed by condensing activated PEG, in which a terminal hydroxy or amino group of PEG has been replaced by an activated linker (see, for example, Karasiewicz *et al.,* U.S. Patent No. 5,382,657).

PEGylation by acylation typically requires reacting an active ester derivative of PEG with a Zace1 polypeptide. An example of an activated PEG ester is PEG esterified to N-hydroxysuccinimide. As used herein, the term "acylation" includes the following types of linkages between Zace1 and a water soluble polymer: amide, carbamate, urethane, and the like. Methods for preparing PEGylated Zace1 by acylation will typically comprise the steps of (a) reacting a Zace1 polypeptide with PEG (such as a reactive ester of an aldehyde derivative of PEG) under conditions whereby one or more PEG groups attach to Zace1, and (b) obtaining the reaction product(s). Generally, the optimal reaction conditions for acylation reactions will be determined based upon known parameters and desired results. For example, the larger the ratio of PEG:Zace1, the greater the percentage of polyPEGylated Zace product.

The product of PEGylation by acylation is typically a polyPEGylated Zace1 product, wherein the lysine ε-amino groups are PEGylated via an acyl linking group. An example of a connecting linkage is an amide. Typically, the resulting Zace1 will be at least 95% mono-, di-, or tri-pegylated, although some species with higher degrees of PEGylation may be formed depending upon the reaction conditions. PEGylated species can be separated from unconjugated Zace1 polypeptides using standard purification methods, such as dialysis, ultrafiltration, ion exchange chromatography, affinity chromatography, and the like.

PEGylation by alkylation generally involves reacting a terminal aldehyde derivative of PEG with Zace1 in the presence of a reducing agent. PEG groups are preferably attached to the polypeptide via a -CH₂-NH group.

Derivatization via reductive alkylation to produce a monoPEGylated product takes advantage of the differential reactivity of different types of primary amino groups available for derivatization. Typically, the reaction is performed at a pH that allows one to take advantage of the pKa differences between the ε-amino groups of the lysine residues and the α-amino group of the *N*-terminal residue of the protein. By such selective derivatization, attachment of a water-soluble polymer that contains a reactive group such as an aldehyde, to a protein is controlled. The conjugation with the polymer occurs predominantly at the N-terminus of the protein without significant modification of other reactive groups such as the lysine side chain amino groups. The present invention provides a substantially homogenous preparation of Zace1 monopolymer conjugates.

Reductive alkylation to produce a substantially homogenous population of monopolymer Zace1 conjugate molecule can comprise the steps of: (a) reacting a Zace1 polypeptide with a reactive PEG under reductive alkylation conditions at a pH suitable to permit selective modification of the α-amino group at the amino terminus of the Zace1, and (b) obtaining the reaction product(s). The reducing agent used for reductive alkylation should be stable in aqueous solution and preferably be able to reduce only the Schiff base formed in the initial process of reductive alkylation. Preferred reducing agents include sodium borohydride, sodium cyanoborohydride, dimethylamine borane, trimethylamine borane, and pyridine borane.

For a substantially homogenous population of monopolymer Zace1 conjugates, the reductive alkylation reaction conditions are those which permit the selective attachment of the water soluble polymer moiety to the *N*-terminus of Zace1. Such reaction conditions generally provide for pKa differences between the lysine amino groups and the α-amino group at the *N*-terminus. The pH also affects the ratio of polymer to protein to be used. In general, if the pH is lower, a larger excess of polymer to protein will be desired because the less reactive the *N*-terminal α-group, the more polymer is needed to achieve optimal conditions. If the pH is higher, the polymer:Zace1 need not be as large because more reactive groups are available. Typically, the pH will fall within the range of about 3 to about 9, or about 3 to about 6.

Another factor to consider is the molecular weight of the water-soluble polymer. Generally, the higher the molecular weight of the polymer, the fewer number of polymer molecules which may be attached to the protein. For PEGylation reactions, the typical molecular weight is about 2 kDa to about 100 kDa, about 5 kDa to about 50 kDa, or about 12 kDa to about 25 kDa. The molar ratio of water-soluble polymer to Zace1 will generally be in the range of 1:1 to 100:1. Typically, the molar ratio of water-soluble polymer to Zace1 will be 1:1 to 20:1 for polyPEGylation, and 1:1 to 5:1 for monoPEGylation.

General methods for producing conjugates comprising a polypeptide and water-soluble polymer moieties are known in the art. See, for example, Karasiewicz et al., U.S. Patent No. 5,382,657, Greenwald *et al.,* U.S. Patent No. 5,738, 846, Nieforth *et al., Clin. Pharmacol. Ther. 59*:636 (1996), Monkarsh *et al., Anal. Biochem. 247*:434 (1997)).

### 7. Isolation of Zace1 Polypeptides

The polypeptides of the present invention can be purified to at least about 80% purity, to at least about 90% purity, to at least about 95% purity, or greater than 95% purity with respect to contaminating macromolecules, particularly other proteins and nucleic acids, and free of infectious and pyrogenic agents. The polypeptides of the present invention may also be purified to a pharmaceutically pure state, which is greater than 99.9% pure. Particular purified polypeptides are substantially free of other polypeptides, particularly other polypeptides of animal origin.

Fractionation and/or conventional purification methods can be used to obtain preparations of Zace1 purified from natural sources, synthetic Zace1 polypeptides, and recombinant Zace1 polypeptides and fusion Zace1 polypeptides purified from recombinant host cells. In general, ammonium sulfate precipitation and acid or chaotrope extraction may be used for fractionation of samples. Exemplary purification steps may include hydroxyapatite, size exclusion, FPLC and reverse-phase high performance liquid chromatography. Suitable chromatographic media include derivatized dextrans, agarose, cellulose, polyacrylamide, specialty silicas, and the like. PEI, DEAE, QAE and Q derivatives are preferred. Exemplary chromatographic media include those media derivatized with phenyl, butyl, or octyl groups, such as Phenyl-Sepharose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties.

Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Selection of a particular method for polypeptide isolation and purification is a matter of routine design and is determined in part by the properties of the chosen support. See, for example, *Affinity Chromatography: Principles & Methods* (Pharmacia LKB Biotechnology 1988), and Doonan, *Protein Purification Protocols* (The Humana Press 1996).

Additional variations in Zace1 isolation and purification can be devised by those of skill in the art. For example, anti-Zace1 antibodies, obtained as described below, can be used to isolate large quantities of protein by immunoaffinity purification.

Moreover, methods for binding enzymes, such as Zace1, to substrates bound to support media are well known in the art. For example, the polypeptides of the present invention can be isolated by exploitation of their homology to somatic ACE and tACE. These enzymes can be purified by affinity chromatography using the ACE inhibitor lisinopril [*N*-[(*S*)-1-carboxy-3-phenylpropyl]-Lys-Pro] as the ligand affixed to a solid support. Improved purification yields can be obtained using a 28 A, rather than a 14 Å, spacer between ligand and solid support.

The polypeptides of the present invention can also be isolated by exploitation of particular properties. For example, immobilized metal ion adsorption (IMAC) chromatography can be used to purify histidine-rich proteins, including those comprising polyhistidine tags. Briefly, a gel is first charged with divalent metal ions to form a chelate (Sulkowski, *Trends in Biochem. 3*:1 (1985)). Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography (M. Deutscher, (ed.), *Meth. Enzymol. 182*:529 (1990)). Within additional embodiments of the invention, a fusion of the polypeptide of interest and an affinity tag (*e.g.*, maltose-binding protein, an immunoglobulin domain) may be constructed to facilitate purification.

Zace1 polypeptides or fragments thereof may also be prepared through chemical synthesis, as described above. Zace1 polypeptides may be monomers or multimers; glycosylated or non-glycosylated; PEGylated or non-PEGylated; and may or may not include an initial methionine amino acid residue.

### 8. Zace1 Analogs and Zace1 Inhibitors

One general class of Zace1 analogs are variants having an amino acid sequence that is a mutation of the amino acid sequence disclosed herein. Another general class of Zace1 analogs is provided by anti-idiotype antibodies, and fragments thereof, as described below. Moreover, recombinant antibodies comprising anti-idiotype variable domains can be used as analogs (see, for example, Monfardini *et al., Proc. Assoc. Am. Physicians 108*:420 (1996)). Since the variable domains of anti-idiotype Zace1 antibodies mimic Zace1, these domains can provide Zace1 enzymatic activity. Methods of producing anti-idiotypic catalytic antibodies are known to those of skill in the art (see, for example, Joron *et al., Ann. N Y Acad. Sci. 672*:216 (1992), Friboulet *et al., Appl. Biochem. Biotechnol. 47*:229 (1994), and Avalle *et al., Ann. N Y Acad .Sci. 864*:118 (1998)).

Another approach to identifying Zace1 analogs is provided by the use of combinatorial libraries. Methods for constructing and screening phage display and other combinatorial libraries are provided, for example, by Kay *et al., Phage Display of Peptides and Proteins* (Academic Press 1996), Verdine, U.S. Patent No. 5,783,384, Kay, *et. al.,* U.S. Patent No. 5,747,334, and Kauffman *et al.,* U.S. Patent No. 5,723,323.

One illustrative *in vitro* use of Zace1 and its analogs is the production of labeled angiotensin II. For example, angiotensin I, radiolabeled at its N-terminus, can be incubated in the presence of Zace1 or an active variant Zace1. The product of the reaction will be radiolabeled angiotensin II. This radiolabeled molecule can be used to study the metabolism of angiotensin II *in vitro* or to observe the tissue distribution of administered angiotensin II *in vivo.*

The activity of Zace1 molecules of the present invention can be measured using a variety of assays that measure catalytic activity of the enzyme in the presence or absence of zinc, or that measure the effects of chloride or other monoanions on the catalytic activity of Zace1. In addition, the Zace1 polypeptides can be characterized by measuring the zinc content of these polypeptides. Radiolabeled ACE inhibitors are useful for detecting high-affinity binding sites in zinc metalloprotease family members. One or more mutations of putative critical or important residues, in conjunction with known assays of ACE activity, permit analysis of mutational effects on Zace1 structure, enzyme activity, and immunological activity. In addition, both synthetic and natural ACE substrates can be useful in characterizing variant or mutated Zace1 polypeptides. Studies that examine the interaction of Zace1 and competitive ACE inhibitors also can be employed to assay and characterize Zace1 polypeptides. Such assays are well known in the art. For a general reference, see Corvol *et al., Meth. Enzymol. 246*:283 (1995). See also Williams *et al., J. Biol. Chem. 269*:29430 (1994), Sturrock *et al., Biochem. 35*:9560 (1996), and Michaud *et al., Molec. Pharmacol. 51*:1070 (1997).

As an illustration, a Zace1 variant can be tested for ACE activity using hippuryl-L-histidyl-L-leucine (Hip-His-Leu) as a substrate (see, for example, Sen *et al., J. Biol. Chem. 268*:25748 (1993)). In one version of this assay, a solubilized test polypeptide is incubated in 0.4 M sodium borate buffer (pH 8.3) containing 300 mM sodium chloride for about 15 to 30 minutes at 37°C in the presence of varying concentrations of Hip-His-Leu (*e.g.*, 0.4 to 5 mM). The amount of His-Leu liberated by the test polypeptide is measured fluorometrically. Hip-His-Leu can also be used to identify Zace1 inhibitors by measuring the suppression of the cleavage of the substrate.

Other ACE substrates are known to those of skill in the art. For example, Isaac *et al., Biochem. J. 328*:587 (1997), have shown that polypeptides having Lys/Arg-Arg at the C-terminus are high-affinity substrates for human tACE. Another useful substrate to measure ACE activity is [³H]benzol-Phe-Ala-Pro (Howell *et al., Am. J. Physiol. 258*:L188 (1990)).

Solid phase systems can also be used to identify a substrate or inhibitor of a Zace1 polypeptide. For example, a Zace1 polypeptide, which may or may not be catalytically active, or Zace1 fusion protein can be immobilized onto the surface of a receptor chip of a commercially available biosensor instrument (BIACORE, Biacore AB; Uppsala, Sweden). The use of this instrument is disclosed, for example, by Karlsson, *Immunol. Methods 145*:229 (1991), and Cunningham and Wells, *J. Mol. Biol. 234*:554 (1993).

In brief, a Zace1 polypeptide or fusion protein is covalently attached, using amine or sulfhydryl chemistry, to dextran fibers that are attached to gold film within a flow cell. A test sample is then passed through the cell. If a Zace1 substrate or inhibitor is present in the sample, it will bind to the immobilized polypeptide or fusion protein, causing a change in the refractive index of the medium, which is detected as a change in surface plasmon resonance of the gold film. This system allows the determination on- and off-rates, from which binding affinity can be calculated, and assessment of the stoichiometry of binding, as well as the kinetic effects of Zace1 mutation. This system can also be used to examine antibody-antigen interactions, and the interactions of other complement/anti-complement pairs.

Zace1 polypeptides can also be immobilized on a solid support, such as beads of agarose, cross-linked agarose, glass, cellulosic resins, silica-based resins, polystyrene, cross-linked polyacrylamide, or like materials that are stable under the conditions of use. Methods for linking polypeptides to solid supports are known in the art, and include amine chemistry, cyanogen bromide activation, N-hydroxysuccinimide activation, epoxide activation, sulfhydryl activation, and hydrazide activation. The resulting medium will generally be configured in the form of a column, and fluids containing substrate or putative substrate are passed through the column one or more times to allow substrate to bind to the Zace1 polypeptide. The substrate is then eluted using changes in salt concentration, chaotropic agents (guanidine HCl), or pH to disrupt substrate-Zace1 binding.

Accordingly, polypeptides of the present invention are useful as targets for identifying modulators of zinc protease activity. More particularly, Zace1 polypeptides are useful for screening or identifying new ACE inhibitors. The Zace1 polypeptides can also be used as a basis for rational drug design of inhibitory molecules. These newly identified inhibitory molecules may be more specific or more potent than known ACE inhibitors. Moreover, Zace1 inhibitors may exhibit a more favorable side effect profile than known ACE inhibitors. For example, Zace1 may contribute to certain unwanted side effects of ACE inhibitors, and as such, Zace1 would be useful to identify more specific ACE inhibitors.

In addition, inhibitory molecules identified using Zace1 polypeptides as a target may modulate different biological or physiological activities than known ACE inhibitors (e.g., the inhibitors may be effective for disorders other than those related to blood pressure and water and salt homeostasis). Zace1 inhibitors may provide broader inhibition than just ACE inhibition (for instance, these inhibitors may modulate many metalloprotease family members). Because Zace1 is more closely homologous to tACE than somatic ACE, Zace1 may permit selection of domain-specific inhibitors (those that inhibit the active site corresponding to the C domain of somatic ACE). Thus, a Zace1 inhibitor may specifically target angiotensin I and bradykinin-mediated effects, but have minimal or no effect on regulating hematopoiesis. Zace1 inhibitors may beneficially improve the status of patients with cardiovascular disease, and atherosclerotic vascular disease in particular, or renal disease, and diabetic nephropathy in particular. The effects of Zace1 inhibitors can be measured *in vitro* using cultured cells or *in vivo* by administering molecules of the claimed invention to the appropriate animal model.

The measurement of Zace1 enzyme activity can also be used for diagnosis. For example, the measurement of serum ACE activity levels provides useful information for the diagnosis of sarcoidosis and response to treatment (Studdy, *Lancet 2(8104-5)*:1331 (1978)).

### 9. Production of Antibodies to Zace1 Proteins

Antibodies to Zace1 can be obtained, for example, using the product of a Zace1 expression vector or Zace1 isolated from a natural source as an antigen. Particularly useful anti-Zace1 antibodies "bind specifically" with Zace1. Antibodies are considered to be specifically binding if the antibodies exhibit at least one of the following two properties: (1) antibodies bind to Zace1 with a threshold level of binding activity, and (2) antibodies do not significantly cross-react with polypeptides related to Zace1.

With regard to the first characteristic, antibodies specifically bind if they bind to a Zace1 polypeptide, peptide or epitope with a binding affinity (Kₐ) of 10⁶ M⁻¹ or greater, preferably 10⁷ M⁻¹ or greater, more preferably 10⁸ M⁻¹ or greater, and most preferably 10⁹ M⁻¹ or greater. The binding affinity of an antibody can be readily determined by one of ordinary skill in the art, for example, by Scatchard analysis (Scatchard, *Ann. NY Acad. Sci. 51*:660 (1949)).

With regard to the second characteristic, antibodies do not significantly cross-react with related polypeptide molecules, for example, if they detect Zace1, but not presently known polypeptides using a standard Western blot analysis. Examples of known related polypeptides are known angiotensin converting enzymes, such as human somatic ACE and tACE. A variety of assays known to those skilled in the art can be utilized to detect antibodies which specifically bind to Zace1 proteins or peptides. Exemplary assays are described, for example, by Harlow and Lane (Eds.), *Antibodies: A Laboratory Manual* (Cold Spring Harbor Laboratory Press 1988). Representative examples of such assays include: concurrent immunoelectrophoresis, radioimmunoassay, radioimmuno-precipitation, enzyme-linked immunosorbent assay (ELISA), dot blot or Western blot assay, inhibition or competition assay, and sandwich assay. In addition, antibodies can be screened for binding to wild-type versus mutant Zace1 protein or polypeptide.

Anti-Zace1 antibodies can be produced using antigenic Zace1 epitope-bearing peptides and polypeptides. Antigenic epitope-bearing peptides and polypeptides of the present invention contain a sequence of at least nine, preferably between 15 to about 30 amino acids contained within SEQ ID NO:1. However, peptides or polypeptides comprising a larger portion of an amino acid sequence of the invention, containing from 30 to 50 amino acids, or any length up to and including the entire amino acid sequence of a polypeptide of the invention, also are useful for inducing antibodies that bind with Zace1. It is desirable that the amino acid sequence of the epitope-bearing peptide is selected to provide substantial solubility in aqueous solvents (*i.e.*, the sequence includes relatively hydrophilic residues, while hydrophobic residues are preferably avoided). Moreover, amino acid sequences containing proline residues may be also be desirable for antibody production.

As an illustration, potential antigenic sites in Zace1 were identified using the Jameson-Wolf method, Jameson and Wolf, *CABIOS 4*:181, (1988), as implemented by the PROTEAN program (version 3.14) of LASERGENE (DNASTAR; Madison, WI). Default parameters were used in this analysis.

The Jameson-Wolf method predicts potential antigenic determinants by combining six major subroutines for protein structural prediction. Briefly, the Hopp-Woods method, Hopp *et al., Proc. Nat'l Acad. Sci. USA 78*:3824 (1981), was first used to identify amino acid sequences representing areas of greatest local hydrophilicity (parameter: seven residues averaged). In the second step, Emini's method, Emini *et al., J. Virology 55*:836 (1985), was used to calculate surface probabilities (parameter: surface decision threshold (0.6) = 1). Third, the Karplus-Schultz method, Karplus and Schultz, *Naturwissenschaften 72*:212 (1985), was used to predict backbone chain flexibility (parameter: flexibility threshold (0.2) = 1). In the fourth and fifth steps of the analysis, secondary structure predictions were applied to the data using the methods of Chou-Fasman, Chou, "Prediction of Protein Structural Classes from Amino Acid Composition," in *Prediction of Protein Structure and the Principles of Protein Conformation,* Fasman (ed.), pages 549-586 (Plenum Press 1990), and Garnier-Robson, Garnier *et al., J. Mol. Biol. 120*:97 (1978) (Chou-Fasman parameters: conformation table = 64 proteins; α region threshold = 103; β region threshold = 105; Garnier-Robson parameters: α and β decision constants = 0). In the sixth subroutine, flexibility parameters and hydropathy/solvent accessibility factors were combined to determine a surface contour value, designated as the "antigenic index." Finally, a peak broadening function was applied to the antigenic index, which broadens major surface peaks by adding 20, 40, 60, or 80% of the respective peak value to account for additional free energy derived from the mobility of surface regions relative to interior regions. This calculation was not applied, however, to any major peak that resides in a helical region, since helical regions tend to be less flexible.

The results of this analysis indicated that the following amino acid sequences of SEQ ID NO: 1 would provide suitable antigenic peptides: amino acids 28 to 34 ("antigenic peptide 1"), amino acids 39 to 56 ("antigenic peptide 2"), amino acids 85 to 92 ("antigenic peptide 3"), amino acids 117 to 125 ("antigenic peptide 4"), amino acids 132 to 147 ("antigenic peptide 5"), amino acids 233 to 245 ("antigenic peptide 6"), amino acids 376 to 394 ("antigenic peptide 7"), amino acids 512 to 523 ("antigenic peptide 8"), amino acids 580 to 586 ("antigenic peptide 9"), amino acids 635 to 649 ("antigenic peptide 10"), and amino acids 655 to 662 ("antigenic peptide 11"). The present invention contemplates the use of any one of antigenic peptides 1 to 11 to generate antibodies to Zace1. The present invention also contemplates polypeptides comprising at least one of antigenic peptides 1 to 11.

Polyclonal antibodies to recombinant Zace1 protein or to Zace1 isolated from natural sources can be prepared using methods well-known to those of skill in the art. See, for example, Green *et al.*, "Production of Polyclonal Antisera," in *Immunochemical Protocols* (Manson, ed.), pages 1-5 (Humana Press 1992), and Williams *et al*., "Expression of foreign proteins in *E. coli* using plasmid vectors and purification of specific polyclonal antibodies," in *DNA Cloning 2*: *Expression Systems, 2nd Edition,* Glover *et al.* (eds.), page 15 (Oxford University Press 1995). The immunogenicity of a Zace1 polypeptide can be increased through the use of an adjuvant, such as alum (aluminum hydroxide) or Freund's complete or incomplete adjuvant. Polypeptides useful for immunization also include fusion polypeptides, such as fusions of Zace1 or a portion thereof with an immunoglobulin polypeptide or with maltose binding protein. The polypeptide immunogen may be a full-length molecule or a portion thereof. If the polypeptide portion is "hapten-like," such portion may be advantageously joined or linked to a macromolecular carrier (such as keyhole limpet hemocyanin (KLH), bovine serum albumin (BSA) or tetanus toxoid) for immunization.

Although polyclonal antibodies are typically raised in animals such as horses, cows, dogs, chicken, rats, mice, rabbits, guinea pigs, goats, or sheep, an anti-Zace1 antibody of the present invention may also be derived from a subhuman primate antibody. General techniques for raising diagnostically and therapeutically useful antibodies in baboons may be found, for example, in Goldenberg *et al.,* international patent publication No. WO 91/11465, and in Losman *et al., Int. J. Cancer 46*:310 (1990).

Alternatively, monoclonal anti-Zace1 antibodies can be generated. Rodent monoclonal antibodies to specific antigens may be obtained by methods known to those skilled in the art (see, for example, Kohler *et al., Nature 256*:495 (1975), Coligan *et al.* (eds.), *Current Protocols in Immunology, Vol. 1,* pages 2.5.1-2.6.7 (John Wiley & Sons 1991) ["Coligan"], Picksley *et al.,* "Production of monoclonal antibodies against proteins expressed in *E. coli*," in *DNA Cloning 2*: *Expression Systems, 2nd Edition,* Glover *et al.* (eds.), page 93 (Oxford University Press 1995)).

Briefly, monoclonal antibodies can be obtained by injecting mice with a composition comprising a *Zace1* gene product, verifying the presence of antibody production by removing a serum sample, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

In addition, an anti-Zace1 antibody of the present invention may be derived from a human monoclonal antibody. Human monoclonal antibodies are obtained from transgenic mice that have been engineered to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described, for example, by Green *et al., Nature Genet. 7*:13 (1994), Lonberg *et al., Nature 368*:856 (1994), and Taylor *et al., Int. Immun. 6*:579 (1994).

Monoclonal antibodies can be isolated and purified from hybridoma cultures by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines *et al*., "Purification of Immunoglobulin G (IgG)," in *Methods in Molecular Biology, Vol. 10,* pages 79-104 (The Humana Press, Inc. 1992)).

For particular uses, it may be desirable to prepare fragments of anti-Zace1 antibodies. Such antibody fragments can be obtained, for example, by proteolytic hydrolysis of the antibody. Antibody fragments can be obtained by pepsin or papain digestion of whole antibodies by conventional methods. As an illustration, antibody fragments can be produced by enzymatic cleavage of antibodies with pepsin to provide a 5S fragment denoted F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, by Goldenberg, U.S. patent No. 4,331,647, Nisonoff *et al., Arch Biochem. Biophys. 89*:230 (1960), Porter, *Biochem. J.* *73*:119 (1959), Edelman *et al.,* in *Methods in Enzymology Vol. 1*, page 422 (Academic Press 1967), and by Coligan at pages 2.8.1-2.8.10 and 2.10.-2.10.4.

Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

For example, Fv fragments comprise an association of V_{H} and V_{L} chains. This association can be noncovalent, as described by Inbar *et al., Proc. Nat'l Acad. Sci. USA 69:*2659 (1972). Alternatively, the variable chains can be linked by an intermolecular disulfide bond or cross-linked by chemicals such as glutaraldehyde (see, for example, Sandhu, *Crit. Rev. Biotech. 12*:437 (1992)).

The Fv fragments may comprise V_{H} and V_{L} chains which are connected by a peptide linker. These single-chain antigen binding proteins (scFv) are prepared by constructing a structural gene comprising DNA sequences encoding the V_{H} and V_{L} domains which are connected by an oligonucleotide. The structural gene is inserted into an expression vector which is subsequently introduced into a host cell, such as *E. coli.* The recombinant host cells synthesize a single polypeptide chain with a linker peptide bridging the two V domains. Methods for producing scFvs are described, for example, by Whitlow *et al., Methods: A Companion to Methods in Enzymology 2*:97 (1991) (also see, Bird *et al., Science 242*:423 (1988), Ladner *et al.,* U.S. Patent No. 4,946,778, Pack *et al., Bio*/*Technology 11*:1271 (1993), and Sandhu, *supra*).

As an illustration, a scFV can be obtained by exposing lymphocytes to Zace1 polypeptide *in vitro*, and selecting antibody display libraries in phage or similar vectors (for instance, through use of immobilized or labeled Zace1 protein or peptide). Genes encoding polypeptides having potential Zace1 polypeptide binding domains can be obtained by screening random peptide libraries displayed on phage (phage display) or on bacteria, such as *E. coli.* Nucleotide sequences encoding the polypeptides can be obtained in a number of ways, such as through random mutagenesis and random polynucleotide synthesis. These random peptide display libraries can be used to screen for peptides which interact with a known target which can be a protein or polypeptide, such as a ligand or receptor, a biological or synthetic macromolecule, or organic or inorganic substances. Techniques for creating and screening such random peptide display libraries are known in the art (Ladner *et al*., U.S. Patent No. 5,223,409, Ladner *et al.,* U.S. Patent No. 4,946,778, Ladner *et al.*, U.S. Patent No. 5,403,484, Ladner *et al.,* U.S. Patent No. 5,571,698, and Kay *et al., Phage Display of Peptides and Proteins* (Academic Press, Inc. 1996)) and random peptide display libraries and kits for screening such libraries are available commercially, for instance from CLONTECH Laboratories, Inc. (Palo Alto, CA), Invitrogen Inc. (San Diego, CA), New England Biolabs, Inc. (Beverly, MA), and Pharmacia LKB Biotechnology Inc. (Piscataway, NJ). Random peptide display libraries can be screened using the Zace1 sequences disclosed herein to identify proteins which bind to Zace1.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells (see, for example, Larrick *et al., Methods: A Companion to Methods in Enzymology 2*:106 (1991), Courtenay-Luck, "Genetic Manipulation of Monoclonal Antibodies," in *Monoclonal Antibodies: Production, Engineering and Clinical Application,* Ritter *et al.* (eds.), page 166 (Cambridge University Press 1995), and Ward *et al.*, "Genetic Manipulation and Expression of Antibodies," in *Monoclonal Antibodies: Principles and Applications,* Birch *et al.,* (eds.), page 137 (Wiley-Liss, Inc. 1995)).

Alternatively, an anti-Zace1 antibody may be derived from a "humanized" monoclonal antibody. Humanized monoclonal antibodies are produced by transferring mouse complementary determining regions from heavy and light variable chains of the mouse immunoglobulin into a human variable domain. Typical residues of human antibodies are then substituted in the framework regions of the murine counterparts. The use of antibody components derived from humanized monoclonal antibodies obviates potential problems associated with the immunogenicity of murine constant regions. General techniques for cloning murine immunoglobulin variable domains are described, for example, by Orlandi *et al., Proc. Nat'l Acad. Sci. USA 86*:3833 (1989). Techniques for producing humanized monoclonal antibodies are described, for example, by *Jones et al., Nature 321*:522 (1986), Carter *et al., Proc. Nat'l Acad. Sci. USA 89*:4285 (1992), Sandhu, *Crit. Rev. Biotech. 12*:437 (1992), Singer *et al., J. Immun. 150*:2844 (1993), Sudhir (ed.), *Antibody Engineering Protocols* (Humana Press, Inc. 1995), Kelley, "Engineering Therapeutic Antibodies," in *Protein Engineering: Principles and Practice,* Cleland *et al.* (eds.), pages 399-434 (John Wiley & Sons, Inc. 1996), and by Queen *et al.,* U.S. Patent No. 5,693,762 (1997).

Polyclonal anti-idiotype antibodies can be prepared by immunizing animals with anti-Zace1 antibodies or antibody fragments, using standard techniques. See, for example, Green *et al.,* "Production of Polyclonal Antisera," in *Methods In Molecular Biology: Immunochemical Protocols,* Manson (ed.), pages 1-12 (Humana Press 1992). Also, see Coligan at pages 2.4.1-2.4.7. Alternatively, monoclonal anti-idiotype antibodies can be prepared using anti-Zace1 antibodies or antibody fragments as immunogens with the techniques, described above. As another alternative, humanized anti-idiotype antibodies or subhuman primate anti-idiotype antibodies can be prepared using the above-described techniques. Methods for producing anti-idiotype antibodies are described, for example, by Irie, U.S. Patent No. 5,208,146, Greene, *et. al.,* U.S. Patent No. 5,637,677, and Varthakavi and Minocha, *J. Gen. Virol. 77*:1875 (1996).

### 10. Use of Zace1 Nucleotide Sequences to Detect Gene Expression and Gene Structure

Nucleic acid molecules can be used to detect the expression of a *Zace1* gene in a biological sample. Probe molecules may be DNA, RNA, oligonucleotides, and the like. As used herein, the term "portion" refers to at least eight nucleotides to at least 20 or more nucleotides. Preferred probes bind with regions of the *Zace1* gene that have a low sequence similarity to comparable regions in other proteins, such as other angiotensin converting enzymes.

In a basic assay, a single-stranded probe molecule is incubated with RNA, isolated from a biological sample, under conditions of temperature and ionic strength that promote base pairing between the probe and target *Zace1* RNA species. After separating unbound probe from hybridized molecules, the amount of hybrids is detected.

Well-established hybridization methods of RNA detection include northern analysis and dot/slot blot hybridization (see, for example, Ausubel (1995) at pages 4-1 to 4-27, and Wu *et al.* (eds.), "Analysis of Gene Expression at the RNA Level," in *Methods in Gene Biotechnology,* pages 225-239 (CRC Press, Inc. 1997)). Nucleic acid probes can be detectably labeled with radioisotopes such as ³²P or ³⁵S. Alternatively, Zace1 RNA can be detected with a nonradioactive hybridization method (see, for example, Isaac (ed.), *Protocols for Nucleic Acid Analysis by Nonradioactive Probes* (Humana Press, Inc. 1993)). Typically, nonradioactive detection is achieved by enzymatic conversion of chromogenic or chemiluminescent substrates. Illustrative nonradioactive moieties include biotin, fluorescein, and digoxigenin.

*Zace1* oligonucleotide probes are also useful for *in vivo* diagnosis. As an illustration, ¹⁸F-labeled oligonucleotides can be administered to a subject and visualized by positron emission tomography (Tavitian *et al., Nature Medicine 4*:467 (1998)).

Numerous diagnostic procedures take advantage of the polymerase chain reaction (PCR) to increase sensitivity of detection methods. Standard techniques for performing PCR are well-known (see, generally, Mathew (ed.), *Protocols in Human* *Molecular Genetics* (Humana Press, Inc. 1991), White (ed.), *PCR Protocols: Current Methods and Applications* (Humana Press, Inc. 1993), Cotter (ed.), *Molecular Diagnosis of Cancer* (Humana Press, Inc. 1996), Hanausek and Walaszek (eds.), *Tumor Marker Protocols* (Humana Press, Inc. 1998), Lo (ed.), *Clinical Applications of PCR* (Humana Press, Inc. 1998), and Meltzer (ed.), *PCR in Bioanalysis* (Humana Press, Inc. 1998)).

Preferably, PCR primers are designed to amplify a portion of the *Zace1* gene that has a low sequence similarity to a comparable region in other proteins, such as other angiotensin converting enzymes.

One variation of PCR for diagnostic assays is reverse transcriptase-PCR (RT-PCR). In the RT-PCR technique, RNA is isolated from a biological sample, reverse transcribed to cDNA, and the cDNA is incubated with *Zace1* primers (see, for example, Wu *et al.* (eds.), "Rapid Isolation of Specific cDNAs or Genes by PCR," in *Methods in Gene Biotechnology,* pages 15-28 (CRC Press, Inc. 1997)). PCR is then performed and the products are analyzed using standard techniques.

PCR amplification products can be detected using a variety of approaches. For example, PCR products can be fractionated by gel electrophoresis, and visualized by ethidium bromide staining. Alternatively, fractionated PCR products can be transferred to a membrane, hybridized with a detectably-labeled Zace1 probe, and examined by autoradiography. Additional alternative approaches include the use of digoxigenin-labeled deoxyribonucleic acid triphosphates to provide chemiluminescence detection, and the C-TRAK colorimetric assay.

Another approach for detection of *Zace1* expression is cycling probe technology (CPT), in which a single-stranded DNA target binds with an excess of DNA-RNA-DNA chimeric probe to form a complex, the RNA portion is cleaved with RNAase H, and the presence of cleaved chimeric probe is detected (see, for example, Beggs *et al., J. Clin. Microbiol. 34*:2985 (1996), Bekkaoui *et al., Biotechniques 20*:240 (1996)). Alternative methods for detection of *Zace1* sequences can utilize approaches such as nucleic acid sequence-based amplification (NASBA), cooperative amplification of templates by cross-hybridization (CATCH), and the ligase chain reaction (LCR) (see, for example, Marshall *et al.,* U.S. Patent No. 5,686,272 (1997), Dyer *et al., J. Virol. Methods 60*:161 (1996), Ehricht *et al., Eur. J. Biochem. 243*:358 (1997), and Chadwick *et al., J. Virol. Methods 70*:59 (1998)). Other standard methods are known to those of skill in the art.

*Zace1* probes and primers can also be used to detect and to localize *Zace1* gene expression in tissue samples. Methods for such *in situ* hybridization are well-known to those of skill in the art (see, for example, Choo (ed.), *In Situ* *Hybridization Protocols* (Humana Press, Inc. 1994), Wu *et al.* (eds.), "Analysis of Cellular DNA or Abundance of mRNA by Radioactive *In Situ* Hybridization (RISH)," in *Methods in Gene Biotechnology,* pages 259-278 (CRC Press, Inc. 1997), and Wu *et al.* (eds.), "Localization of DNA or Abundance of mRNA by Fluorescence *In Situ* Hybridization (RISH)," in *Methods in Gene Biotechnology,* pages 279-289 (CRC Press, Inc. 1997)). Various additional diagnostic approaches are well-known to those of skill in the art (see, for example, Mathew (ed.), *Protocols in Human Molecular Genetics* (Humana Press, Inc. 1991), Coleman and Tsongalis, *Molecular Diagnostics* (Humana Press, Inc. 1996), and Elles, *Molecular Diagnosis of Genetic Diseases* (Humana Press, Inc., 1996)). Suitable test samples include blood, urine, saliva, tissue biopsy, and autopsy material.

Clinically significant polymorphisms of the human *ACE* gene have been discovered (see, for example, Matsusaka and Ichikawa, *Annu. Rev. Physiol. 59*:395 (1997)). A polymorphism associated with intron 16 is associated with plasma and intracellular levels of ACE, as well as increased risk of myocardial infarction. *ACE* polymorphisms are also associated with progression to chronic renal failure in IgA nephropathy, and diabetic nephropathy (Marre *et al., Diabetes 43*:384 (1994); Yoshida *et al., J. Clin. Invest. 96*:2162 (1995)). Other *ACE* gene mutations are associated with the risk of developing cardiovascular disease (Raynolds and Perryman, U.S. Patent No. 5,800, 990).

Nucleic acid molecules comprising *Zace1* nucleotide sequences can be used to determine whether a subject's chromosomes contain a mutation in the *Zace1* gene. Detectable chromosomal aberrations at the *Zace1* gene locus include, but are not limited to, aneuploidy, gene copy number changes, insertions, deletions, restriction site changes and rearrangements. Of particular interest are genetic alterations that inactivate the *Zace1* gene.

Aberrations associated with the *Zace1* locus can be detected using nucleic acid molecules of the present invention by employing molecular genetic techniques, such as restriction fragment length polymorphism (RFLP) analysis, short tandem repeat (STR) analysis employing PCR techniques, amplification-refractory mutation system analysis (ARMS), single-strand conformation polymorphism (SSCP) detection, RNase cleavage methods, denaturing gradient gel electrophoresis, fluorescence-assisted mismatch analysis (FAMA), and other genetic analysis techniques known in the art (see, for example, Mathew (ed.), *Protocols in Human Molecular Genetics* (Humana Press, Inc. 1991), Marian, *Chest 108*:255 (1995), Coleman and Tsongalis, *Molecular Diagnostics* (Human Press, Inc. 1996), Elles (ed.) *Molecular Diagnosis of Genetic Diseases* (Humana Press, Inc. 1996), Landegren (ed.), *Laboratory Protocols for Mutation Detection* (Oxford University Press 1996), Birren *et al.* (eds.), *Genome Analysis, Vol. 2: Detecting Genes* (Cold Spring Harbor Laboratory Press 1998), Dracopoli *et al.* (eds.), *Current Protocols in Human Genetics* (John Wiley & Sons 1998), and Richards and Ward, "Molecular Diagnostic Testing," in *Principles of Molecular Medicine,* pages 83-88 (Humana Press, Inc. 1998)).

The protein truncation test is also useful for detecting the inactivation of a gene in which translation-terminating mutations produce only portions of the encoded protein (see, for example, Stoppa-Lyonnet *et al., Blood 91*:3920 (1998)). According to this approach, RNA is isolated from a biological sample, and used to synthesize cDNA. PCR is then used to amplify the *Zace1* target sequence and to introduce an RNA polymerase promoter, a translation initiation sequence, and an in-frame ATG triplet. PCR products are transcribed using an RNA polymerase, and the transcripts are translated *in vitro* with a T7-coupled reticulocyte lysate system. The translation products are then fractionated by SDS-PAGE to determine the lengths of the translation products. The protein truncation test is described, for example, by Dracopoli *et al.* (eds.), *Current Protocols in Human Genetics,* pages 9.11.1 - 9.11.18 (John Wiley & Sons 1998). Protein truncation can also be examined by comparing Zac1 protein isolated from a subject with a polypeptide comprising the amino acid sequence disclosed herein.

Localization of the chromosomal location of the *Zace1* gene can be achieved using radiation hybrid mapping, which is a somatic cell genetic technique developed for constructing high-resolution, contiguous maps of mammalian chromosomes (Cox *et al., Science 250*:245 (1990)). Partial or full knowledge of a gene's sequence allows one to design PCR primers suitable for use with chromosomal radiation hybrid mapping panels. Radiation hybrid mapping panels are commercially available which cover the entire human genome, such as the Stanford G3 RH Panel and the GeneBridge 4 RH Panel (Research Genetics, Inc., Huntsville, AL). These panels enable rapid, PCR-based chromosomal localizations and ordering of genes, sequence-tagged sites, and other nonpolymorphic and polymorphic markers within a region of interest. This includes establishing directly proportional physical distances between newly discovered genes of interest and previously mapped markers.

The present invention also contemplates kits for performing a diagnostic assay for *Zace1* gene expression or to detect mutations in the *Zace1* gene. Such kits comprise nucleic acid probes, such as double-stranded nucleic acid molecules, as well as single-stranded nucleic acid molecules. Probe molecules may be DNA, RNA, oligonucleotides, and the like. Kits may comprise nucleic acid primers for performing PCR. A kit will comprise at least one container comprising a *Zace1* probe or primer. The kit may also comprise a second container comprising one or more reagents capable of indicating the presence of *Zace1* sequences. Examples of such indicator reagents include detectable labels such as radioactive labels, fluorochromes, chemiluminescent agents, and the like. A kit may also comprise a means for conveying to the user that the *Zace1* probes and primers are used to detect *Zace1* gene expression. For example, written instructions may state that the enclosed nucleic acid molecules can be used to detect either a nucleic acid molecule that encodes Zace1, or a nucleic acid molecule having a nucleotide sequence that is complementary to a *Zace1*-encoding nucleotide sequence. The written material can be applied directly to a container, or the written material can be provided in the form of a packaging insert.

### 11. Use of Anti-Zace1 Antibodies to Detect Zace1

Antibodies to Zace1 can be used for tagging cells that express Zace1, for isolating Zace1 or portions thereof by affinity purification, for diagnostic assays for determining circulating levels of Zace1 polypeptides, for detecting or quantitating Zace1 as a marker of underlying pathology or disease, in analytical methods employing FACS, for screening expression libraries, for generating anti-idiotypic antibodies, and as neutralizing antibodies or as antagonists to block Zace1 effects *in vitro* and *in vivo.* Suitable direct tags or labels include radionuclides, enzymes, substrates, cofactors, inhibitors, fluorescent markers, chemiluminescent markers, magnetic particles and the like; indirect tags or labels may feature use of biotin-avidin or other complement/anti-complement pairs as intermediates. Antibodies herein may also be directly or indirectly conjugated to drugs, toxins, radionuclides and the like, and these conjugates used for *in vivo* diagnostic or therapeutic applications. Moreover, antibodies to Zace1 or fragments thereof can be used *in vitro* to detect denatured Zace1 or fragments thereof in assays, for example, Western Blots or other assays known in the art.

Accordingly, the present invention contemplates the use of anti-Zace1 antibodies to screen biological samples *in vitro* for the presence of Zace1. In one type of *in vitro* assay, anti-Zace1 antibodies are used in liquid phase. For example, the presence of Zace1 in a biological sample can be tested by mixing the biological sample with a trace amount of labeled Zace1 and an anti-Zace1 antibody under conditions that promote binding between Zace1 and its antibody. Complexes of Zace1 and anti-Zace1 in the sample can be separated from the reaction mixture by contacting the complex with an immobilized protein which binds with the antibody, such as an Fc antibody or *Staphylococcus* protein A. The concentration of Zace 1 in the biological sample will be inversely proportional to the amount of labeled Zace1 bound to the antibody and directly related to the amount of free labeled Zace1. Illustrative biological samples include blood, urine, saliva, tissue biopsy, and autopsy material.

Alternatively, *in vitro* assays can be performed in which anti-Zace1 antibody is bound to a solid-phase carrier. For example, antibody can be attached to a polymer, such as aminodextran, in order to link the antibody to an insoluble support such as a polymer-coated bead, a plate or a tube. Other suitable *in vitro* assays will be readily apparent to those of skill in the art.

In another approach, anti-Zace1 antibodies can be used to detect Zace1 in tissue sections prepared from a biopsy specimen. Such immunochemical detection can be used to determine the relative abundance of Zace1 and to determine the distribution of Zace1 in the examined tissue. General immunochemistry techniques are well established (see, for example, Ponder, "Cell Marking Techniques and Their Application," in *Mammalian Development: A Practical Approach,* Monk (ed.), pages 115-38 (IRL Press 1987), Coligan at pages 5.8.1-5.8.8, Ausubel (1995) at pages 14.6.1 to 14.6.13 (Wiley Interscience 1990), and Manson (ed.), *Methods In Molecular Biology, Vol. 10: Immunochemical Protocols* (The Humana Press, Inc. 1992)).

Immunochemical detection can be performed by contacting a biological sample with an anti-Zace1 antibody, and then contacting the biological sample with a detectably labeled molecule which binds to the antibody. For example, the detectably labeled molecule can comprise an antibody moiety that binds to anti-Zace1 antibody. Alternatively, the anti-Zace1 antibody can be conjugated with avidin/streptavidin (or biotin) and the detectably labeled molecule can comprise biotin (or avidin/streptavidin). Numerous variations of this basic technique are well-known to those of skill in the art.

Alternatively, an anti-Zace1 antibody can be conjugated with a detectable label to form an anti-Zace1 immunoconjugate. Suitable detectable labels include, for example, a radioisotope, a fluorescent label, a chemiluminescent label, an enzyme label, a bioluminescent label or colloidal gold. Methods of making and detecting such detectably-labeled immunoconjugates are well-known to those of ordinary skill in the art, and are described in more detail below.

The detectable label can be a radioisotope that is detected by autoradiography. Isotopes that are particularly useful for the purpose of the present invention are ³H, ¹²⁵I, ¹³¹I, ³⁵S and ¹⁴C.

Anti-Zace1 immunoconjugates can also be labeled with a fluorescent compound. The presence of a fluorescently-labeled antibody is determined by exposing the immunoconjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine.

Alternatively, anti-Zace1 immunoconjugates can be detectably labeled by coupling an antibody component to a chemiluminescent compound. The presence of the chemiluminescent-tagged immunoconjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester.

Similarly, a bioluminescent compound can be used to label anti-Zace1 immunoconjugates of the present invention. Bioluminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin.

Alternatively, anti-Zace1 immunoconjugates can be detectably labeled by linking an anti-Zace 1 antibody component to an enzyme. When the anti-Zace1-enzyme conjugate is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include β-galac-tosidase, glucose oxidase, peroxidase and alkaline phosphatase.

Those of skill in the art will know of other suitable labels which can be employed in accordance with the present invention. The binding of marker moieties to anti-Zace1 antibodies can be accomplished using standard techniques known to the art. Typical methodology in this regard is described by Kennedy *et al., Clin. Chim. Acta 70*:1 (1976), Schurs *et al., Clin. Chim. Acta 81*:1 (1977), Shih *et al., Int'l J. Cancer 46*:1101 (1990), Stein *et al., Cancer Res. 50*:1330 (1990), and Coligan, *supra.*

Moreover, the convenience and versatility of immunochemical detection can be enhanced by using anti-Zace1 antibodies that have been conjugated with avidin, streptavidin, and biotin (see, for example, Wilchek *et al.* (eds.), "Avidin-Biotin Technology," *Methods In Enzymology, Vol. 184* (Academic Press 1990), and Bayer *et al.,* "Immunochemical Applications of Avidin-Biotin Technology," in *Methods In Molecular Biology, Vol. 10*, Manson (ed.), pages 149-162 (The Humana Press, Inc. 1992).

Methods for performing immunoassays are well-established. See, for example, Cook and Self, "Monoclonal Antibodies in Diagnostic Immunoassays," in *Monoclonal Antibodies: Production, Engineering, and Clinical Application,* Ritter and Ladyman (eds.), pages 180-208, (Cambridge University Press, 1995), Perry, "The Role of Monoclonal Antibodies in the Advancement of Immunoassay Technology," in *Monoclonal Antibodies: Principles and Applications,* Birch and Lennox (eds.), pages 107-120 (Wiley-Liss, Inc. 1995), and Diamandis. *Immunoassay* (Academic Press, Inc. 1996).

The present invention also contemplates kits for performing an immunological diagnostic assay for *Zace1* gene expression. Such kits comprise at least one container comprising an anti-Zace 1 antibody, or antibody fragment. A kit may also comprise a second container comprising one or more reagents capable of indicating the presence of Zace1 antibody or antibody fragments. Examples of such indicator reagents include detectable labels such as a radioactive label, a fluorescent label, a chemiluminescent label, an enzyme label, a bioluminescent label, colloidal gold, and the like. A kit may also comprise a means for conveying to the user that Zace1 antibodies or antibody fragments are used to detect Zace1 protein. For example, written instructions may state that the enclosed antibody or antibody fragment can be used to detect Zace1. The written material can be applied directly to a container, or the written material can be provided in the form of a packaging insert.

### 12. Bioactive Conjugates of Zace1 Polypeptides and Antibodies

The present invention includes antibodies or polypeptides that are directly or indirectly conjugated to drugs, toxins, radionuclides and the like, which can be used for *in vivo* diagnostic or therapeutic applications. For instance, polypeptides or antibodies of the present invention can be used to identify or treat tissues or organs that express a corresponding anti-complementary molecule (substrate, receptor, or antigen, respectively, for instance). More specifically, Zace1 polypeptides or anti-Zace1 antibodies, or bioactive fragments or portions thereof, can be coupled to detectable or cytotoxic molecules and delivered to a mammal having cells, tissues or organs that express the anti-complementary molecule.

Suitable detectable molecules may be directly or indirectly attached to the polypeptide or antibody, and include radionuclides, enzymes, substrates, cofactors, inhibitors, fluorescent markers, chemiluminescent markers, magnetic particles and the like. Suitable cytotoxic molecules may be directly or indirectly attached to the polypeptide or antibody, and include bacterial or plant toxins (for instance, diphtheria toxin, *Pseudomonas* exotoxin, ricin, abrin and the like), as well as therapeutic radionuclides, such as iodine-131, rhenium-188 or yttrium-90 (either directly attached to the polypeptide or antibody, or indirectly attached through means of a chelating moiety, for instance). Polypeptides or antibodies may also be conjugated to cytotoxic drugs, such as adriamycin. For indirect attachment of a detectable or cytotoxic molecule, the detectable or cytotoxic molecule can be conjugated with a member of a complementary/anticomplementary pair, where the other member is bound to the polypeptide or antibody portion. For these purposes, biotin/streptavidin is an exemplary complementary/ anticomplementary pair.

In another aspect of the invention, polypeptide-toxin fusion proteins or antibody-toxin fusion proteins can be used for targeted cell or tissue inhibition or ablation (for instance, to treat cancer cells or tissues). Alternatively, if the polypeptide has multiple functional domains (i.e., an activation domain or a substrate and/or ligand binding domain, plus a targeting domain), a fusion protein including only the targeting domain may be suitable for directing a detectable molecule, a cytotoxic molecule or a complementary molecule to a cell or tissue type of interest. In instances where the domain only fusion protein includes a complementary molecule, the anti-complementary molecule can be conjugated to a detectable or cytotoxic molecule. Such domain-complementary molecule fusion proteins thus represent a generic targeting vehicle for cell/tissue-specific delivery of generic anti-complementary-detectable/cytotoxic molecule conjugates.

In another embodiment, Zace1-cytokine fusion proteins or anti-Zace1-cytokine fusion proteins can be used for enhancing *in vivo* killing of target tissues, if the Zace1 polypeptide or anti-Zace1 antibody targets the hyperproliferative target cell. For example, Hornick *et al., Blood 89*:4437 (1997), described fusion proteins that enable targeting of a cytokine to a desired site of action, thereby providing an elevated local concentration of cytokine. Suitable cytokines for this purpose include interleukin-2 and granulocyte-macrophage colony-stimulating factor (GM-CSF), and other immunomodulators, for instance. Suitable Zace1 polypeptides or anti-Zace1 antibodies target an undesirable cell or tissue (i.e., a hyperproliferative vascular epithelial cell or a transformed cell). Such polypeptide or antibody can be conjugated with a radionuclide, and particularly with a β-emitting radionuclide, to reduce restenosis or transformed cell mass. Such therapeutic approach poses less danger to clinicians who administer the radioactive therapy. For instance, iridium-192 impregnated ribbons placed into stented vessels of patients until the required radiation dose was delivered showed decreased tissue growth in the vessel and greater luminal diameter than the control group, which received placebo ribbons. Further, revascularisation and stent thrombosis were significantly lower in the treatment group. Similar results are predicted with targeting of a bioactive conjugate containing a radionuclide, as described herein.

The bioactive polypeptide or antibody conjugates described herein can be delivered intravenously, intraarterially or intraductally, or may be introduced locally at the intended site of action. Suitable modes of administration of therapeutic proteins are described below.

### 13. Therapeutic Uses of Polypeptides Having Zace1 Activity

The present invention includes the use of proteins, polypeptides, and peptides having Zace1 activity (such as Zace1 polypeptides (*e.g.*, soluble forms of Zace1), Zace1 analogs (*e.g.*, anti-Zace1 anti-idiotype antibodies), and Zace1 fusion proteins) to a subject who lacks an adequate amount of this polypeptide. In contrast, Zace1 antagonists (*e.g.*, anti-Zace1 antibodies) can be used to treat a subject who produces an excess of Zace1.

The kallikrein-kinin (contact) system modulates the renin-angiotensin-aldosterone system, prostaglandins, vasopressins, sodium-water balance, renal hemodynamics, and blood pressure. Stadnicki *et al., FASEB J. 12*:325 (1998), have shown that a reversible inhibitor of plasma kallikrein decreased chronic intestinal inflammation in an experimental model relevant to Crohn's disease. One of the actions of kallikrein is to cleave high molecular weight kininogen to produce bradykinin, a peptide that enhances vasodilation, increases vascular permeability, and influences intestinal motility and electrolyte secretion (see, for example, Bhoola *et al., Pharmacol. Rev. 44*:1 (1992)). The inhibition of kallikrein by the reversible inhibitor, therefore, should decrease bradykinin activity levels, which is consistent with evidence that kinins mediate gastrointestinal inflammation associated with inflammatory bowel disease, such as Crohn's disease (see, for example, Bachvarov *et al., Gastroenterology 115*:1045 (1998)).

ACE also decreases bradykinin activity by cleaving the peptide. Accordingly, decreased ACE activity should be correlated with increased bradykinin activity. Studies have shown that serum ACE activity is significantly lowered in certain patients who have active Crohn's disease (see, for example, Silverstein *et al., Am. J. Clin. Pathol. 75*:175 (1981); Sommer *et al., Enzyme 35*:181 (1986)). Taken together, these observations indicate that ACE can be used to treat conditions associated with inflammation, such as inflammatory bowel disease.

The present invention therefore includes the use of polypeptides having Zace1 activity (*e.g.*, Zace1 polypeptides, functional fragments of Zace1, anti-Zace1 anti-idiotype antibodies, *etc.*) to treat an inflammatory bowel disease (*e.g.*, Crohn's disease and ulcerative colitis). More generally, the present invention includes the use of polypeptides having Zace1 activity to treat diseases associated with inflammation, such as arthritis and enterocolitis, two conditions which have been treated with a kallikrein inhibitor (see, for example, DeLa *Cadena et al., FASEB J*. *9*:446 (1995); Stadnicki *et al., Dig. Dis. Sci. 41*:912 (1996)). Methods for identification of subjects suitable for such treatment are well known to those of skill in the art (see, for example, Rakel (ed.), *Conn's 1999 Current Therapy* (W.B. Saunders Company 1999)).

Generally, the dosage of administered Zace1 (or Zace1 analog or fusion protein) will vary depending upon such factors as the patient's age, weight, height, sex, general medical condition and previous medical history. Typically, it is desirable to provide the recipient with a dosage of Zace1 which is in the range of from about 1 pg/kg to 10 mg/kg (amount of agent/body weight of patient), although a lower or higher dosage also may be administered as circumstances dictate.

Administration of a molecule having Zace1 activity to a subject can be intravenous, intraarterial, intraperitoneal, intramuscular, subcutaneous, intrapleural, intrathecal, by perfusion through a regional catheter, or by direct intralesional injection. Regional administration is particularly useful for treatment of an inflammatory bowel disease. When administering therapeutic proteins by injection, the administration may be by continuous infusion or by single or multiple boluses.

Additional routes of administration include oral, mucosal-membrane, pulmonary, and transcutaneous. Oral delivery is suitable for polyester microspheres, zein microspheres, proteinoid microspheres, polycyanoacrylate microspheres, and lipid-based systems (see, for example, DiBase and Morrel, "Oral Delivery of Microencapsulated Proteins," in *Protein Delivery: Physical Systems,* Sanders and Hendren (eds.), pages 255-288 (Plenum Press 1997)). The feasibility of an intranasal delivery is exemplified by such a mode of insulin administration (see, for example, Hinchcliffe and Illum, *Adv. Drug Deliv. Rev. 35*:199 (1999)). Dry or liquid particles comprising Zace1 can be prepared and inhaled with the aid of dry-powder dispersers, liquid aerosol generators, or nebulizers (*e.g.*, Pettit and Gombotz, *TIBTECH 16*:343 (1998); Patton *et al., Adv. Drug Deliv. Rev. 35*:235 (1999)). This approach is illustrated by the AERX diabetes management system, which is a hand-held electronic inhaler that delivers aerosolized insulin into the lungs. Studies have shown that proteins as large as 48,000 kDa have been delivered across skin at therapeutic concentrations with the aid of low-frequency ultrasound, which illustrates the feasibility of trascutaneous administration (Mitragotri *et al., Science 269*:850 (1995)). Transdermal delivery using electroporation provides another means to administer a molecule having Zace1 activity (Potts *et al., Pharm. Biotechnol. 10*:213 (1997)).

A pharmaceutical composition comprising a protein, polypeptide, or peptide having Zace1 activity can be formulated according to known methods to prepare pharmaceutically useful compositions, whereby the therapeutic proteins are combined in a mixture with a pharmaceutically acceptable carrier. A composition is said to be a "pharmaceutically acceptable carrier" if its administration can be tolerated by a recipient patient. Sterile phosphate-buffered saline is one example of a pharmaceutically acceptable carrier. Other suitable carriers, such as 5% dextrose in water, are well-known to those in the art. Formulations can further include one or more excipients, preservatives, solubilizers, buffering agents, albumin to prevent protein loss on vial surfaces, *etc.* Methods of formulation are well known in the art and are disclosed, for example, by Gennaro (ed.), *Remington's Pharmaceutical Sciences,* 19th Edition (Mack Publishing Company 1995).

For purposes of therapy, molecules having Zace1 activity and a pharmaceutically acceptable carrier are administered to a patient in a therapeutically effective amount. A combination of a protein, polypeptide, or peptide having Zace1 activity and a pharmaceutically acceptable carrier is said to be administered in a "therapeutically effective amount" if the amount administered is physiologically significant. An agent is physiologically significant if its presence results in a detectable change in the physiology of a recipient patient. For example, common symptoms of Crohn's disease include chronic diarrhea with abdominal pain, fever, anorexia, weight loss, and a right lower quadrant mass. An agent used to treat Crohn's disease is physiologically significant if its presence alleviates at least one of these symptoms.

A pharmaceutical composition comprising Zace1 (or Zace1 analog or fusion protein) can be furnished in liquid form, in an aerosol, or in solid form. Liquid forms, are illustrated by injectable solutions and oral suspensions. Exemplary solid forms include capsules, tablets, and controlled-release forms. The latter form is illustrated by miniosmotic pumps and implants (Bremer *et al., Pharm. Biotechnol. 10*:239 (1997); Ranade, "Implants in Drug Delivery," in *Drug Delivery Systems,* Ranade and Hollinger (eds.), pages 95-123 (CRC Press 1995); Bremer *et al.*, "Protein Delivery with Infusion Pumps," in *Protein Delivery: Physical Systems,* Sanders and Hendren (eds.), pages 239-254 (Plenum Press 1997); Yewey *et al.,* "Delivery of Proteins from a Controlled Release Injectable Implant," in *Protein Delivery: Physical Systems,* Sanders and Hendren (eds.), pages 93-117 (Plenum Press 1997)).

Liposomes provide one means to deliver therapeutic polypeptides to a subject intravenously, intraperitoneally, intrathecally, intramuscularly, subcutaneously, or via oral administration, inhalation, or intranasal administration. Liposomes are microscopic vesicles that consist of one or more lipid bilayers surrounding aqueous compartments (see, generally, Bakker-Woudenberg *et al., Eur. J. Clin. Microbiol. Infect. Dis. 12 (Suppl. 1)*:S61 (1993), Kim, *Drugs 46*:618 (1993), and Ranade, "Site-Specific Drug Delivery Using Liposomes as Carriers," in *Drug Delivery Systems,* Ranade and Hollinger (eds.), pages 3-24 (CRC Press 1995)). Liposomes are similar in composition to cellular membranes and as a result, liposomes can be administered safely and are biodegradable. Depending on the method of preparation, liposomes may be unilamellar or multilamellar, and liposomes can vary in size with diameters ranging from 0.02 µm to greater than 10 µm. A variety of agents can be encapsulated in liposomes: hydrophobic agents partition in the bilayers and hydrophilic agents partition within the inner aqueous space(s) (see, for example, Machy *et al., Liposomes In Cell Biology And Pharmacology* (John Libbey 1987), and Ostro *et al., American J. Hosp. Pharm. 46*:1576 (1989)). Moreover, it is possible to control the therapeutic availability of the encapsulated agent by varying liposome size, the number of bilayers, lipid composition, as well as the charge and surface characteristics of the liposomes.

Liposomes can adsorb to virtually any type of cell and then slowly release the encapsulated agent. Alternatively, an absorbed liposome may be endocytosed by cells that are phagocytic. Endocytosis is followed by intralysosomal degradation of liposomal lipids and release of the encapsulated agents (Scherphof *et al., Ann. N.Y. Acad Sci. 446*:368 (1985)). After intravenous administration, small liposomes (0.1 to 1.0 µm) are typically taken up by cells of the reticuloendothelial system, located principally in the liver and spleen, whereas liposomes larger than 3.0 µm are deposited in the lung. This preferential uptake of smaller liposomes by the cells of the reticuloendothelial system has been used to deliver chemotherapeutic agents to macrophages and to tumors of the liver.

The reticuloendothelial system can be circumvented by several methods including saturation with large doses of liposome particles, or selective macrophage inactivation by pharmacological means (Claassen *et al., Biochim. Biophys. Acta 802*:428 (1984)). In addition, incorporation of glycolipid- or polyethelene glycol-derivatized phospholipids into liposome membranes has been shown to result in a significantly reduced uptake by the reticuloendothelial system (*Allen et al., Biochim. Biophys. Acta 1068*:133 (1991); Allen *et al., Biochim. Biophys. Acta 1150*:9 (1993)).

Liposomes can also be prepared to target particular cells or organs by varying phospholipid composition or by inserting receptors or ligands into the liposomes. For example, liposomes, prepared with a high content of a nonionic surfactant, have been used to target the liver (Hayakawa *et al.,* Japanese Patent 04-244,018; Kato *et al., Biol. Pharm. Bull. 16*:960 (1993)). These formulations were prepared by mixing soybean phospatidylcholine, α-tocopherol, and ethoxylated hydrogenated castor oil (HCO-60) in methanol, concentrating the mixture under vacuum, and then reconstituting the mixture with water. A liposomal formulation of dipalmitoylphosphatidylcholine (DPPC) with a soybean-derived sterylglucoside mixture (SG) and cholesterol (Ch) has also been shown to target the liver (Shimizu *et al., Biol. Pharm. Bull. 20*:881 (1997)).

Alternatively, various targeting ligands can be bound to the surface of the liposome, such as antibodies, antibody fragments, carbohydrates, vitamins, and transport proteins. For example, liposomes can be modified with branched type galactosyllipid derivatives to target asialoglycoprotein (galactose) receptors, which are exclusively expressed on the surface of liver cells (Kato and Sugiyama, *Crit. Rev. Ther. Drug Carrier Syst. 14*:287 (1997); Murahashi *et al., Biol. Pharm. Bull.20*:259 (1997)). Similarly, Wu *et al., Hepatology 27*:772 (1998), have shown that labeling liposomes with asialofetuin led to a shortened liposome plasma half-life and greatly enhanced uptake of asialofetuin-labeled liposome by hepatocytes. On the other hand, hepatic accumulation of liposomes comprising branched type galactosyllipid derivatives can be inhibited by preinjection of asialofetuin (Murahashi *et al., Biol. Pharm. Bull.20*:259 (1997)). Polyaconitylated human serum albumin liposomes provide another approach for targeting liposomes to liver cells (Kamps *et al., Proc. Nat'l Acad. Sci. USA 94*:11681 (1997)). Moreover, *Geho, et al.* U.S. Patent No. 4,603,044, describe a hepatocyte-directed liposome vesicle delivery system, which has specificity for hepatobiliary receptors associated with the specialized metabolic cells of the liver.

In a more general approach to tissue targeting, target cells are prelabeled with biotinylated antibodies specific for a ligand expressed by the target cell (Harasym *et al., Adv. Drug Deliv. Rev. 32*:99 (1998)). After plasma elimination of free antibody, streptavidin-conjugated liposomes are administered. In another approach, targeting antibodies are directly attached to liposomes (Harasym *et al., Adv. Drug Deliv. Rev. 32*:99 (1998)).

Polypeptides having Zace1 activity can be encapsulated within liposomes using standard techniques of protein microencapsulation (see, for example, Anderson *et al., Infect. Immun. 31*:1099 (1981), Anderson *et al., Cancer Res. 50*:1853 (1990), and Cohen *et al., Biochim. Biophys. Acta 1063*:95 (1991), Alving *et al.* "Preparation and Use of Liposomes in Immunological Studies," in *Liposome Technology,* 2nd Edition, Vol. III, Gregoriadis (ed.), page 317 (CRC Press 1993), Wassef *et al., Meth. Enzymol. 149*:124 (1987)). As noted above, therapeutically useful liposomes may contain a variety of components. For example, liposomes may comprise lipid derivatives of poly(ethylene glycol) (Allen *et al., Biochim. Biophys. Acta 1150*:9 (1993)).

Degradable polymer microspheres have been designed to maintain high systemic levels of therapeutic proteins. Microspheres are prepared from degradable polymers such as poly(lactide-co-glycolide) (PLG), polyanhydrides, poly (ortho esters), nonbiodegradable ethylvinyl acetate polymers, in which proteins are entrapped in the polymer (Gombotz and Pettit, *Bioconjugate Chem. 6*:332 (1995); Ranade, "Role of Polymers in Drug Delivery," in *Drug Delivery Systems,* Ranade and Hollinger (eds.), pages 51-93 (CRC Press 1995); Roskos and Maskiewicz, "Degradable Controlled Release Systems Useful for Protein Delivery," in *Protein Delivery: Physical Systems,* Sanders and Hendren (eds.), pages 45-92 (Plenum Press 1997); Bartus *et al., Science 281*:1161 (1998); Putney and Burke, *Nature Biotechnology 16*:153 (1998); Putney, *Curr. Opin. Chem. Biol. 2*:548 (1998)). Polyethylene glycol (PEG)-coated nanospheres can also provide carriers for intravenous administration of therapeutic proteins (see, for example, Gref *et al., Pharm. Biotechnol. 10*:167 (1997)).

The present invention also contemplates chemically modified polypeptides having Zace1 activity and Zace1 antagonists, in which a polypeptide is linked with a polymer, as discussed above.

Other dosage forms can be devised by those skilled in the art, as shown, for example, by Ansel and Popovich, *Pharmaceutical Dosage Forms and Drug Delivery Systems,* 5^{th} Edition (Lea & Febiger 1990), Gennaro (ed.), *Remington's Pharmaceutical Sciences,* 19^{th} Edition (Mack Publishing Company 1995), and by Ranade and Hollinger, *Drug Delivery Systems* (CRC Press 1996).

As an illustration, pharmaceutical compositions may be supplied as a kit comprising a container that comprises a molecule having Zace1 activity or a Zace1 antagonist (*e.g.*, an antibody or antibody fragment that binds a Zace1 polypeptide). Therapeutic polypeptides can be provided in the form of an injectable solution for single or multiple doses, or as a sterile powder that will be reconstituted before injection. Alternatively, such a kit can include a dry-powder disperser, liquid aerosol generator, or nebulizer for administration of a therapeutic polypeptide. Such a kit may further comprise written information on indications and usage of the pharmaceutical composition. Moreover, such information may include a statement that the Zace1 composition is contraindicated in patients with known hypersensitivity to Zace1.

### 14. Therapeutic Uses of Zace1 Nucleotide Sequences

The present invention includes the use of *Zace1* nucleotide sequences to provide Zace1 to a subject in need of such treatment. In addition, a therapeutic expression vector can be provided that inhibits *Zace1* gene expression, such as an anti-sense molecule, a ribozyme, or an external guide sequence molecule.

There are numerous approaches to introduce a *Zace1* gene to a subject, including the use of recombinant host cells that express *Zace1,* delivery of naked nucleic acid encoding Zace1, use of a cationic lipid carrier with a nucleic acid molecule that encodes Zace1, and the use of viruses that express *Zace1,* such as recombinant retroviruses, recombinant adeno-associated viruses, recombinant adenoviruses, and recombinant Herpes simplex viruses (see, for example, Mulligan, *Science 260*:926 (1993), Rosenberg *et al., Science 242*:1575 (1988), LaSalle *et al., Science 259*:988 (1993), Wolff *et al., Science 247*:1465 (1990), Breakfield and Deluca, *The New Biologist 3*:203 (1991)). In an *ex vivo* approach, for example, cells are isolated from a subject, transfected with a vector that expresses a *Zace1* gene, and then transplanted into the subject.

In order to effect expression of a *Zace1* gene, an expression vector is constructed in which a nucleotide sequence encoding a *Zace1* gene is operably linked to a core promoter, and optionally a regulatory element, to control gene transcription. The general requirements of an expression vector are described above.

Alternatively, a *Zace1* gene can be delivered using recombinant viral vectors, including for example, adenoviral vectors (*e.g*., Kass-Eisler *et al., Proc. Nat'l Acad. Sci. USA 90*:11498 (1993), Kolls *et al., Proc. Nat'l Acad. Sci. USA 91*:215 (1994), Li *et al., Hum. Gene Ther. 4*:403 (1993), Vincent *et al., Nat. Genet. 5*:130 (1993), and Zabner *et al., Cell 75*:207 (1993)), adenovirus-associated viral vectors (Flotte *et al., Proc. Nat'l Acad. Sci. USA 90*:10613 (1993)), alphaviruses such as Semliki Forest Virus and Sindbis Virus (Hertz and Huang, *J. Vir. 66*:857 (1992), Raju and Huang, *J. Vir. 65*:2501 (1991), and Xiong *et al., Science* 243:1188 (1989)), herpes viral vectors (*e.g.*, U.S. Patent Nos. 4,769,331, 4,859,587, 5,288,641 and 5,328,688), parvovirus vectors (Koering *et al., Hum. Gene Therap. 5*:457 (1994)), pox virus vectors (Ozaki *et al., Biochem. Biophys. Res. Comm. 193*:653 (1993), Panicali and Paoletti, *Proc. Nat'l Acad. Sci. USA 79*:4927 (1982)), pox viruses, such as canary pox virus or vaccinia virus (Fisher-Hoch *et al., Proc. Nat'l Acad. Sci. USA 86*:317 (1989), and Flexner *et al., Ann. N. Y. Acad. Sci. 569*:86 (1989)), and retroviruses (*e.g.*, Baba *et al., J. Neurosurg 79*:729 (1993), Ram *et al., Cancer Res. 53*:83 (1993), Takamiya *et al., J. Neurosci. Res 33*:493 (1992), Vile and Hart, *Cancer Res. 53*:962 (1993), Vile and Hart, *Cancer Res. 53*:3860 (1993), and Anderson *et al.,* U.S. Patent No. 5,399,346). Within various embodiments, either the viral vector itself, or a viral particle which contains the viral vector may be utilized in the methods and compositions described below.

As an illustration of one system, adenovirus, a double-stranded DNA virus, is a well-characterized gene transfer vector for delivery of a heterologous nucleic acid molecule (for a review, see Becker *et al., Meth. Cell Biol. 43*:161 (1994); Douglas and Curiel, *Science & Medicine 4*:44 (1997)). The adenovirus system offers several advantages including: (i) the ability to accommodate relatively large DNA inserts, (ii) the ability to be grown to high-titer, (iii) the ability to infect a broad range of mammalian cell types, and (iv) the ability to be used with many different promoters including ubiquitous, tissue specific, and regulatable promoters. In addition, adenoviruses can be administered by intravenous injection, because the viruses are stable in the bloodstream.

Using adenovirus vectors where portions of the adenovirus genome are deleted, inserts are incorporated into the viral DNA by direct ligation or by homologous recombination with a co-transfected plasmid. In an exemplary system, the essential E1 gene is deleted from the viral vector, and the virus will not replicate unless the E1 gene is provided by the host cell. When intravenously administered to intact animals, adenovirus primarily targets the liver. Although an adenoviral delivery system with an E1 gene deletion cannot replicate in the host cells, the host's tissue will express and process an encoded heterologous protein. Host cells will also secrete the heterologous protein if the corresponding gene includes a secretory signal sequence. Secreted proteins will enter the circulation from tissue that expresses the heterologous gene (*e.g.*, the highly vascularized liver).

Moreover, adenoviral vectors containing various deletions of viral genes can be used to reduce or eliminate immune responses to the vector. Such adenoviruses are E1-deleted, and in addition, contain deletions of E2A or E4 (*Lusky et al., J. Virol. 72*:2022 (1998); Raper *et al., Human Gene Therapy 9*:671 (1998)). The deletion of E2b has also been reported to reduce immune responses (Amalfitano *et al., J. Virol. 72*:926 (1998)). By deleting the entire adenovirus genome, very large inserts of heterologous DNA can be accommodated. Generation of so called "gutless" adenoviruses, where all viral genes are deleted, are particularly advantageous for insertion of large inserts of heterologous DNA (for a review, see Yeh. and Perricaudet, *FASEB J. 11*:615 (1997)).

High titer stocks of recombinant viruses capable of expressing a therapeutic gene can be obtained from infected mammalian cells using standard methods. For example, recombinant herpes simplex virus can be prepared in Vero cells, as described by Brandt *et al., J. Gen. Virol. 72*:2043 (1991), Herold *et al., J. Gen. Virol. 75*:1211 (1994), Visalli and Brandt, *Virology 185*:419 (1991), Grau *et al., Invest. Ophthalmol. Vis. Sci. 30*:2474 (1989), Brandt *et al., J. Virol. Meth. 36*:209 (1992), and by Brown and MacLean (eds.), *HSV Virus Protocols* (Humana Press 1997).

Alternatively, an expression vector comprising a *Zace1* gene can be introduced into a subject's cells by lipofection *in vivo* using liposomes. Synthetic cationic lipids can be used to prepare liposomes for *in vivo* transfection of a gene encoding a marker (Felgner *et al., Proc. Nat'l Acad. Sci. USA 84*:7413 (1987); Mackey *et al., Proc. Nat'l Acad. Sci. USA 85*:8027 (1988)). The use of lipofection to introduce exogenous genes into specific organs *in vivo* has certain practical advantages. Liposomes can be used to direct transfection to particular cell types, which is particularly advantageous in a tissue with cellular heterogeneity, such as the pancreas, liver, kidney, and brain. Lipids may be chemically coupled to other molecules for the purpose of targeting. Targeted peptides (e.g., hormones or neurotransmitters), proteins such as antibodies, or non-peptide molecules can be coupled to liposomes chemically.

Electroporation is another alternative mode of administration. For example, Aihara and Miyazaki, *Nature Biotechnology 16*:867 (1998), have demonstrated the use of *in vivo* electroporation for gene transfer into muscle.

In an alternative approach to gene therapy, a therapeutic gene may encode a *Zace1* anti-sense RNA that inhibits the expression of *Zace1.* Suitable sequences for anti-sense molecules can be derived from the nucleotide sequences of *Zace1* disclosed herein.

Alternatively, an expression vector can be constructed in which a regulatory element is operably linked to a nucleotide sequence that encodes a ribozyme. Ribozymes can be designed to express endonuclease activity that is directed to a certain target sequence in a mRNA molecule (see, for example, Draper and Macejak, U.S. Patent No. 5,496,698, McSwiggen, U.S. Patent No. 5,525,468, Chowrira and McSwiggen, U.S. Patent No. 5,631,359, and Robertson and Goldberg, U.S. Patent No. 5,225,337). In the context of the present invention, ribozymes include nucleotide sequences that bind with *Zace1* mRNA.

In another approach, expression vectors can be constructed in which a regulatory element directs the production of RNA transcripts capable of promoting RNase P-mediated cleavage of mRNA molecules that encode a *Zace1* gene. According to this approach, an external guide sequence can be constructed for directing the endogenous ribozyme, RNase P, to a particular species of intracellular mRNA, which is subsequently cleaved by the cellular ribozyme (see, for example, Altman *et al*., U.S. Patent No. 5,168,053, Yuan *et al., Science 263*:1269 (1994), Pace *et al.,* international publication No. WO 96/18733, George *et al.*, international publication No. WO 96/21731, and Werner *et al.,* international publication No. WO 97/33991). Preferably, the external guide sequence comprises a ten to fifteen nucleotide sequence complementary to *Zace1* mRNA, and a 3'-NCCA nucleotide sequence, wherein N is preferably a purine. The external guide sequence transcripts bind to the targeted mRNA species by the formation of base pairs between the mRNA and the complementary external guide sequences, thus promoting cleavage of mRNA by RNase P at the nucleotide located at the 5'-side of the base-paired region.

In general, the dosage of a composition comprising a therapeutic vector having a *Zace1* nucleotide acid sequence, such as a recombinant virus, will vary depending upon such factors as the subject's age, weight, height, sex, general medical condition and previous medical history. Suitable routes of administration of therapeutic vectors include intravenous injection. intraarterial injection, intraperitoneal injection, intramuscular injection, intratumoral injection. and injection into a cavity that contains a tumor. As an illustration, Horton *et al., Proc. Nat'l Acad. Sci. USA 96*:1553 (1999), demonstrated that intramuscular injection of plasmid DNA encoding interferon-α produces potent antitumor effects on primary and metastatic tumors in a murine model.

A composition comprising viral vectors, non-viral vectors, or a combination of viral and non-viral vectors of the present invention can be formulated according to known methods to prepare pharmaceutically useful compositions, whereby vectors or viruses are combined in a mixture with a pharmaceutically acceptable carrier. As noted above, a composition, such as phosphate-buffered saline is said to be a "pharmaceutically acceptable carrier" if its administration can be tolerated by a recipient subject. Other suitable carriers are well-known to those in the art (see, for example, *Remington's Pharmaceutical Sciences, 19th Ed.* (Mack Publishing Co. 1995), and *Gilman's the Pharmacological Basis of Therapeutics, 7th Ed.* (MacMillan Publishing Co. 1985)).

For purposes of therapy, a therapeutic gene expression vector, or a recombinant virus comprising such a vector, and a pharmaceutically acceptable carrier are administered to a subject in a therapeutically effective amount. A combination of an expression vector (or virus) and a pharmaceutically acceptable carrier is said to be administered in a "therapeutically effective amount" if the amount administered is physiologically significant. An agent is physiologically significant if its presence results in a detectable change in the physiology of a recipient subject. For example, common symptoms of Crohn's disease include chronic diarrhea with abdominal pain, fever, anorexia, weight loss, and a right lower quadrant mass. An agent used to treat Crohn's disease is physiologically significant if its presence alleviates at least one of these symptoms.

When the subject treated with a therapeutic gene expression vector or a recombinant virus is a human, then the therapy is preferably somatic cell gene therapy. That is, the preferred treatment of a human with a therapeutic gene expression vector or a recombinant virus does not entail introducing into cells a nucleic acid molecule that can form part of a human germ line and be passed onto successive generations (*i.e.*, human germ line gene therapy).

### 15. Production of Transgenic Mice

Transgenic mice can be engineered to over-express the *Zace1* gene in all tissues or under the control of a tissue-specific or tissue-preferred regulatory element. These over-producers of Zace1 can be used to characterize the phenotype that results from over-expression, and the transgenic animals can serve as models for human disease caused by excess Zace1. Transgenic mice that over-express Zace1 also provide model bioreactors for production of Zace1 in the milk or blood of larger animals. Methods for producing transgenic mice are well-known to those of skill in the art (see, for example, Jacob, "Expression and Knockout of Interferons in Transgenic Mice," in *Overexpression and Knockout of Cytokines in Transgenic Mice,* Jacob (ed.), pages 111-124 (Academic Press, Ltd. 1994), Monastersky and Robl (eds.), *Strategies in Transgenic Animal Science* (ASM Press 1995), and Abbud and Nilson, "Recombinant Protein Expression in Transgenic Mice," in *Gene Expression Systems: Using Nature for the Art of Expression,* Fernandez and Hoeffler (eds.), pages 367-397 (Academic Press, Inc. 1999)).

For example, a method for producing a transgenic mouse that expresses a *Zace1* gene can begin with adult, fertile males (studs) (B6C3f1, 2-8 months of age (Taconic Farms, Germantown, NY)), vasectomized males (duds) (B6D2f1, 2-8 months, (Taconic Farms)), prepubescent fertile females (donors) (B6C3f1, 4-5 weeks, (Taconic Farms)) and adult fertile females (recipients) (B6D2f1, 2-4 months, (Taconic Farms)). The donors are acclimated for one week and then injected with approximately 8 IU/mouse of Pregnant Mare's Serum gonadotrophin (Sigma Chemical Company; St. Louis, MO) I.P., and 46-47 hours later, 8 IU/mouse of human Chorionic Gonadotropin (hCG (Sigma)) I.P. to induce superovulation. Donors are mated with studs subsequent to hormone injections. Ovulation generally occurs within 13 hours of hCG injection. Copulation is confirmed by the presence of a vaginal plug the morning following mating.

Fertilized eggs are collected under a surgical scope. The oviducts are collected and eggs are released into urinanalysis slides containing hyaluronidase (Sigma). Eggs are washed once in hyaluronidase, and twice in Whitten's W640 medium (described, for example, by Menino and O'Claray, *Biol. Reprod. 77*:159 (1986), and Dienhart and Downs, *Zygote 4*:129 (1996)) that has been incubated with 5% CO₂, 5% O₂, and 90% N₂ at 37°C. The eggs are then stored in a 37°C/5% CO₂ incubator until microinjection.

Ten to twenty micrograms of plasmid DNA containing a *Zace1* encoding sequence is linearized, gel-purified, and resuspended in 10 mM Tris-HCl (pH 7.4), 0.25 mM EDTA (pH 8.0), at a final concentration of 5-10 nanograms per microliter for microinjection. For example, the *Zace1* encoding sequences can encode a polypeptide comprising the amino acid residues of SEQ ID NO:1, or a fragment thereof.

Plasmid DNA is microinjected into harvested eggs contained in a drop of W640 medium overlaid by warm, CO₂-equilibrated mineral oil. The DNA is drawn into an injection needle (pulled from a 0.75mm ID, 1mm OD borosilicate glass capillary), and injected into individual eggs. Each egg is penetrated with the injection needle, into one or both of the haploid pronuclei.

Picoliters of DNA are injected into the pronuclei, and the injection needle withdrawn without coming into contact with the nucleoli. The procedure is repeated until all the eggs are injected. Successfully microinjected eggs are transferred into an organ tissue-culture dish with pre-gassed W640 medium for storage overnight in a 37°C/5% CO₂ incubator.

The following day, two-cell embryos are transferred into pseudopregnant recipients. The recipients are identified by the presence of copulation plugs, after copulating with vasectomized duds. Recipients are anesthetized and shaved on the dorsal left side and transferred to a surgical microscope. A small incision is made in the skin and through the muscle wall in the middle of the abdominal area outlined by the ribcage, the saddle, and the hind leg, midway between knee and spleen. The reproductive organs are exteriorized onto a small surgical drape. The fat pad is stretched out over the surgical drape, and a baby serrefine (Roboz, Rockville, MD) is attached to the fat pad and left hanging over the back of the mouse, preventing the organs from sliding back in.

With a fine transfer pipette containing mineral oil followed by alternating W640 and air bubbles, 12-17 healthy two-cell embryos from the previous day's injection are transferred into the recipient. The swollen ampulla is located and holding the oviduct between the ampulla and the bursa, a nick in the oviduct is made with a 28 g needle close to the bursa, making sure not to tear the ampulla or the bursa.

The pipette is transferred into the nick in the oviduct, and the embryos are blown in, allowing the first air bubble to escape the pipette. The fat pad is gently pushed into the peritoneum, and the reproductive organs allowed to slide in. The peritoneal wall is closed with one suture and the skin closed with a wound clip. The mice recuperate on a 37°C slide warmer for a minimum of four hours.

The recipients are returned to cages in pairs, and allowed 19-21 days gestation. After birth, 19-21 days postpartum is allowed before weaning. The weanlings are sexed and placed into separate sex cages, and a 0.5 cm biopsy (used for genotyping) is snipped off the tail with clean scissors.

Genomic DNA is prepared from the tail snips using, for example, a QIAGEN DNEASY kit following the manufacturer's instructions. Genomic DNA is analyzed by PCR using primers designed to amplify a *Zace1* gene or a selectable marker gene that was introduced in the same plasmid. After animals are confirmed to be transgenic, they are back-crossed into an inbred strain by placing a transgenic female with a wild-type male, or a transgenic male with one or two wild-type female(s). As pups are born and weaned, the sexes are separated, and their tails snipped for genotyping.

To check for expression of a transgene in a live animal, a partial hepatectomy is performed. A surgical prep is made of the upper abdomen directly below the zyphoid process. Using sterile technique, a small 1.5-2 cm incision is made below the sternum and the left lateral lobe of the liver exteriorized. Using 4-0 silk, a tie is made around the lower lobe securing it outside the body cavity. An atraumatic clamp is used to hold the tie while a second loop of absorbable Dexon (American Cyanamid; Wayne, N.J.) is placed proximal to the first tie. A distal cut is made from the Dexon tie and approximately 100 mg of the excised liver tissue is placed in a sterile petri dish. The excised liver section is transferred to a 14 ml polypropylene round bottom tube and snap frozen in liquid nitrogen and then stored on dry ice. The surgical site is closed with suture and wound clips, and the animal's cage placed on a 37°C heating pad for 24 hours post operatively. The animal is checked daily post operatively and the wound clips removed 7-10 days after surgery. The expression level of Zace1 mRNA is examined for each transgenic mouse using an RNA solution hybridization assay or polymerase chain reaction.

In addition to producing transgenic mice that over-express Zace1, it is useful to engineer transgenic mice with either abnormally low or no expression of the gene. Such transgenic mice provide useful models for diseases associated with a lack of Zace1. As discussed above, *Zace1* gene expression can be inhibited using anti-sense genes, ribozyme genes, or external guide sequence genes. To produce transgenic mice that under-express the *Zace1* gene, such inhibitory sequences are targeted to *Zace1* mRNA. Methods for producing transgenic mice that have abnormally low expression of a particular gene are known to those in the art (see, for example, Wu *et al.,* "Gene Underexpression in Cultured Cells and Animals by Antisense DNA and RNA Strategies," in *Methods in Gene Biotechnology,* pages 205-224 (CRC Press 1997)).

An alternative approach to producing transgenic mice that have little or no *Zace1* gene expression is to generate mice having at least one normal *Zace1* allele replaced by a nonfunctional *Zace1* gene. One method of designing a nonfunctional *Zace1* gene is to insert another gene, such as a selectable marker gene, within a nucleic acid molecule that encodes Zace1. Standard methods for producing these so-called "knockout mice" are known to those skilled in the art (see, for example, Jacob, "Expression and Knockout of Interferons in Transgenic Mice," in *Overexpression and Knockout of Cytokines in Transgenic Mice,* Jacob (ed.), pages 111-124 (Academic Press, Ltd. 1994), and Wu *et al.,* "New Strategies for Gene Knockout," in *Methods in Gene Biotechnology,* pages 339-365 (CRC Press 1997)).

## Claims

1. An isolated polypeptide, comprising an amino acid sequence that is at least 70% identical to a reference amino acid sequence selected from the group consisting of:
(a) amino acid residues 367 to 430 of SEQ ID NO:1.
(b) amino acid residues 163 to 563 of SEQ ID NO: 1,
(c) amino acid residues 52 to 563 of SEQ ID NO:1,
(d) amino acid residues 52 to 644 of SEQ ID NO: 1,
(e) amino acid residues 52 to 648 of SEQ ID NO:1,
(f) amino acid residues 52 to 655 of SEQ ID NO:1,
(g) amino acid residues 52 to 662 of SEQ ID NO: 1,
(h) amino acid residues 52 to 682 of SEQ ID NO:1,
(i) amino acid residues 52 to 694 of SEQ ID NO: 1, and
(j) amino acid residues 1 to 694 of SEQ ID NO:1,
wherein the isolated polypeptide either (a) specifically binds with an antibody that specifically binds with a polypeptide consisting of the amino acid sequence of SEQ ID NO:1, or (b) exhibits dipeptidyl carboxypepridase activity.

2. The isolated polypeptide of claim 1, wherein the isolated polypeptide has an amino acid sequence that is at least 80% identical to the reference amino acid sequence.

3. The isolated polypeptide of claim 1, wherein the isolated polypeptide has an amino acid sequence that is at least 90% identical to the reference amino acid sequence.

4. The isolated polypeptide of claim 1, wherein the isolated polypeptide comprises an amino acid sequence selected from the group consisting of: (a) amino acid residues 367 to 430 of SEQ ID NO:1, (b) amino acid residues 163 to 563 of SEQ ID NO:1, (c) amino acid residues 52 to 563 of SEQ ID NO:1, (d) amino acid residues 52 to 644 of SEQ ID NO:1, (e) amino acid residues 52 to 648 of SEQ ID NO:1, (f) amino acid residues 52 to 655 of SEQ ID NO:1, (g) amino acid residues 52 to 662 of SEQ ID NO:1, (h) amino acid residues 52 to 682 of SEQ ID NO:1, (i) amino acid residues 52 to 694 of SEQ ID NO:1, and (j) amino acid residues 1 to 694 of SEQ ID NO:1.

5. The isolated polypeptide of claim 1. wherein the polypeptide is a metallopeptidase.

6. The isolated polypeptide of claim 1. wherein the polypeptide comprises an amino acid sequence comprising the motif "X₁-X₂-X₃-H-E-X₄-X₅-H-X₆-X₇," wherein
X₁ is selected from G, S, T, A, L, I, V, and N,
X₂, X₃, and X₆ are any amino acid residue,
X₄ is selected from L, I, V, M, F, Y, and W,
X₅ is any amino acid residue except D, E, H, R, K, or P, and
X₇ is selected from L, I, V, M, F, Y, W, G, S, P, and Q.

7. The isolated polypeptide of claim 6, wherein the polypeptide comprises amino acid residues 395 to 404 of SEQ ID NO:1.

8. An isolated polypeptide, wherein the amino acid sequence of the polypeptide shares an identity with the amino acid sequence of SEQ ID NO:1 selected from the group consisting of at least 70% identity, at least 80% identity, at least 90% identity, at least 95% identity, or greater than 95% identity, and wherein any difference between the amino acid sequence of the variant polypeptide and the amino acid sequence of SEQ ID NO:1 is due to one or more conservative amino acid substitutions.

9. The isolated polypeptide of claim 1, comprising the amino acid sequence of SEQ ID NO:1.

10. An isolated nucleic acid molecule that encodes polypeptide comprising an amino acid sequence selected from the group consisting of: (a) amino acid residues 367 to 430 of SEQ ID NO:1, (b) amino acid residues 163 to 563 of SEQ ID NO:1, (c) amino acid residues 52 to 563 of SEQ ID NO: 1. (d) amino acid residues 52 to 644 of SEQ ID NO: 1, (e) amino acid residues S2 to 648 of SBQ ID NO:1, (f) amino acid residues 52 to 655 of SEQ ID NO:1, (g) amino acid residues 52 to 662 of SEQ ID NO:1, (h) amino acid residues 52 to 682 of SEQ ID NO:1, (i) amino acid residues 52 to 694 of SEQ ID NO:1, and (j) amino acid residues 1 to 694 of SEQ ID NO:1.

11. The isolated nucleic acid molecule of claim 10, wherein the nucleic acid molecule encodes amino acid residues 1 to 694 of SEQ ID NO: 1.

12. A vector, comprising the isolated nucleic acid molecule of claim 11,

13. An expression vector, comprising the isolated nucleic acid molecule of claim 11, a transcription promoter, and a transcription terminator, wherein the promoter is operably linked with the nucleic acid molecule, and wherein the nucleic acid molecule is operably linked with the transcription terminator.

14. A recombinant host cell comprising the expression vector of claim 13, wherein the host cell is selected from the group consisting of bacterium, yeast cell, fungal cell, insect cell, mammalian cell, and plant cell.

15. A method of using the expression vector of claim 13 to prepare a polypeptide that comprises amino acid residues 1 to 694 of SEQ ID NO:1, comprising culturing recombinant host cells that comprise the expression vector and that produce the polypeptide.

16. The method of claim 15, further comprising isolating the polypeptide from the cultured recombinant host cells.

17. An antibody or antibody fragment that specifically binds with the polypeptide of claim 4.

18. A method of detecting in a biological sample the presence of a polypeptide that comprises amino acid residues 1 to 694 of SEQ ID NO:1, comprising: (a) contacting the biological sample with an antibody, or an antibody fragment, of claim 17, wherein the contacting is performed under conditions that allow the binding of the antibody or antibody fragment to the biological sample, and (b) detecting any of the bound antibody or bound antibody fragment.

19. A fusion protein comprising a polypeptide of claim 4.

20. An anti-idiotype antibody, or anti-idiotype antibody fragment, that specifically binds with the antibody or antibody fragment of claim 17.

21. The anti-idiotype antibody, or anti-idiotype antibody fragment of claim 20, wherein the anti-idiotype antibody, or anti-idiotype antibody fragment, possesses dipeptidyl carboxypeptidase activity.

22. An isolated polypeptide comprising at least 15 contiguous amino acid residues of the amino acid sequence of SEQ ID NO:1 wherein the polypeptide either (a) specifically binds with an antibody that specifically binds with a polypeptide consisting of the amino acid sequence of SEQ ID NO:1, or (b) exhibits dipeptidyl carboxypeptidase activity.

## Patentansprüche

1. Isoliertes Polypeptid, umfassend eine Aminosäuresequenz, die zu mindestens 70 % identisch zu einer Referenzaminosäuresequenz ist, ausgewählt aus der Gruppe, bestehend aus:
(a) den Aminosäureresten 367 bis 430 der SEQ ID Nr. 1,
(b) den Aminosäureresten 163 bis 563 der SEQ ID Nr. 1,
(c) den Aminosäureresten 52 bis 563 der SEQ ID Nr. 1,
(d) den Aminosäureresten 52 bis 644 der SEQ ID Nr. 1,
(e) den Aminosäureresten 52 bis 648 der SEQ ID Nr. 1,
(f) den Aminosäureresten 52 bis 655 der SEQ ID Nr. 1,
(g) den Aminosäureresten 52 bis 662 der SEQ ID Nr. 1,
(h) den Aminosäureresten 52 bis 682 der SEQ ID Nr. 1,
(i) den Aminosäureresten 52 bis 694 der SEQ ID Nr. 1, und
(j) den Aminosäureresten 1 bis 694 der SEQ ID Nr. 1,
worin das isolierte Polypeptid entweder (a) spezifisch an einen Antikörper bindet, der spezifisch an ein Polypeptid bindet, bestehend aus der Aminosäuresequenz der SEQ ID Nr. 1, oder (b) eine Dipeptidylcarboxypeptidaseaktivität aufweist.

2. Isoliertes Polypeptid nach Anspruch 1, worin das isolierte Polypeptid eine Aminosäuresequenz aufweist, die zu mindestens 80 % identisch zur Referenzaminosäuresequenz ist.

3. Isoliertes Polypeptid nach Anspruch 1, worin das isolierte Polypeptid eine Aminosäuresequenz aufweist, die zu mindestens 90 % identisch zur Referenzaminosäuresequenz ist.

4. Isoliertes Polypeptid nach Anspruch 1, worin das isolierte Polypeptid eine Aminosäuresequenz umfasst, ausgewählt aus der Gruppe, bestehend aus: (a) den Aminosäureresten 367 bis 430 der SEQ ID Nr. 1, (b) den Aminosäureresten 163 bis 563 der SEQ ID Nr. 1, (c) den Aminosäureresten 52 bis 563 der SEQ ID Nr. 1, (d) den Aminosäureresten 52 bis 644 der SEQ ID Nr. 1, (e) den Aminosäureresten 52 bis 648 der SEQ ID Nr. 1, (f) den Aminosäureresten 52 bis 655 der SEQ ID Nr. 1, (g) den Aminosäureresten 52 bis 662 der SEQ ID Nr. 1, (h) den Aminosäureresten 52 bis 682 der SEQ ID Nr. 1, (i) den Aminosäureresten 52 bis 694 der SEQ ID Nr. 1, und (j) den Aminosäureresten 1 bis 694 der SEQ ID Nr. 1.

5. Isoliertes Polypeptid nach Anspruch 1, worin das Polypeptid eine Metallopeptidase ist.

6. Isoliertes Polypeptid nach Anspruch 1, worin das Polypeptid eine Aminosäuresequenz umfasst, umfassend das Motiv "X₁-X₂-X₃-H-E-X₄-X₅-H-X₆-X₇", worin
X₁ ausgewählt ist unter G, S, T, A, L, I, V und N,
X₂, X₃ und X₆ ein beliebiger Aminosäurerest sind,
X₄ ausgewählt ist unter L, I, V, M, F, Y und W,
X₅ ein beliebiger Aminosäurerest ist, ausgenommen D, E, H, R, K oder P, und
X₇ ausgewählt ist unter L, I, V, M, F, Y, W, G, S, P und Q.

7. Isoliertes Polypeptid nach Anspruch 6, worin das Polypeptid die Aminosäurereste 395 bis 404 der SEQ ID Nr. 1 umfasst.

8. Isoliertes Polypeptid, worin die Aminosäuresequenz des Peptids eine Identität mit der Aminosäuresequenz der SEQ ID Nr. 1 teilt, ausgewählt aus der Gruppe, bestehend aus mindestens 70 % Identität, mindestens 80 % Identität, mindestens 90 % Identität, mindestens 95 % Identität oder mehr als 95 % Identität, und worin jeder Unterschied hinsichtlich der Aminosäuresequenz des varianten Polypeptids und der Aminosäuresequenz der SEQ ID Nr. 1 auf einer oder mehreren konservativen Aminosäuresubstitution(en) beruht.

9. Isoliertes Polypeptid nach Anspruch 1, umfassend die Aminosäuresequenz der SEQ ID Nr. 1.

10. Isoliertes Nukleinsäuremolekül, das ein Polypeptid kodiert, umfassend eine Aminosäuresequenz, ausgewählt aus der Gruppe, bestehend aus (a) den Aminosäureresten 367 bis 430 der SEQ ID Nr. 1, (b) den Aminosäureresten 163 bis 563 der SEQ ID Nr. 1, (c) den Aminosäureresten 52 bis 563 der SEQ ID Nr. 1, (d) den Aminosäureresten 52 bis 644 der SEQ ID Nr. 1, (e) den Aminosäureresten 52 bis 648 der SEQ ID Nr. 1, (f) den Aminosäureresten 52 bis 655 der SEQ ID Nr. 1, (g) den Aminosäureresten 52 bis 662 der SEQ ID Nr. 1, (h) den Aminosäureresten 52 bis 682 der SEQ ID Nr. 1, (i) den Aminosäureresten 52 bis 694 der SEQ ID Nr. 1, und (j) den Aminosäureresten 1 bis 694 der SEQ ID Nr. 1.

11. Isoliertes Nukleinsäuremolekül nach Anspruch 10, worin das Nukleinsäuremolekül die Aminosäurereste 1 bis 694 der SEQ ID Nr. 1 kodiert.

12. Vektor, umfassend das isolierte Nukleinsäuremolekül nach Anspruch 11.

13. Expressionsvektor, umfassend das isolierte Nukleinsäuremolekül nach Anspruch 11, einen Transkriptionspromoter und einen Transkriptionsterminator, worin der Promoter operativ mit dem Nukleinsäuremolekül verbunden ist und worin das Nukleinsäuremolekül operativ mit dem Transkriptionsterminator verbunden ist.

14. Rekombinante Wirtszelle, umfassend den Expressionsvektor nach Anspruch 13, worin die Wirtszelle ausgewählt ist aus der Gruppe, bestehend aus einem Bakterium, einer Hefezelle, einer Pilzzelle, einer Insektenzelle, einer Säugerzelle und einer Pflanzenzelle.

15. Verfahren zur Verwendung des Expressionsvektors nach Anspruch 13 zur Herstellung eines Polypeptids, das die Aminosäurereste 1 bis 694 der SEQ ID Nr. 1 umfasst, umfassend das Kultivieren von rekombinanten Wirtszellen, welche den Expressionsvektor enthalten und das Polypeptid produzieren.

16. Verfahren nach Anspruch 15, zusätzlich umfassend das Isolieren des Polypeptids aus den kultivierten rekombinanten Wirtszellen.

17. Antikörper oder Antikörperfragment, der bzw. das spezifisch an das Polypeptid aus Anspruch 4 bindet.

18. Verfahren zum Detektieren der Anwesenheit eines Polypeptids in einer biologischen Probe, das die Aminosäurereste 1 bis 694 der SEQ ID Nr. 1 umfasst, umfassend: (a) In-Kontakt-Bringen der biologischen Probe mit einem Antikörper oder Antikörperfragment aus Anspruch 17, worin das In-Kontakt-Bringen unter Bedingungen durchgeführt wird, die die Bindung des Antikörpers oder Antikörperfragments an die biologische Probe gestatten,und (b) das Detektieren jeglichen gebundenen Antikörpers oder gebundenen Antikörperfragmentes.

19. Fusionsprotein, umfassend ein Polypeptid nach Anspruch 4.

20. Antiidiotypischer Antikörper oder antiidiotypisches Antikörperfragment, das spezifisch an den Antikörper oder das Antikörperfragment nach Anspruch 17 bindet.

21. Antiidiotypischer Antikörper oder antiidiotypisches Antikörperfragment nach Anspruch 20, worin der antiidiotypische Antikörper oder das antiidiotypische Antikörperfragment eine Dipeptidylcarboxypeptidaseaktivität aufweisen.

22. Isoliertes Polypeptid, umfassend mindestens 15 fortlaufende Aminosäurereste der Aminosäuresequenz der SEQ ID Nr. 1, worin das Polypeptid entweder (a) spezifisch an einen Antikörper bindet, der spezifisch an ein Polypeptid bindet, bestehend aus der Aminosäuresequenz der SEQ ID Nr. 1, oder (b) eine Dipeptidylcarboxypeptidaseaktivität aufweist.

## Revendications

1. Un polypeptide isolé, comprenant une séquence d'acides aminés qui est au moins à 70 % identique à une séquence d'acides aminés de référence sélectionnée dans le groupe consistant en :
a) les résidus d'acides aminés 367 à 430 de la SEQ ID NO : 1,
b) les résidus d'acides aminés 163 à 563 de la SEQ ID NO : 1,
c) les résidus d'acides aminés 52 à 563 de la SEQ ID NO : 1,
d) les résidus d'acides aminés 52 à 644 de la SEQ ID NO : 1,
e) les résidus d'acides aminés 52 à 648 de la SEQ ID NO : 1,
f) les résidus d'acides aminés 52 à 655 de la SEQ ID NO : 1,
g) les résidus d'acides aminés 52 à 662 de la SEQ ID NO : 1,
h) les résidus d'acides aminés 52 à 682 de la SEQ ID NO : 1,
i) les résidus d'acides aminés 52 à 694 de la SEQ ID NO : 1 et
j) les résidus d'acides aminés 1 à 694 de la SEQ ID NO : 1,
dans lequel le polypeptide isolé soit (a) se lie spécifiquement à un anticorps qui se lie spécifiquement à un polypeptide consistant en la séquence d'acides aminés de la SEQ ID NO : 1, soit (b) exhibe une activité de dipeptidyl carboxypeptidase.

2. Le polypeptide isolé selon la revendication 1, dans lequel le polypeptide isolé a une séquence d'acides aminés qui est au moins à 80 % identique à la séquence d'acides aminés de référence.

3. Le polypeptide isolé selon la revendication 1, dans lequel le polypeptide isolé a une séquence d'acides aminés qui est au moins à 90 % identique à la séquence d'acides aminés de référence.

4. Le polypeptide isolé selon la revendication 1, dans lequel le polypeptide isolé comprend une séquence d'acides aminés sélectionnée dans le groupe consistant en : (a) les résidus d'acides aminés 367 à 430 de la SEQ ID NO : 1, (b) les résidus d'acides aminés 163 à 563 de la SEQ ID NO : 1, (c) les résidus d'acides aminés 52 à 563 de la SEQ ID NO : 1, (d) les résidus d'acides aminés 52 à 644 de la SEQ ID NO : 1, (e) les résidus d'acides aminés 52 à 648 de la SEQ ID NO : 1, (f) les résidus d'acides aminés 52 à 655 de la SEQ ID NO : 1, (g) les résidus d'acides aminés 52 à 662 de la SEQ ID NO : 1, (h) les résidus d'acides aminés 52 à 682 de la SEQ ID NO : 1, (i) les résidus d'acides aminés 52 à 694 de la SEQ ID NO : 1, et (j) les résidus d'acides aminés 1 à 694 de la SEQ ID NO : 1.

5. Le polypeptide isolé selon la revendication 1, dans lequel le polypeptide est une métallo-peptidase.

6. Le polypeptide isolé selon la revendication 1, dans lequel le polypeptide comprend une séquence d'acides aminés comprenant le motif "X₁-X₂-X₃-H-E-X₄-X₅-H-X₆-X₇", dans lequel
X₁ est sélectionné parmi G, S, T, A, L, I, V et N,
X₂, X₃ et X₆ sont n'importe quel résidu d'acide aminé,
X₄ est sélectionné parmi L, I, V, F, Y et W,
X₅ est n'importe quel résidu d'acide aminé excepté D, E, H, R, K ou P et
X₇ est sélectionné parmi L, I, V, M, F, Y, W, G, S, P et Q.

7. Le polypeptide isolé selon la revendication 6, dans lequel le polypeptide comprend les résidus d'acides aminés 395 à 404 de la SEQ ID NO : 1.

8. Un polypeptide isolé, dans lequel la séquence d'acides aminés du polypeptide partage une identité avec la séquence d'acides aminés de la SEQ ID NO : 1 sélectionnée dans le groupe consistant en au moins 70 % d'identité, au moins 80 % d'identité, au moins 90 % d'identité, au moins 95 % d'identité ou plus grand que 95 % d'identité et dans lequel toute différence entre la séquence d'acides aminés du polypeptide variant et la séquence d'acides aminés de la SEQ ID NO : 1 est due à une ou plusieurs substitutions d'acides aminés conservatrices.

9. Le polypeptide isolé selon la revendication 1, comprenant la séquence d'acides aminés de la SEQ ID NO : 1.

10. Une molécule d'acide nucléique isolée qui code pour un polypeptide comprenant une séquence d'acides aminés sélectionnée dans le groupe consistant en : (a) les résidus d'acides aminés 367 à 430 de la SEQ ID NO : 1, (b) les résidus d'acides aminés 163 à 563 de la SEQ ID NO : 1, (c) les résidus d'acides aminés 52 à 563 de la SEQ ID NO : 1, (d) les résidus d'acides aminés 52 à 644 de la SEQ ID NO : 1, (e) les résidus d'acides aminés 52 à 648 de la SEQ ID NO : 1, (f) les résidus d'acides aminés 52 à 655 de la SEQ ID NO : 1, (g) les résidus d'acides aminés 52 à 662 de la SEQ ID NO : 1, (h) les résidus d'acides aminés 52 à 682 de la SEQ ID NO: 1, (i) les résidus d'acides aminés 52 à 694 de la SEQ ID NO : 1, et (j) les résidus d'acides aminés 1 à 694 de la SEQ ID NO : 1.

11. La molécule d'acide nucléique isolée selon la revendication 10, dans laquelle la molécule d'acide nucléique code pour les résidus d'acides aminés 1 à 694 de la SEQ ID NO : 1.

12. Un vecteur, comprenant la molécule d'acide nucléique isolée de la revendication 11.

13. Un vecteur d'expression, comprenant la molécule d'acide nucléique isolée selon la revendication 11, un promoteur de transcription et un terminateur de transcription, dans lequel le promoteur est lié de manière opérationnelle à la molécule d'acide nucléique et dans lequel la molécule d'acide nucléique est liée de manière opérationnelle au terminateur de transcription.

14. Une cellule hôte recombinante comprenant le vecteur d'expression selon la revendication 13, dans laquelle la cellule hôte est sélectionnée dans le groupe consistant en une bactérie, une cellule de levure, une cellule fongique, une cellule d'insecte, une cellule mammalienne et une cellule de plante.

15. Un procédé d'utilisation du vecteur d'expression de la revendication 13 pour préparer un polypeptide qui comprend les résidus d'acides aminés 1 à 694 de la SEQ ID NO : 1, comprenant la culture de cellules hôtes recombinantes qui comprennent le vecteur d'expression et qui produisent le polypeptide.

16. Le procédé selon la revendication 15, comprenant en outre l'isolement du polypeptide à partir des cellules hôtes recombinantes cultivées.

17. Un anticorps ou un fragment d'anticorps qui se lie spécifiquement au polypeptide selon la revendication 4.

18. Un procédé pour détecter dans un échantillon biologique la présence d'un polypeptide qui comprend les résidus d'acides aminés 1 à 694 de la SEQ ID NO : 1, comprenant: (a) la mise en contact de l'échantillon biologique avec un anticorps, ou un fragment d'anticorps, selon la revendication 17, dans lequel la mise en contact est effectuée dans des conditions qui permettent la liaison de l'anticorps ou du fragment d'anticorps à l'échantillon biologique et (b) la détection de tout anticorps lié ou fragment d'anticorps lié.

19. Une protéine de fusion comprenant un polypeptide selon la revendication 4.

20. Un anticorps anti-idiotype, ou un fragment d'anticorps anti-idiotype, qui se lie spécifiquement avec l'anticorps ou le fragment d'anticorps selon la revendication 17.

21. L'anticorps anti-idiotype, ou le fragment d'anticorps anti-idiotype, selon la revendication 20, dans lequel l'anticorps anti-idiotype, ou le fragment d'anticorps anti-idiotype, possède une activité de dipeptidyl carboxypeptidase.

22. Un polypeptide isolé comprenant au moins 15 résidus d'acides aminés contigus de la séquence d'acides aminés de la SEQ ID NO : 1, dans lequel le polypeptide soit (a) se lie spécifiquement à un anticorps qui se lie spécifiquement à un polypeptide consistant en la séquence d'acides aminés de la SEQ ID NO: 1, soit (b) présente une activité de dipeptidyl carboxypeptidase.
